(19) Europäisches Patentamt
European Patent Office
Office européen des brevets

(11) EP 2 518 014 A1

(12) # EUROPEAN PATENT APPLICATION

published in accordance with Art. 153(4) EPC

(43) Date of publication:
**31.10.2012 Bulletin 2012/44**

(21) Application number: **10839375.2**

(22) Date of filing: **21.12.2010**

(51) Int Cl.:
***C01B 31/02** (2006.01)* ***B01J 23/745** (2006.01)*

(86) International application number:
**PCT/JP2010/072957**

(87) International publication number:
**WO 2011/078145 (30.06.2011 Gazette 2011/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.12.2009 JP 2009292120**
**22.02.2010 JP 2010035939**
**31.03.2010 JP 2010080641**

(71) Applicant: **Toray Industries, Inc.**
**Tokyo 103-8666 (JP)**

(72) Inventors:
- **ASANO, Itaru**
**Nagoya-shi**
**Aichi 455-8502 (JP)**
- **TAKEZAKI, Hiroshi**
**Nagoya-shi**
**Aichi 455-8502 (JP)**

(74) Representative: **Webster, Jeremy Mark**
**Mewburn Ellis LLP**
**33 Gutter Lane**
**London**
**EC2V 8AS (GB)**

(54) **CARBON MICROPARTICLE AND PROCESS FOR PRODUCTION THEREOF**

(57) A process for producing carbon microparticles, characterised in that synthetic resin microparticles, metal-containing synthetic resin microparticles or child-particle-containing synthetic resin microparticles are subjected to carbonization baking, wherein the synthetic resin microparticles, the metal-containing synthetic resin microparticles or the child-particle-containing synthetic resin microparticles are produced by a process comprising mixing a polymer (A) such as polyacrylonitrile copolymer microparticles composed of a copolymer of an acrylonitrile monomer and a hydrophilic vinyl monomer with a polymer (B) that is different from the polymer (A) in an organic solvent to produce an emulsion and bringing the emulsion into contact with a poor solvent for the polymer (A), thereby causing the polymer (A) to precipitate; and the carbon microparticles.

[Fig. 3]

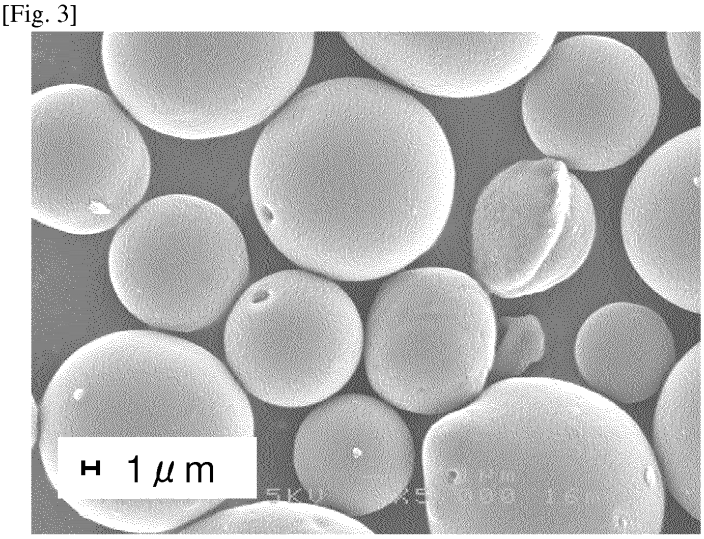


EP 2 518 014 A1

## Description

Technical field

The present invention relates to high-quality carbon particles with a narrow particle diameter distribution, and a production method thereof.

Background art

High in mechanical characteristics, chemical resistance, heat resistance, wear resistance, and electric conductivity, carbon particles have been used widely as heat resistant filler, composite material reinforcement, filling materials, and the like in various fields such as automobile parts, electric parts, industrial machinery, and office equipment, and there is a call for higher-quality carbon particles with a narrow particle diameter distribution to meet enhanced function requirements.

To provide these carbon particles with high functions, increasing attention is attracted to metal-containing carbon particles with magnetic characteristics or catalytic function and porous carbon particles having pores in their interior to increase the specific surface.

Metal-containing carbon particles may vary in functionality depending on the metal contained, and it is known, for instance, that contrast dyes for MRI and DDS carriers can be developed by adding metal magnetism (Patent documents 1 and 2) and that catalysts for fuel cells can be developed by combining metal and carbon particles to develop a catalyst function (Patent document 3).

Activated carbon, a typical porous carbon particle material containing pores in its interior, has been in industrial use in a wide variety of fields such as adsorption agents, molecular sieving carbon materials, water treatment agents, air cleaning agents, catalysts, and capacitor electrodes, which make effective use of such pores (Patent document 4). Activated carbon can be produced by subjecting carbon particles, used as input material, to activation treatment with an appropriate gas such as water vapor or activation treatment with an appropriate chemical agent such as potassium hydroxide, but it is difficult to control its pore size. Functionality of activated carbon depends on the size and capacity (specific surface) of pores and accordingly, it is important to use an appropriate technique to produce pores with a desired size. Attention is now focused also on development of activated carbon with improved quality which may be achieved through control of particle shape, particle diameter and narrow particle diameter distribution.

One of the methods disclosed so far is to calcine core-shell type fine particles consisting of a heat decomposable polymer, such as styrene, as core and a carbon precursor polymer, such as polyacrylonitrile, as shell to produce hollow carbon particles (Patent document 5). In hollow carbon particles produced by this method, hollow structures have large diameters equivalent to 20% or more of those of the carbon particles, failing to have an adequate specific surface, and furthermore, they are produced by emulsion polymerization, limiting the particle diameter control range to 0.5 $\mu$m or less.

In another disclosed method, hollow carbon material, such as fullerene, and a carbon precursor are dispersed by melt-kneading, crushed, calcined, and subjected to activation treatment to produce activated carbon (Patent document 6). With this method, however, the crushing step leads to undesired results such as activated carbon grains having irregular shapes or a wide particle diameter distribution.

Conventionally, there have been disclosed production methods for these carbon particles including metal-containing carbon particles and porous carbon particles, such as a process comprising calcination of coke, pitch and other materials and subsequent mechanical crushing and a process comprising preparation and subsequent calcination of synthetic polymer particles.

Although carbon materials produced by calcination of coke and pitch have a high degree of graphitization, the step of mechanical crushing for particle size reduction leads to carbon particles with a wide particle diameter distribution and nonspherical particle shapes, which will be poor in dispersibility and space-filling capability in resin when used as fillers.

The disclosed methods for producing carbon particles by calcining synthetic polymer particles include calcination of phenol formaldehyde resin particles and calcination of polyacrylonitrile particles. In the case of a production method where phenol formaldehyde resin particles are calcined, phenol formaldehyde particles resulting from condensation polymerization of phenol and formaldehyde used as input materials will have a large particle diameter distribution index, and accordingly, the resulting carbon particles will also have a wide particle diameter distribution (Patent document 7). For carbon particle production from polyacrylonitrile-based polymer particles, on the other hand, polymerization is performed to produce the particles, and accordingly, if the reaction is performed in an aqueous polymerization system with a high copolymerization ratio for polyacrylonitrile, it can suffer from significant coagulation and difficulty in control, leading to a wide particle diameter distribution and difficulty in producing perfectly spherical particles, as already disclosed (Patent documents 8 and 9). The use of a special polymerization method to produce carbon particles with a narrow particle diameter distribution has been disclosed, but there are problems such as still failing to achieve sufficient quality

and adequate characteristics, which may be attributed to the use of fine particles of a copolymer produced from copolymerizing acrylonitrile with copolymerization units such as divinylbenzene and styrene (Patent documents 10 and 11). Consequently, it is difficult for the conventional methods to provide an industrially simplified process to produce high-quality carbon particles with a narrow particle diameter distribution.

Prior art documents

Patent documents

Patent document 1: Japanese Unexamined Patent Publication (Kokai) No. 2009-293052
Patent document 2: Japanese Unexamined Patent Publication (Kokai) No. 2009-292651
Patent document 3: Japanese Unexamined Patent Publication (Kokai) No. 2009-291707
Patent document 4: Japanese Unexamined Patent Publication (Kokai) No. 2001-122607
Patent document 5: Japanese Unexamined Patent Publication (Kokai) No. HEI 5-254814
Patent document 6: Japanese Unexamined Patent Publication (Kokai) No. 2007-269551
Patent document 7: Japanese Unexamined Patent Publication (Kokai) No. SHO 63-129006
Patent document 8: Japanese Unexamined Patent Publication (Kokai) No. 2003-226720
Patent document 9: Japanese Unexamined Patent Publication (Kokai) No. SHO 61-26505
Patent document 10: Japanese Unexamined Patent Publication (Kokai) No. HEI 05-139711
Patent document 11: International Publication WO2005/098998

Summary of the invention

Problems to be solved by the invention

The present invention aims to provide a simplified method for production of high-quality carbon particles with a narrow particle diameter distribution, and furthermore, production of carbon particles with a narrow particle diameter distribution and a high degree of graphitization, and also provides metal-containing carbon particles and hollow carbon particles produced by applying the method, as well as a process for production thereof.

Means of solving the problems

As a result of intensive studies, the present inventors found that it was possible to meet the above aim by carbonizing synthetic polymer particles, metal-containing synthetic polymer particles, or daughter-particle-containing synthetic polymer particles that have a narrow particle diameter distribution.

Specifically, the present invention has a constitution as described below.

[1] A carbon particle production process comprising mixing polymer A which is at least one or more selected from the group of a polyolefin based copolymer, polyacrylonitrile based copolymer consisting of an acrylonitrile based monomer and a hydrophilic vinyl monomer, polyacrylamide based polymer, polyvinyl acetate based polymer, polyvinyl chloride based polymer, polyvinylidene chloride based polymer, polyamide, polyarylene ether, polyarylene sulfide, polysulfone, polyether ketone, polyether ether ketone, polyarylate, polyamide-imide, and polyimide, and polymer B which is dissimilar thereto in an organic solvent to form an emulsion in a phase-separated system consisting of a solution phase containing polymer A as primary constituent and another solution phase containing polymer B as primary constituent, which is then brought into contact with a poor solvent for polymer A to precipitate polymer A, followed by producing particles of polymer A and carbonizing the resulting polymer particles.

[2] A carbon particle production process as described in [1] wherein said polymer A is a polyacrylonitrile based copolymer consisting of an acrylonitrile based monomer and a hydrophilic vinyl monomer, or a polyamide-imide.

[3] A carbon particle production process as described in either [1] or [2] wherein said polyacrylonitrile based copolymer consisting of an acrylonitrile based monomer and a hydrophilic vinyl monomer is an acrylonitrile copolymer produced by copolymerizing 100 parts by mass of an acrylonitrile based monomer with more than 0 to 25 parts by mass of a hydrophilic vinyl monomer.

[4] A carbon particle production process as described in any of [1] to [3] wherein said hydrophilic vinyl monomer contains one or more of the following: hydroxyl group, carboxyl group, amino group, amide group, sulfonic acid group and phosphoric acid group.

[5] A carbon particle production process as described in any of [1] to [4] wherein said hydrophilic vinyl monomer

contains one or more of the following: amide groups and carboxyl groups.
[6] A carbon particle production process as described in any of [1] to [5] wherein said hydrophilic vinyl monomer is one or more selected from the following: acrylic acids, methacrylic acids, itaconic acid, and acrylamide.
[7] A carbon particle production process wherein polyamide-imide particles are carbonized.
[8] A carbon particle production process as described in any of [1] to [7] wherein the degree of graphitization is 1.0 or more.
[9] A carbon particle production process as described in any of [1] to [8] wherein the number-average particle diameter is 0.1 to 100 μm and the particle diameter distribution index is 1.0 to 2.0.
[10] A carbon particle production process as described in any of [1] to [9] wherein said synthetic polymer particles contains metal.
[11] A carbon particle production process as described in any of [1] to [10] wherein the synthetic polymer particles contain daughter particles and can be carbonized into porous carbon particles.
[12] A carbon particle production process as described in any of claims [1] to [9] and [11] wherein the daughter particles are organic particles.
[13] Carbon particles having a number average particle diameter of 0.1 to 100 μm, a particle diameter distribution index of 1.0 to 2.0, a degree of graphitization of 1.0 or more, and a sphericity of 80 or more.
[14] Carbon particles as described in [13] that contain metal.
[15] Carbon particles as described in [14] wherein the metal is a transition metal.
[16] Metal-containing carbon particles as described in either [14] or [15] wherein the metal is at least one selected from the group of cobalt, iron, manganese, nickel, copper, titanium, chrome, and zinc.
[17] Carbon particles as described in any of [14] to [16] wherein the metal is a magnetic metal.
[18] A catalyst comprising carbon particles as described in any of [14] to [17].

Effect of the invention

The carbon particle production process according to the present invention serves to produce high-quality carbon particles with a narrow particle diameter distribution and a high degree of graphitization in a simple manner, and the process can be applied to production of metal-containing carbon particles that are perfectly spherical and porous carbon particles containing porous structures of a specific size.
Having particle diameters that give high handleability and also having a narrow particle diameter distribution, the carbon particles, metal-containing carbon particles and porous carbon particles according to the present invention are preferred from the viewpoint of forming a coating film with a uniformity thickness when spread, in particular, over a membrane-like material such as film. In a preferable embodiment, they are spherical and preferred in terms of decreased coating irregularities.
The carbon particles, metal-containing carbon particles and porous carbon particles according to the present invention show high dispersibility and gap-filling capability when used with resin or rubber, and can be used preferably in producing a resin modifying agent, electric conductive rubber, anisotropically conductive particles, chromatographic carrier, molecule adsorption agent, toner, negative electrode material for lithium ion batteries, catalyst, catalyst carrier, activated carbon, and capacitor electrode.
Furthermore, metal-containing carbon particles containing, as metal component, a magnetic substance or a metal that can form a magnetic substance can be used preferably in producing a carrier for diagnosis that needs magnetic characteristics and a contrast dye for MRI.

Brief description of the drawings

[Fig. 1] Fig. 1 is a scanning electronic microscope photograph of polyacrylonitrile based copolymer particles produced in Production example 1.
[Fig. 2] Fig. 2 is a scanning electronic microscope photograph of polyacrylonitrile based copolymer particles produced in Production example 2.
[Fig. 3] Fig. 3 is a scanning electronic microscope photograph of carbon particles produced in Example 1.
[Fig. 4] Fig. 4 is a scanning electronic microscope photograph of carbon particles produced in Comparative example 2.
[Fig. 5] Fig. 5 is a scanning electronic microscope photograph of polyamide-imide particles produced in Production example 7.
[Fig. 6] Fig. 6 is a scanning electronic microscope photograph of carbon particles produced in Example 4.
[Fig. 7] Fig. 7 is a scanning electronic microscope photograph of a cross section of a carbon particle produced in Example 7.

[Fig. 8] Fig. 8 is a scanning electronic microscope photograph of carbon particles produced in Example 7.

[Fig. 9] Fig. 9 is a scanning electronic microscope photograph of cross sections of carbon particles produced in Example 1, which is given for comparison with Example 7.

Description of embodiments

The present invention described more in detail below.

The carbon particles according to the present invention are fine particles substantially made of carbon alone, and are produced by carbonizing synthetic polymer particles of a material selected from the group of a polyolefin based copolymer, polyacrylonitrile based copolymer consisting of an acrylonitrile based monomer and a hydrophilic vinyl monomer, polyacrylamide based polymer, polyvinyl acetate based polymer, polyvinyl chloride based polymer, polyvinylidene chloride based polymer, polyamide, polyarylene ether, polyarylene sulfide, polysulfone, polyether ketone, polyether ether ketone, polyarylate, polyamide-imide, and polyimide.

The carbon particles according to the present invention have a number average particle diameter of 100 μm or less, preferably 50 μm or less, and more preferably 30 μm or less as upper limit. As lower limit, it is 0.1 μm or more, preferably 0.5 μm or more, more preferably 0.7 μm or more, and particularly preferably 1 μm or more. The handleability deteriorates if the number average particle diameter is too small while the dispersibility in resin etc. deteriorates if it is too large, both of which are unpreferable. Here, the number average particle diameter of the carbon particles is determined by observing 100 particles randomly selected from scanning electronic microscope photographs, measuring their diameters, and making calculations by Equation (1) given below.

Here, if a particle is not perfectly circular, its major axis is taken as its diameter.

The particle diameter distribution of the carbon particles according to the present invention can be represented by the particle diameter distribution index, and their particle diameter distribution index is in the range of 1.0 to 2.0, preferably 1.0 to 1.8, more preferably 1.0 to 1.5, and particularly preferably 1.0 to 1.3. A particle diameter distribution index out of these ranges is not preferable because in that case, the particles will be low in flowability and gap-filling capability for resin and other materials. Here, the particle diameter distribution index is calculated by Equation (3) given below from the ratio of the volume average particle diameter to the number average particle diameter. The volume average particle diameter is determined by observing 100 particles randomly selected from scanning electronic microscope photographs, measuring their diameters, and making calculations by Equation (2) given below. Here, if a particle is not perfectly circular, its major axis is taken as its diameter.

[Mathematical formula 1]

$$Dn = \frac{\sum_{i=1}^{n} Ri}{n} \quad (1)$$

$$Dv = \frac{\sum_{i=1}^{n} Ri^{4}}{\sum_{i=1}^{n} Ri^{3}} \quad (2)$$

$$PDI = \frac{Dv}{Dn} \quad (3)$$

Here, Ri, n, Dn, Dv, and PDI denote the particle diameter of each particle, number of measurements (100), number average particle diameter, volume average particle diameter, and particle diameter distribution index, respectively.

The carbon in the carbon particles according to the present invention may be amorphous carbon, graphitized carbon, crystallized graphite with a laminated structure composed of graphite layers, or diamond-like carbon with a sp3 bonding structure, which may be used independently or as a mixture. It is preferably graphitized carbon, crystallized graphite with a laminated structure composed of graphite layers, or a mixture thereof, more preferably a mixture of graphitized carbon and crystallized graphite with a laminated structure composed of graphite layers.

The carbon particles according to the present invention have a degree of graphitization of 1.0 or more, preferably 1.2 or more. An excessively small degree of graphitization is not preferable because in that case, the electric conductivity and heat conductivity, for instance, will deteriorate. There are no specific limitations on the upper limit, but it is commonly about 3.0. Here, the degree of graphitization is calculated as the ratio (B)/(A) where (A) and (B) represent the height of Raman shift (A) (D-band attributed to defects in amorphous carbon and graphite) appearing at about 1,350±100 $cm^{-1}$ and the height of Raman shift (B) (G-band attributed to graphite) appearing at about 1,590±100 $cm^{-1}$ in Raman spectra observed by Raman spectroscopic analysis using laser with a wavelength of 532 nm.

The carbon particles according to the present invention are also characterized by having a nearly perfect spherical shape. The carbon particles according to the present invention have a sphericity of 80 or more, preferably 85 or more, more preferably 90 or more, particularly preferably 92 or more, still more preferably 95 or more, and most preferably 99 or more. A sphericity of less than 80 is not preferable because in that case, the gap-filling capability for resin or other materials will deteriorate. Here, the sphericity is determined by observing particles by scanning electronic microscopy, measuring their major and minor axes, and making calculations by Equation (4) from their averages over randomly selected 30 particles.

[Mathematical formula 2]

$$\text{Sphericity} = \frac{\sum_{i=1}^{n}(\text{minor axes}/\text{major axes})}{n} \times 100 \qquad (4)$$

Here, n denotes the number of measurements, which is equal to 30.

The metal-containing carbon particles according to the present invention are fine particles of carbon that contain metal, and are carbonized metal-containing synthetic polymer particles produced by adding metal to synthetic polymer particles of a material selected from the group of a polyolefin based copolymer, polyacrylonitrile based copolymer consisting of an acrylonitrile based monomer and a hydrophilic vinyl monomer, polyacrylamide based polymer, polyvinyl acetate based polymer, polyvinyl chloride based polymer, polyvinylidene chloride based polymer, polyamide, polyarylene ether, polyarylene sulfide, polysulfone, polyether ketone, polyether ether ketone, polyarylate, polyamide-imide, and polyimide.

The metal-containing carbon particles according to the present invention have a number average particle diameter of 100 μm or less, preferably 50 μm or less, and more preferably 30 μm or less, as upper limit. As lower limit, it is 0.1 μm or more, preferably 0.5 μm or more, more preferably 0.7 μm or more, and particularly preferably 1 μm or more. The handleability deteriorates if the number average particle diameter is too small while if it is too large, the particles can act as foreign objects when applied over a thin membrane-like material such as film, both of which are unpreferable.

Here, the number average particle diameter of the metal-containing carbon particles is defined as the arithmetic average determined by observing 100 particles randomly selected from scanning electronic microscope photographs, measuring their diameters, and making calculations by Equation (1) given below. Note that if a particle observed in a photograph is not perfectly circular, its major axis is taken as its diameter.

The particle diameter distribution of the metal-containing carbon particles according to the present invention can be represented by the particle diameter distribution index, and their particle diameter distribution index is in the range of 1.0 to 2.0, preferably 1.0 to 1.8, more preferably 1.0 to 1.5, and particularly preferably 1.0 to 1.3. A particle diameter distribution index out of these ranges is not preferable because in that case, the particles will be low in flowability and gap-filling capability for resin and other materials. Here, the particle diameter distribution index is calculated by Equation (3) given below from the ratio of the volume average particle diameter to the number average particle diameter. The volume average particle diameter is determined by observing 100 particles randomly selected from scanning electronic microscope photographs, measuring their diameters, and making calculations by Equation (2) given below. Here, if a particle is not perfectly circular, its major axis is taken as its diameter.

[Mathematical formula 3]

$$Dn = \frac{\sum_{i=1}^{n} Ri}{n} \quad (1)$$

$$Dv = \frac{\sum_{i=1}^{n} Ri^{4}}{\sum_{i=1}^{n} Ri^{3}} \quad (2)$$

$$PDI = \frac{Dv}{Dn} \quad (3)$$

Here, Ri, n, and Dn denote the particle diameter of a particular particle, number of measurements (100), and number average particle diameter, respectively.

The metal-containing carbon particles according to the present invention are also characterized by having a nearly perfect spherical shape.

The spherical shape as referred to herein can be evaluated in terms of sphericity, and the particles have a sphericity of 80 or more, preferably 85 or more, more preferably 90 or more, particularly preferably 92 or more, still more preferably 95 or more, and most preferably 99 or more. A sphericity of less than 80 is not preferable because in that case, the gap-filling capability for resin or other materials will deteriorate. The sphericity as referred to herein is determined by observing particles by scanning electronic microscopy, measuring their major and minor axes, and making calculations by Equation (4) from their averages over randomly selected 30 particles.

[Mathematical formula 4]

$$\text{Sphericity} = \frac{\sum_{i=1}^{n} (\text{minor axes} / \text{major axes})}{n} \times 100 \quad (4)$$

Here, n denotes the number of measurements, which is equal to 30.

The carbon in the metal-containing carbon particles according to the present invention may be amorphous carbon, graphitized carbon, crystallized graphite with a laminated structure composed of graphite layers, or diamond-like carbon with a sp3 bonding structure, which may be used independently or as a mixture. It is preferably graphitized carbon, crystallized graphite with a laminated structure composed of graphite layers, or a mixture thereof, more preferably a mixture of graphitized carbon and crystallized graphite with a laminated structure composed of graphite layers.

For a mixture of graphitized carbon and crystallized graphite with a laminated structure composed of graphite layers as referred to herein, the degree of graphitization can be defined as the G/D ratio determined by Raman spectroscopic analysis, and this degree of graphitization is preferably 1.0 or more, more preferably 1.2 or more. If the degree of graphitization is too small, the electric conductivity and heat conductivity, for instance, will tend to deteriorate. There are no specific limitations on the upper limit, but it is commonly about 3.0. Here, the degree of graphitization is calculated as the ratio (B)/(A) where (A) and (B) represent the height of Raman shift (A) (D-band attributed to defects in amorphous carbon and graphite) appearing at about 1,350±100 $cm^{-1}$ and the height of Raman shift (B) (G-band attributed to graphite) appearing at about 1,590±100 $cm^{-1}$ in Raman spectra observed by Raman spectroscopic analysis using laser with a wavelength of 532 nm.

The metal in the metal-containing carbon particles according to the present invention is contained there to serve for developing a specific function, and it may be any metal included in the periodic table. It is only necessary to simply select a metal that serves to develop an intended function. The usable metals include alkali metals, alkaline earth metals, transition metals, light metals, lanthanoids, and actinoids, of which transition metals are preferable.

Specifically, they include alkali metals such as lithium, sodium, potassium, rubidium, cesium, francium; alkaline earth metals such as beryllium, magnesium, calcium, strontium, barium, and radium; transition metals such as scandium, yttrium, lanthanum, actinium, titanium, zirconium, hafnium, vanadium, niobium, tantalum, chrome, molybdenum, tungsten, manganese, technetium, rhenium, iron, ruthenium, osmium, cobalt, rhodium, iridium, nickel, palladium, platinum, copper, silver, gold, zinc, cadmium, and mercury; light metals such as aluminum, gallium, indium, thallium, silicon, germanium, tin, lead, antimony, bismuth, and polonium; and others such as lanthanoids and actinoids.

These metals may be elemental metals, salts thereof, or oxides thereof. Two or more of these may be contained.

The preferable ones include magnesium, calcium, titanium, zirconium, niobium, tantalum, chrome, molybdenum, tungsten, manganese, rhenium, iron, ruthenium, osmium, cobalt, rhodium, iridium, nickel, palladium, platinum, copper, silver, gold, zinc, indium, and silicon, as well as salts and oxides thereof, of which titanium, chrome, manganese, iron, cobalt, nickel, copper, and zinc, as well as salts, oxides, and organometallic complexes thereof are more preferable.

The salts that constitute these metal salts may be in the form of a chloride salt, bromide salt, iodide salt, sulfonate salt, nitrate, phosphate, borate, perchlorate, carbonate, quaternary ammonium salt, alkoxy salt, carboxy salt, boron tetrafluoride salt, or acetylacetonate salt. In particular, the preferable ones include chloride salt, carbonate, alkoxy salt, carboxyl salt, and acetylacetonate salt.

The organometallic complexes include, for instance, phthalocyanine complexes, Schiff base complexes, and bipyridine complexes.

For the metal-containing carbon particles according to the present invention, an appropriate amount of a metal may be added depending on the intended function, but in order to develop a function adequately, the metal atoms account for 0.05 parts by mass to 50 parts by mass, preferably 0.05 parts by mass to 40 parts by mass, more preferably 0.05 parts by mass to 25 parts by mass, and particularly preferably 0.05 parts by mass to 15 parts by mass per 100 parts by mass of the metal-containing carbon particles.

The porous carbon particles according to the present invention are characterized by having porous structures formed in their interior. The porous structures as referred to herein have shapes such as spherical, ellipsoidal, flattened, rock-like, konpeito-like (pointed sugar candy ball), and irregular, of which the spherical and ellipsoidal shapes are preferable and the spherical shape is the most preferable.

The porous carbon particles according to the present invention have a number average particle diameter of 100 $\mu$m or less, preferably 50 $\mu$m or less, and more preferably 30 $\mu$m or less, as upper limit. As lower limit, it is 0.1 $\mu$m or more, preferably 0.5 $\mu$m or more, more preferably 0.7 $\mu$m or more, and particularly preferably 1 $\mu$m or more. The handleability deteriorates if the number average particle diameter is too small while if it is too large, the particles can act as foreign objects when applied over a thin membrane-like material such as film, both of which are unpreferable.

Here, the number average particle diameter of the porous carbon particles is defined as the arithmetic average determined by observing 100 particles randomly selected from scanning electronic microscope photographs, measuring their diameters, and making calculations by Equation (1) given below. Note that if a particle observed in a photograph is not perfectly circular, its major axis is taken as its diameter.

The particle diameter distribution of the porous carbon particles according to the present invention can be represented by the particle diameter distribution index, and their particle diameter distribution index is in the range of 1.0 to 2.0, preferably 1.0 to 1.8, more preferably 1.0 to 1.5, and particularly preferably 1.0 to 1.3. A particle diameter distribution index out of these ranges is not preferable because in that case, the particles will be low in flowability and gap-filling capability for resin and other materials. Here, the particle diameter distribution index is calculated by Equation (3) given below from the ratio of the volume average particle diameter to the number average particle diameter. The volume average particle diameter is determined by observing 100 particles randomly selected from scanning electronic microscope photographs, measuring their diameters, and making calculations by Equation (2) given below. Here, if a particle is not perfectly circular, its major axis is taken as its diameter.

[Mathematical formula 5]

$$Dn = \frac{\sum_{i=1}^{n} Ri}{n} \quad (1)$$

$$Dv = \frac{\sum_{i=1}^{n} Ri^{4}}{\sum_{i=1}^{n} Ri^{3}} \quad (2)$$

$$PDI = \frac{Dv}{Dn} \quad (3)$$

Here, Ri, n, and Dn denote the particle diameter of a particular particle, number of measurements (100), and number average particle diameter, respectively.

The porous carbon particles according to the present invention are also characterized by having a nearly perfect spherical shape. The spherical shape as referred to herein can be evaluated in terms of sphericity, and the particles have a sphericity of 80 or more, preferably 85 or more, more preferably 90 or more, particularly preferably 92 or more, still more preferably 95 or more, and most preferably 99 or more. A sphericity of less than 80 is not preferable because in that case, the gap-filling capability for resin or other materials will deteriorate. The sphericity as referred to herein is determined by observing particles by scanning electronic microscopy, measuring their major and minor axes, and making calculations by Equation (4) from their averages over randomly selected 30 particles.

[Mathematical formula 6]

$$\text{Sphericity} = \frac{\sum_{i=1}^{n} (\text{minor axes} / \text{major axes})}{n} \times 100 \quad (4)$$

Here, n denotes the number of measurements, which is equal to 30.

"Being porous" as referred to herein means that one or more holes exist in each of the porous carbon particles.

For the present invention, the pore parts in porous particles may have a size appropriately selected for particular uses. Commonly, the size of pore parts in porous particles is preferably in the range of 0.01 to 10 μm depending on the particle diameter of the porous carbon particles. It is preferably 0.01 to 1 μm, more preferably 0.01 to 0.5 μm, and most preferably 0.01 to 0.25 μm in order to making it possible to prevent a decrease in the density of the porous carbon particles and improve the specific surface at the same time. Here, the major axis of hollow structures is determined by fixing porous carbon particles with epoxy resin or the like, preparing ultrathin sections for electronic microscope observation, and measuring the size of 100 pore structures in randomly selected porous carbon particles by scanning microscopy. If such observation is difficult, a transmission microscope is used for measurement. If the porous structures are not perfectly spherical, their major axis is measured.

There are no specific limitations on the specific surface of the porous carbon particles, and an appropriate specific surface may be adopted arbitrarily.

The porous carbon particles according to the present invention may contain pore structures with an average pore diameter of 0.01 μm or less. For their production, generally known methods may be used, such as, for instance, the gas activation method in which carbon particles are heated at about 800°C in a gas atmosphere such as water vapor and carbon dioxide, and the chemical activation method in which carbon particles are heated at about 800°C with a chemical such as potassium hydroxide.

The porous carbon particles according to the present invention preferably have a degree of graphitization of

1.0 or more, more preferably 1.2 or more. An excessively small degree of graphitization is not preferable because in that case, the graphite content in the carbon particles will be too low and the electric conductivity and heat conductivity, for instance, will tend to deteriorate. There are no specific limitations on the upper limit, but it is commonly about 3.0. Here, the degree of graphitization is defined as the ratio (B)/(A) where (A) and (B) represent the height of Raman shift (A) (D-band attributed to defects in amorphous carbon and graphite) appearing at about 1,350$\pm$100 $cm^{-1}$ and the height of Raman shift (B) (G-band attributed to graphite) appearing at about 1,590$\pm$100 $cm^{-1}$ in Raman spectra observed by Raman spectroscopic analysis using laser with a wavelength of 532 nm.

The porous carbon particles according to the present invention may contain metal in order to develop a specific function. For the metal, any metal may be adopted if it can serve to develop an intended function, and the usable ones include alkali metals, alkaline earth metals, transition metals, light metals, lanthanoids, and actinoids, of which transition metals are preferable.

Specifically, they include alkali metals such as lithium, sodium, potassium, rubidium, cesium, francium; alkaline earth metals such as beryllium, magnesium, calcium, strontium, barium, and radium; transition metals such as scandium, yttrium, lanthanum, actinium, titanium, zirconium, hafnium, vanadium, niobium, tantalum, chrome, molybdenum, tungsten, manganese, technetium, rhenium, iron, ruthenium, osmium, cobalt, rhodium, iridium, nickel, palladium, platinum, copper, silver, gold, zinc, cadmium, and mercury; light metals such as aluminum, gallium, indium, thallium, silicon, germanium, tin, lead, antimony, bismuth, and polonium; and others such as lanthanoids and actinoids.

These metals may be elemental metals, salts thereof, or oxides thereof. Two or more thereof may be contained.

The preferable ones include magnesium, calcium, titanium, zirconium, niobium, tantalum, chrome, molybdenum, tungsten, manganese, rhenium, iron, ruthenium, osmium, cobalt, rhodium, iridium, nickel, palladium, platinum, copper, silver, gold, zinc, indium, and silicon, as well as salts and oxides thereof, of which titanium, chrome, manganese, iron, cobalt, nickel, copper, and zinc, as well as salts, oxides, and organometallic complexes thereof are more preferable.

The salts that constitute these metal salts may be in the form of a chloride salt, bromide salt, iodide salt, sulfonate salt, nitrate, phosphate, borate, perchlorate, carbonate, quaternary ammonium salt, alkoxy salt, carboxy salt, boron tetrafluoride salt, or acetylacetonate salt. In particular, the preferable ones include chloride salt, carbonate, alkoxy salt, carboxyl salt, and acetylacetonate salt.

The organometallic complexes include, for instance, phthalocyanine complexes, Schiff base complexes, and bipyridine complexes.

Described below is the carbon particle production process according to the present invention.

The carbon particles, metal-containing carbon particles, and porous carbon particles according to the present invention are produced by preparing fine particles using a common technique where polymer A, which is to form particles, and polymer B, which is a dissimilar polymer of a specific type (which may be hereinafter referred to as dissimilar polymer B or polymer B) are mixed in an organic solvent and precipitated with a poor solvent, followed by carbonizing the fine particles by calcination.

The high-quality carbon particles with a narrow particle diameter distribution according to the present invention can be produced by carbonizing particles of at least one synthetic polymer selected from the group of a polyolefin based copolymer, polyacrylonitrile based copolymer consisting of an acrylonitrile based monomer and a hydrophilic vinyl monomer, polyacrylamide based polymer, polyvinyl acetate based polymer, polyvinyl chloride based polymer, polyvinylidene chloride based polymer, polyamide, polyarylene ether, polyarylene sulfide, polysulfone, polyether ketone, polyether ether ketone, polyarylate, polyamide-imide, and polyimide that have a narrow particle diameter distribution.

Of these, polyacrylonitrile based copolymer consisting of an acrylonitrile based monomer and a hydrophilic vinyl monomer, polyamide, polyarylene ether, polyarylene sulfide, polysulfone, polyether ketone, polyether ether ketone, polyarylate, polyamide-imide, and polyimide are preferable because they serve to increase the degree of graphitization and produce higher-quality carbon particles, and polyacrylonitrile based copolymer consisting of an acrylonitrile based monomer and a hydrophilic vinyl monomer, polyamide-imide, polyetherimide, and polyimide are particularly preferable, of which polyacrylonitrile based copolymer consisting of an acrylonitrile based monomer and a hydrophilic vinyl monomer, and polyamide-imide are the most preferable, because their particles will not suffer from significant fusion during carbonization.

In the case of polyamide-imide, although details have not yet been clarified sufficiently, it serves to produce high-quality carbon particles, metal-containing carbon particles, and porous carbon particles probably because the cyclic imide structure and amide structure existing in their polymer chains can be easily carbonized and cyclized during the carbonizing step, and any appropriate particle production method may be used without being limited to the above-mentioned one. However, the use of the above-mentioned production method is preferable.

Examples of the above polyolefin based copolymer include polyethylene, polypropylene, and copolymers thereof.

Examples of the copolymerization units for these copolymers include cyclopentene, dicyclopentadiene and derivatives thereof, cyclohexene, and dicyclohexadiene and derivatives thereof.

With respect to the polyacrylonitrile based copolymer, a polyacrylonitrile based copolymer consisting of an

acrylonitrile based monomer and a hydrophilic vinyl monomer is used as described above. An acrylonitrile based monomer is represented by general formula (1) given below.

[Chemical compound 1]

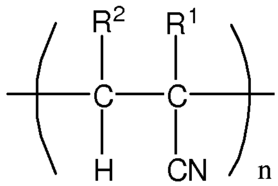

(1)

In the above formula, $R^1$ and $R^2$ represent any one selected from the group of hydrogen atom and a substituent group with a carbon number of 1 to 5 such as alkyl group, halogen group, hydroxyl group, alkoxyl group, ester group, carboxyl group, and sulfonic group. They may be identical or different.

Of these, preferable ones include hydrogen atom, methyl group, ethyl group, and isopropyl group, of which more preferable are hydrogen atom, methyl group, and ethyl group, and particularly preferable are hydrogen atom and methyl group. Examples of said acrylonitrile based monomer include (meth)acrylonitrile and chloroacrylonitrile. These may be used singly or as a mixture of two or more thereof. The most preferable is acrylonitrile.

Examples of said hydrophilic vinyl monomer include unsaturated monomers in which one or more of the following are substituted: hydroxyl group, carboxyl group, amino group, amide group, sulfonic acid group and phosphoric acid group. Specifically, they include unsaturated monomers substituted with hydroxyl groups such as hydroxyethyl (meth) acrylate, hydroxypropyl (meth)acrylate, and polyethylene glycol mono(meth)acrylate; unsaturated monomers, and salts thereof, substituted with carboxylic acid groups such as (meth)acrylic acid, itaconic acid, crotonic acid, fumaric acid, maleic acid, citraconic acid, methaconic acid, glutaric acid, tetrahydrophthalic acid, isocrotonic acid, nadic acid, butene tricarboxylic acid, monobutyl fumarate, monoethyl maleate, monomethyl itaconate, and p-vinylbenzoic acid; unsaturated monomers substituted with tertiary amino groups or quaternary ammonium salts such as dimethylaminoethyl (meth) acrylate, diethylaminoethyl (meth)acrylate, vinyl pyrolidone, N-methylvinylpyridnium chloride, (meth)allyl triethyl ammonium chloride, and 2-hydroxy-3-(metha)acryloyloxy propyl trimethyl ammonium chloride; unsaturated monomers substituted with amide groups such as (meth)acrylamide, N-hydroxyalkyl (meth)acrylamide, N-alkyl (meth)acrylamide, N, N-dialkyl (meth)acrylamide, and vinyl lactam; unsaturated monomers substituted with sulfonic acid groups such as vinyl sulfonic acid, methyl vinyl sulfonic acid, styrene sulfonic acid, (meth)acrylsulfonic acid, ethyl (meth)acrylate-2-sulfonate, and 2-acrylamide-2-hydroxypropane sulfonic acid; and unsaturated monomers substituted with phosphoric acid groups such as propyl (meth)acrylate-3-chloro-2-phosphate, ethyl (meth)acrylate-2-phosphate, 3-allyloxy-2-hydroxypropane phosphoric acid. Being highly effective in depressing fusion of particles during calcination, unsaturated monomers substituted with one or more carboxyl groups or amide groups are preferable, and (meth)acrylic acid, itaconic acid and acrylamide are particularly preferable. These may be used singly or as a mixture of two or more thereof.

With respect to the quantity of hydrophilic vinyl monomers for copolymerization, it is preferable that they account for more than 0 to 25 parts by mass per 100 parts by mass of the acrylonitrile based monomer. The upper limit is preferably 20 parts by mass, more preferably 15 parts by mass, particularly preferably 10 parts by mass, and extremely preferably 5 parts by mass. The lower limit, on the other hand, is preferably 0.5 parts by mass, more preferably 1 part by mass, and still more preferably 2 parts by mass. If the quantity of hydrophilic vinyl monomers is out of these ranges, the degree of graphitization of carbon particles will tend to decrease.

The polyacrylonitrile based copolymer consisting of an acrylonitrile based monomer and a hydrophlic vinyl monomer to be used for the present invention may be copolymerized with other copolymerizable unsaturated monomers as long as it meets the above quantity requirements. These may be polyfunctional monomers.

Specific examples of said other copolymerizable unsaturated monomers include unsaturated carboxylic acid alkyl ester based monomers such as methyl (meth)acrylate, ethyl (meth)acrylate, n-propyl (meth)acrylate, n-butyl (meth) acrylate, t-butyl (meth)acrylate, n-hexyl (meth)acrylate, cyclohexyl (meth)acrylate, chloromethyl (meth)acrylate, 2-chloroethyl (meth)acrylate, 2-hydroxyethyl (meth)acrylate, 3-hydroxypropyl (meth)acrylate, 2,3,4,5,6-pentahydroxyhexyl (meth)acrylate, 2,3,4,5-tetrahydroxypentyl (meth)acrylate, phenyl (meth)acrylate, benzyl (meth)acrylate; aromatic vinyl based monomers such as styrene, $\alpha$-methyl styrene, 1-vinylnaphthalene, 3-methylstyrene, 4-propylstyrene, 4-cyclohexyl styrene, 4-dodecyl styrene, 2-ethyl-4-benzylstyrene, 4-(phenylbutyl)styrene, and halogenated styrene; and conjugated diene based monomers such as butadiene, isoprene, 2,3-dimethyl butadiene, 2-methyl-3-ethylbutadiene, 1,3-pentadiene, 3-methyl-1,3-pentadiene, 2-ethyl-1,3-pentadiene, 1,3-hexadiene, 2-methyl-1,3-hexadiene, 3,4-dimethyl-1,3-hexadiene, 1,3-heptadiene, 3-methyl-1,3-heptadiene, 1,3-octadiene, cyclopentadiene, chloroprene, and myrcene. These

may be used singly or as a mixture of two or more thereof. In particular, methyl (meth)acrylate, ethyl (meth)acrylate, butyl (meth)acrylate, t-butyl (meth)acrylate, 2-hydroxyethyl (meth)acrylate, styrene, and preferable, and methyl (meth) acrylate and ethyl (meth)acrylate are particularly preferable.

Said other copolymerizable polyfunctional monomers are those containing two or more carbon-carbon double bonds in their molecules, and examples include, for instance, allyl (meth)acrylate, methallyl (meth)acrylate, allyl cinnamate, methallyl cinnamate, diallyl maleate, diallyl phthalate, diallyl terephthalate, diallyl isophthalate, divinylbenzene, ethylene di(meth)acrylate, butanediol di(meth)acrylate, hexanediol di(meth)acrylate, trimethylolpropane di(meth)acrylate, trimethylolpropane tri(meth)acrylate, pentaerythritol tri(meth)acrylate, pentaerythritol tetra(meth)acrylate, polyethylene glycol di(meth)acrylate, and polypropylene glycol di(meth)acrylate. These may be used singly or as a mixture of two or more thereof. In particular, allyl (meth)acrylate, diallyl maleate, diallyl phthalate, divinylbenzene, ethylene di(meth) acrylate, polyethylene glycol di(meth)acrylate, and polypropylene glycol di(meth)acrylate are preferable, and allyl (meth) acrylate, diallyl maleate, divinylbenzene, and ethylene di(meth)acrylate are particularly preferable.

With respect to the copolymer composition, said other copolymerizable unsaturated monomers account for 0 to 25 parts by mass, preferably 0 to 15 parts by mass, and more preferably 0 to 5 parts by mass, per 100 parts by mass of the acrylonitrile based monomer. If said other copolymerizable monomers account for more than 25 parts by mass in the copolymer, the degree of graphitization of carbon particles will tend to decrease, which requires caution.

In the case where hydrophilic vinyl monomers along with two or more other copolymerizable monomers as arbitrary components are used as comonomers for copolymerization with said acrylonitrile, the preferable combinations include methyl (meth)acrylate and (meth)acrylamide, methyl (meth)acrylate and itaconic acid, methyl (meth)acrylate and (meth)acrylic acid, (meth)acrylamide and (meth)acrylic acid, (meth)acrylamide and itaconic acid, and itaconic acid and (meth)acrylic acid.

Examples of said polyamide include polyamides that can be produced through condensation polymerization of (1) lactams with a 3- or more-membered ring, (2) polymerizable aminocarboxylic acids, (3) combination of dibasic acids and diamines, (4) salts thereof, and (5) mixtures thereof.

Specifically, they include polycaproamide (nylon 6), polyhexamethylene adipamide (nylon 66), polypentamethylene adipamide (nylon 56), polyhexamethylene sebacamide (nylon 610), polyundeca amide (nylon 11), polydodeca amide (nylon 12), and polyhexamethylene terephthalamide (nylon 6T), as well as amorphous polyamides such as copolymers of 3,3'-dimethyl-4,4'-diaminodicyclohexyl methane, isophthalic acid, and 12-aminododecanoic acid (for instance, Grilamide (registered trademark) TR55, supplied by Emser Werke, Inc.), copolymers of 3,3'-dimethyl-4,4'-diaminodicyclohexyl methane and dodeca diacid (for instance, Grilamide (registered trademark) TR90, supplied by Emser Werke, Inc.), mixtures of a copolymer of 3,3'-dimethyl-4,4'-diaminodicyclohexyl methane, isophthalic acid, and 12-aminododecanoic acid, and a copolymer of 3,3'-dimethyl-4,4'-diaminodicyclohexyl methane and dodeca diacid (for instance, Grilamide (registered trademark) TR70LX, supplied by Emser Werke, Inc.), and copolymers of 4,4'-diaminodicyclohexyl methane and dodeca diacid (for instance, TROGAMID (registered trademark) CX7323, supplied by Degussa AG).

Said polyarylene ether is a polymer consisting of aryl groups connected by ether bonds and has a structure as represented by general formula (2).

[Chemical compound 2]

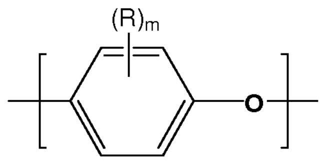

(2)

Here, the aromatic ring may or may not have substituent group R, and the number, m, of the substituent groups contained is 1 or more and 4 or less. Preferred substituent groups include saturated hydrocarbon groups with a carbon number of 1 to 6 such as methyl group, ethyl group, propyl group; unsaturated hydrocarbon groups such as vinyl group and allyl group; halogen groups such as fluorine atom, chlorine atom, and bromine atom; and others such as amino group, hydroxyl group, thiol group, carboxyl group, carboxyaliphatic hydrocarbon ester group.

Examples of said polyarylene ether include poly(2,6-dimethyl phenylene ether).

Said polyarylene sulfide is a polymer consisting of aryl groups connected by sulfide bonds, and has a structure as represented by general formula (3).

[Chemical compound 3]

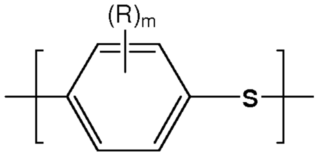

(3)

Here, the aromatic ring may or may not have substituent group R, and the number, m, of the substituent groups contained is 1 or more and 4 or less. Such substituent groups include saturated hydrocarbon groups such as methyl group, ethyl group, propyl group; unsaturated hydrocarbon groups such as vinyl group and allyl group; halogen groups such as fluorine atom, chlorine atom, and bromine atom; and others such as amino group, hydroxyl group, thiol group, carboxyl group, carboxyaliphatic hydrocarbon ester group. The paraphenylene sulfide unit in general formula (3) given above may be replaced with a methaphenylene unit, orthophenylene unit, or copolymer thereof.

Examples of said polyarylene sulfide include polyphenylene sulfide.

Preferable examples of said polysulfone include those having a structure as represented by general formula (4).

[Chemical compound 4]

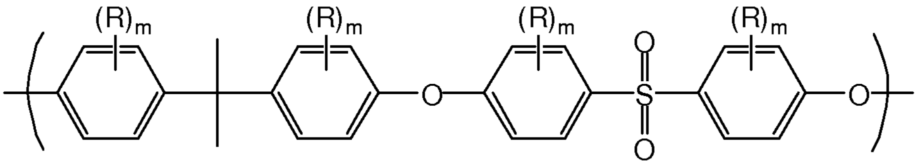

(4)

(In the formula, R represents an alkyl group with a carbon number of 1 to 6 or an aryl group with a carbon number of 6 to 8, and m represents an integer of 0 to 3.)

Said polyetherketone is a polymer having an ether bond and a carbonyl group. Specifically, preferred examples include those having a structure as represented by general formula (5).

[Chemical compound 5]

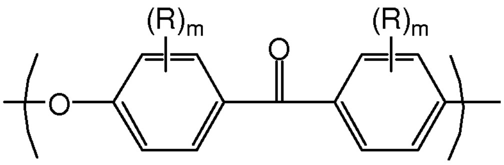

(5)

(In the formula, R represents an alkyl group with a carbon number of 1 to 6 or an aryl group with a carbon number of 6 to 8, and m represents an integer of 0 to 3.)

Of the various polyetherketones, those having a structure as represented by general formula (6) are referred to as polyether ether ketones.

[Chemical compound 6]

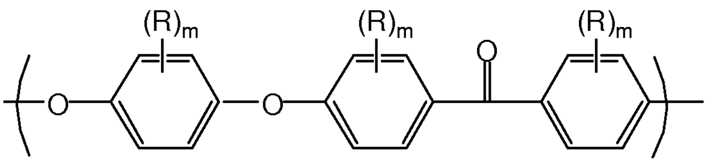

(6)

(In the formula, R represents an alkyl group with a carbon number of 1 to 6 or an aryl group with a carbon number of 6 to 8, and m represents an integer of 0 to 3.)

Said polyarylate is a polyester consisting of an aromatic polyhydric alcohol and an aromatic carboxylic acid, or more specifically a copolymer of either a bisphenol A or a bisphenol F with an aromatic dicarboxylic acid, and its examples include bisphenol A /terephthalic acid, bisphenol A / isophthalic acid, bisphenol A / terephthalic acid /isophthalic acid, bisphenol F / terephthalic acid, bisphenol F / isophthalic acid, and bisphenol F / terephthalic acid / isophthalic acid, such as, for instance, Upolymer supplied by Unitika Ltd.

Said polyamide-imide is a polymer having an imide bond and an amide bond in its polymer backbone chain, and may have a structure as represented by general formula (7).

[Chemical compound 7]

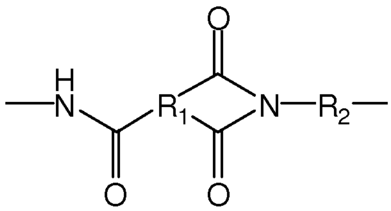

(7)

(In the formula, $R_1$ and $R_2$ independently represent an aromatic or an aliphatic hydrocarbon group, and may have, in its interior, a structure containing an ether bond, thioether bond, carboxyl group, halogen bond, or amide bond.)

Said polyamide-imide preferably contains an aromatic hydrocarbon group in its backbone chain, and $R_1$ and $R_2$ are preferably an aromatic hydrocarbon group because higher-quality carbon particles can be produced.

Specifically, its examples include copolymers of trimellitic anhydride and phenylene diamine or a derivative thereof, copolymers of trimellitic anhydride and 4,4'-diaminobiphenyl or a derivative thereof, copolymers of trimellitic anhydride and o-toluidine or a derivative thereof, copolymers of trimellitic anhydride and 4,4'-diaminodiphenyl ether or a derivative thereof, copolymers of trimellitic anhydride and 4,4'-methylene dianiline or a derivative thereof, copolymers of trimellitic anhydride and 4,4'-isopropylidene dianiline or a derivative thereof, trimellitic anhydride and hexamethylene diamine or a derivative thereof, copolymers of trimellitic anhydride and pentamethylene diamine or a derivative thereof, copolymers of trimellitic anhydride and 4,4'-diaminodicyclohexy methylene or a derivative thereof.

The copolymers as listed above may contain, in its structure, a halogen group or a hydrocarbon group with a carbon number of 1 to 4 in the form of substituent group.

Said polyimide is a polymer containing an imide bond, and commonly has a structure as represented by general formula (8).

[Chemical compound 8]

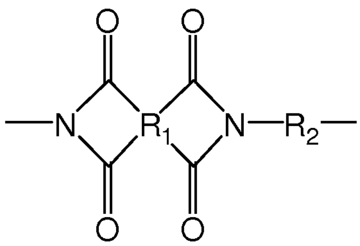

(8)

(In the formula, $R_1$ and $R_2$ represent an aromatic or an aliphatic hydrocarbon group, and may have, in its interior, a structure containing an ether bond, thioether bond, carboxyl group, halogen bond, or amide bond.)

For the present system, in particular, thermoplastic polyimide is preferable, and its specific examples include condensation polymers of 1,2,4,5-benzene tetracarboxylic anhydride and 4,4'-bis(3-aminophenyloxy)biphenyl and condensation polymers of 3,3',4,4'-biphenyl tetracarboxylic anhydride and 1,3-bis(4-aminophenyloxy)benzene.

For the present invention, the synthetic resins listed above have a weight average molecular weight in the range of 1,000 to 100,000,000, preferably 1,000 to 10,000,000, more preferably 5,000 to 1,000,000, particularly preferably 10,000 to 500,000, and most preferably 10,000 to 100,000.

The weight average molecular weight as referred to herein is defined as a weight average molecular weight measured by gel permeation chromatography (GPC) using dimethyl formamide as solvent and converted in terms of polystyrene.

Tetrahydrofuran is used if measurement is impossible with dimethyl formamide, hexafluoroisopropanol is used if it is still impossible, and 2-chloronaphthalene is used if measurement is still impossible with hexafluoroisopropanol.

The synthetic polymer particles used for the present invention have a number average particle diameter of 100 μm or less, preferably 50 μm or less, and more preferably 30 μm or less, as upper limit. As lower limit, it is 0.1 μm or more, preferably 0.5 μm or more, more preferably 0.7 μm or more, and particularly preferably 1 μm or more. The handleability deteriorates if the number average particle diameter is too small while the resulting carbon particles will increase in diameter, leading to a decrease in the dispersibility as filler in resin etc. if it is too large, both of which are unpreferable.

The number average particle diameter is determined by observing 100 particles randomly selected from scanning electronic microscope photographs, measuring their diameters, and making calculations by Equation (1) given below. Here, if a particle is not perfectly circular, its major axis is taken as its diameter.

The particle diameter distribution of the synthetic polymer particles can be represented by their particle diameter distribution index, and it is in the range of 1.0 to 2.0, preferably 1.0 to 1.8, more preferably 1.0 to 1.5, and particularly preferably 1.0 to 1.3. A particle diameter distribution index out of these ranges is not preferable because in that case, it will be impossible to produce carbon particles with a narrow particle diameter distribution. Here, the particle diameter distribution index is calculated by Equation (3) given below from the ratio of the volume average particle diameter to the number average particle diameter. The volume average particle diameter is determined by observing 100 particles randomly selected from scanning electronic microscope photographs, measuring their diameters, and making calculations by Equation (2) given below. Here, if a particle is not perfectly circular, its major axis is taken as its diameter.

[Mathematical formula 7]

$$Dn = \frac{\sum_{i=1}^{n} Ri}{n} \qquad (1)$$

$$Dv = \frac{\sum_{i=1}^{n} Ri^{4}}{\sum_{i=1}^{n} Ri^{3}} \qquad (2)$$

$$PDI = \frac{Dv}{Dn} \qquad (3)$$

Here, Ri, n, Dn, Dv, and PDI denote the particle diameter of a particular particle, number of measurements (100), number average particle diameter, volume average particle diameter, and particle diameter distribution index, respectively.

The synthetic polymer particles used for the present invention have a sphericity of 80 or more, preferably 85 or more, more preferably 90 or more, still more preferably 92 or more, particularly preferably 95 or more, and most preferably 99 or more. As synthetic polymer particles with a high sphericity tend to provide carbon particles with a high sphericity, a sphericity in said range is preferable because in that case, perfectly spherical carbon particles can be produced easily. Here, the sphericity is determined by observing particles by scanning electronic microscopy, measuring their major and minor axes, and making calculations from their average over randomly selected 30 particles by Equation (4).

[Mathematical formula 8]

$$\text{Sphericity} = \frac{\sum_{i=1}^{n} (\text{minor axes} / \text{major axes})}{n} \times 100 \qquad (4)$$

Here, n denotes the number of measurements, which is equal to 30.

Synthetic polymer particles as described above should be produced by dissolving and mixing a polymer to be processed into particles (hereinafter, polymer A) and a dissimilar polymer that can dissolve in a poor solvent for polymer A, which is referred to as polymer B, in an organic solvent, to form an emulsion in a two-phase separated system consisting of a solution phase composed mainly of polymer A (which may be hereinafter referred to as the polymer A solution phase) and a solution phase composed mainly of polymer B (which may be hereinafter referred to as the polymer B solution phase), and bringing it into contact with a poor solvent for polymer A to precipitate polymer A.

In synthetic polymer particles thus produced, polymer chains will be oriented so that their folded state in the particles is thermodynamically stable, accordingly allowing cyclization reaction to proceed easily during carbonization, which is considered to be the major factor in rapid cyclization reaction and an improved degree of graphitization in carbonized particles.

The above method is described below more specifically.

Said "two-phase separated system produced by dissolving and mixing polymer A, polymer B, and an organic solvent and consisting of a solution phase composed mainly of polymer A and a solution phase composed mainly of polymer B", as mentioned above is a system that is separated into a solution phase mainly containing polymer A and a solution phase mainly containing polymer B when mixing polymer A, polymer B, and the organic solvent.

The use of a system that undergoes such phase separation serves to form an emulsion when it is mixed under phase-separating conditions to cause emulsification.

Note that whether or not the polymer dissolves in the above step is judged according to whether it is dissolved up to more than 1 mass% in the organic solvent at the temperature where this method is carried out, that is, the temperature where polymer A and polymer B are dissolved and mixed to produce a two-phase separated solution.

Concerning this emulsion, as the polymer A solution phase forms a dispersed phase while the polymer B solution phase forms a continuous phase, this emulsion is brought into contact with a poor solvent for polymer A so that polymer A is precipitated from the polymer A solution phase in the emulsion to form synthetic polymer particles of polymer A.

For this method, there are no specific limitations on the combination of polymer A, polymer B, an organic solvent to dissolve them, and a poor solvent for polymer A, as long as synthetic polymer particles as intended in this method are obtained.

Since the method is characterized by bringing polymer A into contact with a poor solvent to precipitate particles, it is preferable that the polymer is insoluble in poor solvents, more preferably insoluble in the poor solvents mentioned later, and particularly preferably insoluble in water.

Here, a water-insoluble polymer as referred to above has a water-solubility of 1 mass% or less, preferably 0.5 mass% or less, and more preferably 0.1 mass% or less.

Said polymer B may be a thermoplastic resin or a thermosetting resin, and it is preferably soluble in the organic solvent used for dissolving polymer A and also in the poor solvent for polymer A used for the method, in particular, more preferably soluble in said organic solvent and also in alcohol based solvents or water from the viewpoint of high industrial handleability, and still more preferably soluble in said organic solvent and also in methanol, ethanol, or water.

Specific examples of polymer B include poly(vinyl alcohol) (which may be either completely saponified type or partially saponified type poly(vinyl alcohol)), poly(vinyl alcohol-ethylene) copolymer (which may be either a completely saponified type or a partially saponified type poly(vinyl alcohol-ethylene) copolymer), polyvinyl pyrolidone, poly(ethylene glycol), sucrose fatty acid ester, poly(oxy ethylene fatty acid ester), poly(oxy ethylene lauric fatty acid ester), poly(oxy ethylene glycol monofatty acid ester), poly(oxy ethylene alkyl phenyl ether), poly(oxy alkyl ether), polyacrylic acid, sodium polyacrylate, polymethacrylic acid, sodium polymethacrylate, polystyrene sulfonic acid, sodium polystyrene sulfonate, polyvinyl pyrrolidinium chloride, poly(styrene-maleic acid) copolymer, aminopoly(acrylamide), poly(para-vinyl phenol), polyallyl amine, polyvinyl ether, polyvinyl formal, poly(acrylamide), poly(methacrylamide), poly(oxy ethylene amine), poly (vinyl pyrolidone), poly(vinyl pyridine), polyamino sulfone, polyethylene imine, and other synthetic resins; maltose, cellobiose , lactose, sucrose, and other disaccharides; cellulose, chitosan, hydroxyethyl cellulose, hydroxypropyl cellulose, methyl cellulose, ethyl cellulose, ethyl hydroxycellulose, carboxymethyl ethyl cellulose, carboxymethyl cellulose, sodium carboxymethyl cellulose, cellulose ester, and other cellulose derivatives; amylose and derivatives thereof, starch

and derivatives thereof, dextrin, cyclodextrin, sodium alginate and derivatives thereof, and other polysaccharides and derivatives thereof; and gelatin , casein, collagen, albumin, fibroin, keratin, fibrin, carrageenan, chondroitin sulfate, gum arabic, agar, protein, and others; of which preferable are poly(vinyl alcohol) (which may be either completely saponified type or partially saponified type poly(vinyl alcohol)), poly(vinyl alcohol-ethylene) copolymer (which may be either a completely saponified type or a partially saponified type poly(vinyl alcohol-ethylene) copolymer), polyethylene glycol, sucrose fatty acid ester, poly(oxy ethylene alkyl phenyl ether), poly(oxy alkyl ether), poly(acrylic acid), poly(methacrylic acid), carboxymethyl cellulose, hydroxyethyl cellulose, hydroxypropyl cellulose, methyl cellulose, ethyl cellulose, ethyl hydroxycellulose, carboxymethyl ethyl cellulose, carboxymethyl cellulose, sodium carboxymethyl cellulose, cellulose ester, other cellulose derivatives, and polyvinyl pyrolidone, more preferable being poly(vinyl alcohol) (which may be either completely saponified type or partially saponified type poly(vinyl alcohol)), poly(vinyl alcohol-ethylene) copolymer (which may be either a completely saponified type or a partially saponified type poly(vinyl alcohol-ethylene) copolymer), polyethylene glycol, carboxymethyl cellulose, hydroxyethyl cellulose, hydroxypropyl cellulose, methyl cellulose, ethyl cellulose, ethyl hydroxycellulose, carboxymethyl ethyl cellulose, carboxymethyl cellulose, sodium carboxymethyl cellulose, cellulose ester, other cellulose derivatives, and polyvinyl pyrolidone, and particularly preferable are poly(vinyl alcohol) (which may be either completely saponified type or partially saponified type poly(vinyl alcohol)), poly(ethylene glycol), carboxymethyl cellulose, hydroxyethyl cellulose, hydroxypropyl cellulose, methyl cellulose, ethyl cellulose, ethyl hydroxycellulose, other cellulose derivatives, and polyvinyl pyrolidone.

With respect to the molecular weight of polymer B, the polymer preferably has a weight average molecular weight in the range of 1,000 to 100,000,000, more preferably 1,000 to 10,000,000, still more preferably 5,000 to 1,000,000, and particularly preferably 10,000 to 500,000, and most preferably 10,000 to 100,000.

The weight average molecular weight as referred to herein is defined as a weight average molecular weight measured by gel permeation chromatography (GPC) using water as solvent and converted in terms of polyethylene glycol.

Dimethyl formamide is used if measurement is impossible with water, tetrahydrofuran is used if it is still impossible, and hexafluoroisopropanol is used if measurement is still impossible.

Said organic solvent to be used for dissolving polymer A and polymer B is an organic solvent that can dissolve polymer A and polymer B to be used and may be selected according to the types of these polymers.

Specific examples include pentane, hexane, heptane, octane, nonane, n-decane, n-dodecane, n-tridecane, tetradecane, cyclohexane, cyclopentane, and other aliphatic hydrocarbon based solvents; benzene, toluene, xylene, 2-methyl naphthalene, and other aromatic hydrocarbon based solvents; ethyl acetate, methyl acetate, butyl acetate, butyl propionate, butyl butyrate, and other ester based solvents; chloroform, bromoform, methylene chloride, carbon tetrachloride, 1,2-dichloroethane, 1,1,1-trichloroethane, chlorobenzene, 2,6-dichlorotoluene, hexafluoroisopropanol, and other halogenated hydrocarbon based solvents; acetone, methylethylketone, methylisobutylketone, methylbutylketone, and other ketone based solvents; methanol, ethanol, 1-propanol, 2-propanol, and other alcohol based solvents; N-methyl-2-pyrolidone (NMP), dimethyl sulfoxide (DMSO), N,N-dimethyl formamide (DMF), N,N-dimethyl acetamide (DMA), propylene carbonate, trimethyl phosphate, 1,3-dimethyl-2-imidazolidinone, sulfolane, and other aprotic polar solvents; formic acid, acetic acid, propionic acid, butyric acid, lactic acid, and other carboxylic acid solvents; anisole, diethyl ether, tetrahydrofuran, diisopropyl ether, dioxane, diglyme, dimethoxy ethane, and other ether based solvents; 1-butyl-3-methyl imidazolium acetate, 1-butyl-3-methyl imidazolium hydrogen sulfate, 1-ethyl-3-imidazolium acetate, 1-ethyl-3-methyl imidazolium thiocyanate, and other ionic liquid; and mixtures thereof. Preferable are aromatic hydrocarbon based solvents, aliphatic hydrocarbon based solvents, halogenated hydrocarbon based solvents, alcohol based solvents, ether based solvents, aprotic polar solvents, and carboxylic acid solvents, more preferable being water-soluble alcohol based solvents, aprotic polar solvents, and carboxylic acid solvents, extremely preferable are aprotic polar solvents, and carboxylic acid solvents, and most preferable being N-methyl-2-pyrolidone, dimethyl sulfoxide, N,N-dimethyl formamide, N,N-dimethyl acetamide, propylene carbonate, formic acid, and acetic acid because they are so widely available and able to dissolve a variety of polymers that they can suit various polymers used as polymer A and also because they can mix uniformly with water, alcohol based solvents, and other solvents listed later that can be used preferably as said poor solvent.

These organic solvents may be used as a mixture of two or more thereof or in combination with others, but from the viewpoint of producing particles with relatively small particle diameters and a narrow particle diameter distribution and avoiding troublesome work in a separation step for recycling of used solvents to reduce the process load for production, it is preferable to use a single organic solvent and more preferable to use a single organic solvent that can dissolve both polymer A and polymer B.

Said poor solvent for polymer A used for the present method is defined as a solvent that does not dissolve polymer A. Here, for said solvent that does not dissolve polymer A, the polymer has a solubility of 1 mass% or less, preferably 0.5 mass% or less, and more preferably 0.1 mass% or less.

The present method uses a poor solvent for polymer A, and it is preferable that the poor solvent can dissolve polymer B in addition to being a poor solvent for polymer A. In such a case, polymer particles constituted of polymer A can be precipitated efficiently. It is also preferable that the solvent to dissolve polymer A, polymer B and the poor solvent

for polymer A can mix homogeneously.

The type of poor solvent to be used effectively for the present method varies depending on the type of polymer A used or desirably depending on the type of both polymers A and B used, but specifically, it may be one solvent selected from the following: pentane, hexane, heptane, octane, nonane, n-decane, n-dodecane, n-tridecane, tetradecane, cyclohexane, cyclopentane,and other aliphatic hydrocarbon based solvents; benzene, toluene, xylene, 2-methyl naphthalene, and other aromatic hydrocarbon based solvents; ethyl acetate, methyl acetate, butyl acetate, butyl propionate, butyl butyrate, and other ester based solvents; chloroform, bromoform, methylene chloride, carbon tetrachloride, 1,2-dichloroethane, 1,1,1-trichloroethane, chlorobenzene, 2,6-dichlorotoluene, hexafluoroisopropanol, and other halogenated hydrocarbon based solvents; acetone, methyethylketone, methylisobutylketone, methylbutylketone, and other ketone based solvents; methanol, ethanol, 1-propanol, 2-propanol, and other alcohol based solvents; dimethyl sulfoxide, N,N-dimethyl formamide, N,N-dimethyl acetamide, trimethyl phosphate, N-methyl-2-pyrolidone, 1,3-dimethyl-2-imidazolidinone, sulfolane, and other aprotic polar solvents; formic acid, acetic acid, propionic acid, butyric acid, lactic acid, and other carboxylic acid solvents; anisole, diethyl ether, tetrahydrofuran, diisopropyl ether, dioxane, diglyme, dimethoxy ethane, and other ether based solvents; and water.

From the viewpoint of efficient production of particles from polymer A, preferable ones include aromatic hydrocarbon based solvents, aliphatic hydrocarbon based solvents, alcohol based solvents, ether based solvents, and water, of which the most preferable are alcohol based solvent and water, and particularly preferable is water.

For the present method, synthetic polymer particles can be produced when polymer A, polymer B, an organic solvent that dissolves them, and a poor solvent for polymer A are appropriately selected and used in combination to allow polymer A to be precipitated efficiently.

Here, it is necessary for the liquid prepared by mixing and dissolving polymers A and B, and an organic solvent that dissolves them is phase-separated into two phases: a solution phase composed mainly of polymer A and a solution phase composed mainly of polymer B.

Note that the organic solvent used for the solution phase composed mainly of polymer A and the organic solvent used for the solution phase composed mainly of polymer B may be either identical or different, but it is preferable that they are substantially identical.

The conditions to develop a state of two-phase separation varies depending on types of polymers A and B, molecular weight of polymers A and B, type of the organic solvents, concentration of polymers A and B, and the temperature and pressure where the present method is to be carried out.

To produce conditions where phase separation easily takes place, it is preferable that the solubility parameter (which may be hereinafter referred to as SP value) of polymer A is largely different from that of polymer B.

Here, the difference in SP value is 1 $(J/cm^3)^{1/2}$ or more, more preferably 2 $(J/cm^3)^{1/2}$ or more, still more preferably 3 $(J/cm^3)^{1/2}$ or more, particularly preferably 5 $(J/cm^3)^{1/2}$ or more, and extremely preferably 8 $(J/cm^3)^{1/2}$ or more. Phase separation is caused easy if the SP value is in this range.

There are no specific limitations if both polymer A and polymer B can be dissolved in the organic solvent used, but the upper limit on the difference in SP value is preferably 20 $(J/cm^3)^{1/2}$ or less, more preferably 15 $(J/cm^3)^{1/2}$ or less, and still more preferably 10 $(J/cm^3)^{1/2}$ or less.

The SP value as referred to herein is calculated according to Fedor's estimation method and calculated based on cohesive energy density and molecular volume (hereinafter, sometimes referred simply to calculation method) ("SP-chi: Kiso • Oyo to Keisan Hoho (SP Value: Basics/Applications and Calculation Method), Hideki Yamamoto, Johokiko Co., Ltd, Mar. 31, 2005).

If the solubility parameter cannot be calculated by the estimation method, the SP value is calculated by the experiment method in which it is determined based on whether the polymer can dissolve in solvents with a known solubility parameter (hereinafter, sometimes referred to simply as experiment method) and used instead (Polymer Handbook Fourth Edition, J. Brand, Wiley, 1998).

Conditions to cause phase separation are set up according to a three-component phase diagram, which can be prepared based on a simple preliminary experiment to observe various states at different ratios of the three components of polymer A, polymer B and an organic solvent that dissolves them.

A phase diagram can be prepared by mixing and dissolving polymers A and B and a solvent at least 3 or more, preferably 5 or more, and more preferably 10 or more, appropriately selected proportions, leaving them to stand, and determining whether an interface develops, and conditions for phase separation are identified based on differentiation of regions where two phases are formed and those where only one phase is formed.

To determine whether a liquid is in a phase separation state, polymers A and B, and a solvent are mixed at an appropriate proportion under the temperature and pressure conditions where the present invention is to be implemented, and subsequently, polymers A and B are dissolved completely, stirred adequately after the dissolution, left to stand for 3 days, and observed whether macroscopic phase separation has occurred.

In the case where an adequately stable emulsion is formed, however, macroscopic phase separation may not take place even when the liquid is left to stand for 3 days. In that case, occurrence of phase separation is judged by

using an optical microscope or phase contrast microscope to observe whether microscopic phase separation takes place.

Phase separation occurs as a result of separation between the polymer A solution phase composed mainly of polymer A and the polymer B solution phase composed mainly of polymer B in the organic solvent. Here, the polymer A solution phase is the phase to which mainly polymer A is distributed while the polymer B solution phase is the phase to which mainly polymer B is distributed. Here, the polymer A solution phase and the polymer B solution phase each appear to have a volume ratio depending on the type and content of polymers A and B, respectively.

The contents of polymers A and B should, as a prerequisite, be such that a phase separation state can develop and also that they make the method industrially practicable and are as high as possible in the organic solvent, but they are preferably more than 1 mass% to 50 mass%, more preferably more than 1 mass% to 30 mass%, and still more preferably more than 2 mass% to 20 mass%.

With respect to the polymer A solution phase and the polymer B solution phase for the present method, the interfacial tension between the two phases is small since both phases are of an organic solvent, and this nature allows the resulting emulsion to be maintained stable, possibly serving to narrow the particle diameter distribution. In particular, this effect is noticeable when the polymer A phase and the polymer B phase are formed of the same organic solvent.

For the present method, the interfacial tension between the two phases are so small that the interfacial tension cannot be measured directly by the commonly used hanging-drop method in which measurements are made by adding a dissimilar solution to the solution, but the interfacial tension can be estimated from the surface tension of each phase in air. Denoting the surface tension of each phase in air by $r_1$ and $r_2$, the interfacial tension between them, $r_{12}$, is estimated as the absolute value of $r_{12} = r_1 - r_2$. Here, the value of $r_{12}$ is preferably in the range of more than 0 to 10 mN/m, more preferably more than 0 to 5 mN/m, still more preferably more than 0 to 3 mN/m, and particularly preferably more than 0 to 2 mN/m.

For the present method, the viscosity between the two phases has influence on the average particle diameter and particle diameter distribution, and the particle diameter distribution index tends to decrease with an decreasing viscosity ratio. If it is assumed that the viscosity ratio is defined as the ratio of the polymer A solution phase / the polymer B solution under the temperature conditions where the present invention is to be implemented, it is preferably in the range of 0.1 or more and 10 or less, more preferably in the range of 0.2 or more and 5 or less, still more preferably in the range of 0.3 or more and 3 or less, particularly preferably in the range of 0.5 or more and 1.5 or less, and extremely preferably in the range of 0.8 or more and 1.2 or less.

A phase-separated system thus obtained is used to produce synthetic polymer particles. A common reaction tank is used to produce particles. From the viewpoint of industrial practicability, the temperature range suitable for implementing the present invention is -50°C to 200°C, preferably -20°C to 150°C, more preferably 0°C to 120°C, still more preferably 10°C to 100°C, particularly preferably 20°C to 80°C, and most preferably 20°C to 50°C. From the viewpoint of industrial practicability, the pressure range suitable for implementing the present invention is from a reduced pressure to 100 atm, preferably 1 atm to 5 atm, more preferably 1 atm to 2 atm, and particularly preferably atmospheric pressure.

An emulsion is formed by mixing the components under such conditions to develop a phase-separated state.

Specifically, an emulsion is produced by applying a shear force to the resulting phase-separated solution.

In the emulsion formation step, an emulsion is produced in such a way that the polymer A solution phase forms particle-like droplets. It is commonly seen that when a solution is phase-separated, such an emulsion tends to form easily when the volume of the polymer B solution phase is larger than that of the polymer A solution phase, and the ratio of the volume of the polymer A solution phase to that of the total volume of the two phases is preferably 0.4 or less, more preferably in the range of 0.4 to 0.1. When preparing said phase diagram, an appropriate range can be set up if the volume ratio of each component is measured at the same time.

Synthetic polymer particles produced by the present production method are in the form of fine particles with a narrow particle diameter distribution, which is made possible because a highly uniform emulsion is formed in the emulsion formation step. This tendency is noticeable when a single solvent that can dissolve both polymers A and B is used. Thus, the use of a conventionally known stirring method is sufficiently effective to produce a shear force required for the emulsion formation, and examples of said method to be used for mixing include liquid phase stirring by stirring blades, stirring by a continuous biaxial stirrer, mixing by a homogenizer, and ultrasonic irradiation.

When agitating blades are used for stirring, in particular, the stirring speed is preferably 50 rpm to 1,200 rpm, more preferably 100 rpm to 1,000 rpm, still more preferably 200 rpm to 800 rpm, and particularly preferably 300 rpm to 600 rpm, depending on the shape of the agitating blades used.

The agitating blade may specifically be in the shape of propeller, paddle, flat paddle, turbine, double cone, single cone, single ribbon, double ribbon, screw, or helical ribbon, but it is not limited thereto as long as a sufficient shear force can be applied to the system. In addition, baffle boards etc. may be provided in the tank to ensure efficient stirring.

For formation of an emulsion, not only a stirrer but also other generally known tools such as emulsifier and dispersing machine may be used. Specific examples include Homogenizer (supplied by IKA), Polytron (supplied by Kinematica, Inc.), T. K. Autohomemixer (supplied by Tokushu Kika Kogyo Co., Ltd.), other batch-wise emulsifiers, Ebara

Milder (supplied by Ebara Corporation), T. K. Filmix, T. K. Pipeline Homomixer (supplied by Tokushu Kika Kogyo Co., Ltd.), Colloid Mill (supplied by Shinko-Pantec Co., Ltd.), Slusher, Trigonal Wet Grinder (supplied by Mitsui Miike Kakoki Co., Ltd.), ultrasonic homogenizers, and static mixers.

The resulting emulsion is then fed to a step for precipitating synthetic polymer particles.

To produce synthetic polymer particles, a poor solvent for polymer A is brought into contact with the emulsion produced in the above step to precipitate synthetic polymer particles having a diameter corresponding to that of the emulsion droplets.

Bringing the poor solvent and the emulsion into contact with each other may be achieved by pouring the emulsion in the poor solvent, and pouring the poor solvent into the emulsion, of which pouring the poor solvent into the emulsion is referable.

Here, there are no specific limitations on the method to be used to pour the poor solvent as long as synthetic polymer particles produced according to the present invention are obtained, and the useful methods include continuous dropping, divided addition, and on-step addition. In order to prevent widening in the particle diameter distribution and formation of bulky lumps exceeding 1,000 μm from being caused by coagulation, fusion, or coalescence of the emulsion at the time of adding the poor solvent, it is preferable to use continuous dropping and divided dropping, and it is most preferable to use continuous dropping to ensure industrially efficient implementation.

The duration of adding the poor solvent is 10 min or more and 50 hours or less, more preferably 30 min or more and 10 hours or less, and still more preferably 1 hour or more and 5 hours or less.

If the implementation duration is shorter than these ranges, widening in the particle diameter distribution and formation of bulky lumps may result from coagulation, fusion, or coalescence of the emulsion. An implementation duration longer than them is impractical from an industrial point of view.

Implementation in these ranges makes it possible to depress coagulation of particles during conversion from an emulsion into synthetic polymer particles, thereby providing synthetic polymer particles with a narrow particle diameter distribution.

Depending on the conditions of the emulsion, the quantity of the poor solvent to be added is preferably 0.1 part by mass to 10 parts by mass, more preferably 0.1 part by mass to 5 parts by mass, still more preferably 0.2 part by mass to 3 parts by mass, particularly preferably 0.2 part by mass to 1 part by mass, and most preferably 0.2 part by mass to 0.5 part by mass, per 1 part by mass of the total weight of the emulsion.

There are no specific limitations on the duration of contact between the poor solvent and the emulsion as long as it is sufficiently long for precipitation of synthetic polymer particles, but in order to cause precipitation adequately and ensure high productivity, it is 5 min to 50 hours, preferably 5 min or more and 10 hours or less, more preferably 10 min or more and 5 hours or less, particularly preferably 20 min or more and 4 hours or less, and extremely preferably 30 min or more and 3 hours or less, after the end of addition of the poor solvent.

From a dispersion liquid of synthetic polymer particles thus obtained, particulate powder can be recovered by solid-liquid separation using a generally known method such as filtration, decantation, vacuum filtration, compression filtration, centrifugal separation, centrifugal filtration, spray dry, acid precipitation, salt precipitation, and freeze coagulation.

The synthetic polymer particles obtained by solid-liquid separation is cleaned with a solvent or the like, as required, to remove impurities and so on attached or contained, and then purified. Preferable solvents to be used here include the poor solvents mentioned above, and more preferable are a solvent selected from the group of water, methanol, and ethanol, or a mixture of two or more thereof.

The resulting synthetic polymer particles may be dried to remove the residual solvent. The drying methods that can be used here include air drying, heat drying, vacuum drying, and freeze drying. The temperature used for heating is preferably lower than the glass transition temperature, and specifically, a temperature of 50 to 150°C is preferable.

The method according to the present invention is advantageous in that the organic solvent and polymer B that are separated in the solid-liquid separation step carried out to obtain synthetic polymer particles can be recycled to ensure their efficient use.

The solvent resulting from the solid-liquid separation step is actually a mixture of polymer B, organic solvent, and poor solvent. Removal of the poor solvent from the mixture allows the remaining solvent to be re-used for emulsion formation. For removing the poor solvent, generally known methods may be used, including, specifically, simple distillation, reduced pressure distillation, precision distillation, thin film distillation, extraction, and membrane separation, of which simple distillation, reduced pressure distillation, and precision distillation ate preferable.

Heat is applied to the system when performing distillation operations such as simple distillation and reduced pressure distillation, possibly accelerating the thermal decomposition of polymer B and organic solvents, and therefore, they are performed in an atmosphere with as low an oxygen content as possible, preferably in an inactive atmosphere. Specifically, they should be performed in an atmosphere of nitrogen, helium, argon, or carbon dioxide.

Methods to carbonize the resulting synthetic polymer particles are described below. The carbonizing step is carried out by heating the synthetic polymer particles in an inert gas atmosphere that does not react with the synthetic

polymer particles. It is preferable to use an inert gas such as helium, argon, and nitrogen.
The atmosphere used for heating may be either of a flow type or of a closed type, but a flow type atmosphere is preferable because the gas generated by heating can be removed. With respect to pressure, heating may be performed under compressed pressure, reduced pressure, or vacuum conditions, but commonly, it is performed under atmospheric pressure. When carried out under atmospheric pressure, heating is performed at a temperature of 400°C or more, preferably 800°C or more.

The degree of graphitization of the carbon particles tends to increase with an increasing heating temperature and an increasing duration during which the heating temperature is maintained. The heating temperature is preferably 400°C or more, more preferably 800°C or more, and most preferably 1,000°C or more. There are no specific limitations on the upper limit, but it is commonly 3,000°C and preferably 2,500°C.

The heating rate to reach the heating temperature is commonly 20°C/min or less, preferably 10°C/min or less, and more preferably 5°C/min or less. As the calcination temperature exceeds 20°C/min, the particles tend to undergo fusion.

Depending on the heating temperature, the heating-temperature holding duration is preferably 0.5 hour or more, more preferably 1 hour or more, still more preferably 5 hours or more, particularly preferably 10 hours or more, extremely considerably preferably 50 hours or more, as lower limit, and it is preferably 1,000 hours or less, more preferably 500 hours or less, and still more preferably 100 hours or less, as upper limit. With respect to the heating temperature, furthermore, the particles may be maintained at an appropriate temperature for an appropriate time, followed by further heating up to a desired heating temperature.

Said method may also be used in the case where the synthetic polymer particles are carbonized, but it is preferable to perform pre-treatment as described below because it serves to allow the synthetic polymer particles to form an oxidized structure and also serves to improve the carbonization yield and prevent fusion of particles.

An oxidized structure can be formed by heating the synthetic polymer particles in an oxidizing atmosphere. Said oxidizing atmosphere may contain oxygen, sulfur, or the like, but it is preferably an air atmosphere. There are no specific limitations on the heating temperature as long as an oxidized structure is formed, but it is 100 to 300°C, preferably 150 to 250°C. A heating temperature of less than 100°C is not preferable because in that case, the formation of an oxidized structure does not progress, while the synthetic polymer particles start to melt before an oxidized structure is formed if the temperature is above 300°C. The heating rate to reach the heating temperature is commonly 20°C/min or less, preferably 10°C/min or less, and more preferably 5°C/min or less. A calcination temperature of above 20°C/min is not preferable because it causes fusion of the particles. The heating temperature, furthermore, may be maintained for an appropriate time after heating up to a predetermined temperature. Depending on the heating temperature, the heating duration is 0.5 to 10 hours, preferably 0.5 to 5 hours, at the most. With respect to the heating temperature, furthermore, the particles may be maintained at an appropriate temperature for an appropriate time, followed by further heating up to a desired heating temperature.

The carbon particle production process according to the present invention serves to produce high-quality carbon particles with a narrow particle diameter distribution and a high degree of graphitization in a simple manner.

Described below is the production process for metal-containing carbon particles.

Synthetic polymer particles that contains metal (hereinafter, referred to as metal-containing synthetic polymer particles) can be produced by a process as described below.

Metal-containing polymer particles as described above can be produced by dissolving and mixing a polymer to be processed into particles (hereinafter, polymer A) and a dissimilar polymer that can dissolve in a poor solvent for polymer A, which is referred to as polymer B, in an organic solvent, to form an emulsion in a two-phase separated system consisting of a solution phase composed mainly of polymer A (which may be hereinafter referred to as polymer A solution phase) and a solution phase composed mainly of polymer B (which may be hereinafter referred to as polymer B solution phase), and bringing it into contact with a poor solvent for polymer A to precipitate polymer A.

For the present method, polymer A refers to a polymeric material (synthetic resin) that constitutes said synthetic polymer particles, and the high-quality metal-containing carbon particles with a narrow particle diameter distribution according to the present invention can be produced by carbonizing particles of at least one metal-containing synthetic polymer selected from the group of a polyolefin based copolymer, polyacrylonitrile based copolymer consisting of an acrylonitrile based monomer and a hydrophilic vinyl monomer, polyacrylamide based polymer, polyvinyl acetate based polymer, polyvinyl chloride based polymer, polyvinylidene chloride based polymer, polyamide, polyarylene ether, polyarylene sulfide, polysulfone, polyether ketone, polyether ether ketone, polyarylate, polyamide-imide, and polyimide that have a narrow particle diameter distribution.

Of these, metal-containing synthetic polymer particles produced by adding a metal component to polyacrylonitrile based copolymer consisting of an acrylonitrile based monomer and a hydrophilic vinyl monomer, polyamide, polyarylene ether, polyarylene sulfide, polysulfone, polyether ketone, polyether ether ketone, polyarylate, polyamide-imide, or polyimide are preferable because they serve to increase the degree of graphitization and produce higher-quality carbon particles, and metal-containing synthetic polymer particles produced by adding a metal component to polyacrylonitrile based copolymer consisting of an acrylonitrile based monomer and a hydrophilic vinyl monomer, polyamide-imide, polyetherimide, or polyimide are particularly preferable, of which polyacrylonitrile based copolymer consisting of an acrylonitrile based monomer and a hydrophilic vinyl monomer, and polyamide-imide are the most preferable, because their particles will not suffer from significant fusion when carbonized.

In the case of polyamide-imide, although details have not yet been clarified sufficiently, it serves to produce high-quality metal-containing carbon particles probably because the cyclic imide structure and amide structure existing in their polymer chains can be easily carbonized and cyclized during the carbonizing step, and any appropriate particle production technique may be used without being limited to the above-mentioned one.

A metal component as referred to herein falls in the category described above which encompasses salts thereof, oxides thereof, coordinated complexes thereof with organic compounds, and others having similar chemical properties, and they may be in the shape of particles, powder, liquid, gas, or cluster.

The above method is described in more detail below.

Said "two-phase separated system produced by mixing polymer A, polymer B, a metal component, and an organic solvent and consisting of a solution phase composed mainly of polymer A and a solution phase composed mainly of polymer B", as mentioned above is a system that is separated into a solution phase mainly containing polymer A and a solution phase mainly containing polymer B when mixing polymer A, polymer B, the metal component, and the organic solvent. Here, polymers A and B each can dissolve in an organic solvent to form the polymer A solution phase and the polymer B solution phase, but it does not matter whether they contain a dissolved metal component (hereinafter the same applies unless otherwise specified).

The use of a system that undergoes such phase separation serves to form an emulsion when it is mixed under phase-separating conditions to cause emulsification.

Note that whether or not the polymer dissolves in the above step is judged according to whether it is dissolved up to more than 1 mass% in the organic solvent at the temperature where this method is carried out, that is, the temperature where polymer A and polymer B are dissolved and mixed to produce a two-phase separated solution.

It is necessary for this emulsion to consist of a polymer A solution phase acting as a dispersed phase and a polymer B solution phase acting as a continuous phase, with a metal component existing in the polymer A solution, although the metal component may be distributed to both polymer A and polymer B solution phases.

When a poor solvent for polymer A is brought into contact with this emulsion, polymer A is precipitated from the polymer A solution phase in the emulsion to form metal-containing synthetic polymer particles composed of the metal component and polymer A.

For this method, there are no specific limitations on the combination of polymer A, polymer B, an organic solvent to dissolve them, a metal component and a poor solvent for polymer A, as long as metal-containing synthetic polymer particles as intended in this method are obtained.

Since the method is characterized by bringing polymer A into contact with a poor solvent to precipitate particles, it is preferable that the polymer is insoluble in poor solvents, more preferably insoluble in the poor solvents mentioned later, and particularly preferably insoluble in water.

Here, a water-insoluble polymer as referred to above has a water-solubility of 1 mass% or less, preferably 0.5 mass% or less, and more preferably 0.1 mass% or less.

Said polymer B may be a thermoplastic resin or a thermosetting resin, and it is preferably soluble in the organic solvent used for dissolving polymer A for the method and also in the poor solvent for polymer A, in particular, more preferably soluble in said organic solvent and also in alcohol based solvents or water from the viewpoint of high industrial handleability, and still more preferably soluble in said organic solvent and also in methanol, ethanol, or water.

Specific examples of polymer B include poly(vinyl alcohol) (which may be either completely saponified type or partially saponified type poly(vinyl alcohol)), poly(vinyl alcohol-ethylene) copolymer (which may be either a completely saponified type or a partially saponified type poly(vinyl alcohol-ethylene) copolymer), polyvinyl pyrolidone, poly(ethylene glycol), sucrose fatty acid ester, poly(oxy ethylene fatty acid ester), poly(oxy ethylene lauric fatty acid ester), poly(oxy ethylene glycol monofatty acid ester), poly(oxy ethylene alkyl phenyl ether), poly(oxy alkyl ether), polyacrylic acid, sodium polyacrylate, polymethacrylic acid, sodium polymethacrylate, polystyrene sulfonic acid, sodium polystyrene sulfonate, polyvinyl pyrrolidinium chloride, poly(styrene-maleic acid) copolymer, aminopoly(acrylamide), poly(para-vinyl phenol), polyallyl amine, polyvinyl ether, polyvinyl formal, poly(acrylamide), poly(methacrylamide), poly(oxy ethylene amine), poly (vinyl pyrolidone), poly(vinyl pyridine), polyamino sulfone, polyethylene imine, and other synthetic resins; maltose, cellobiose , lactose, sucrose, and other disaccharides; cellulose, chitosan, hydroxyethyl cellulose, hydroxypropyl cellulose, methyl cellulose, ethyl cellulose, ethyl hydroxycellulose, carboxymethyl ethyl cellulose, carboxymethyl cellulose, sodium carboxymethyl cellulose, cellulose ester, and other cellulose derivatives; amylose and derivatives thereof, starch and derivatives thereof, dextrin, cyclodextrin, sodium alginate and derivatives thereof, and other polysaccharides and derivatives thereof; and gelatin , casein, collagen, albumin, fibroin, keratin, fibrin, carrageenan, chondroitin sulfate, gum arabic, agar, protein, and others; of which preferable are poly(vinyl alcohol) (which may be either completely saponified type or partially saponified type poly(vinyl alcohol)), poly(vinyl alcohol-ethylene) copolymer (which may be either a

completely saponified type or a partially saponified type poly(vinyl alcohol-ethylene) copolymer), polyethylene glycol, sucrose fatty acid ester, poly(oxy ethylene alkyl phenyl ether), poly(oxy alkyl ether), poly(acrylic acid), poly(methacrylic acid), carboxymethyl cellulose, hydroxyethyl cellulose, hydroxypropyl cellulose, methyl cellulose, ethyl cellulose, ethyl hydroxycellulose, carboxymethyl ethyl cellulose, carboxymethyl cellulose, sodium carboxymethyl cellulose, cellulose ester, other cellulose derivatives, and polyvinyl pyrolidone, more preferable being poly(vinyl alcohol) (which may be either completely saponified type or partially saponified type poly(vinyl alcohol)), poly(vinyl alcohol-ethylene) copolymer (which may be either a completely saponified type or a partially saponified type poly(vinyl alcohol-ethylene) copolymer), polyethylene glycol, carboxymethyl cellulose, hydroxyethyl cellulose, hydroxypropyl cellulose, methyl cellulose, ethyl cellulose, ethyl hydroxycellulose, carboxymethyl ethyl cellulose, carboxymethyl cellulose, sodium carboxymethyl cellulose, cellulose ester, other cellulose derivatives, and polyvinyl pyrolidone, and particularly preferable are poly(vinyl alcohol) (which may be either completely saponified type or partially saponified type poly(vinyl alcohol)), poly(ethylene glycol), carboxymethyl cellulose, hydroxyethyl cellulose, hydroxypropyl cellulose, methyl cellulose, ethyl cellulose, ethyl hydroxycellulose, other cellulose derivatives, and polyvinyl pyrolidone.

With respect to the molecular weight of polymer B, the polymer preferably has a weight average molecular weight in the range of 1,000 to 100,000,000, more preferably 1,000 to 10,000,000, still more preferably 5,000 to 1,000,000, and particularly preferably 10,000 to 500,000, and most preferably 10,000 to 100,000.

The weight average molecular weight as referred to herein is defined as weight average molecular weight measured by gel permeation chromatography (GPC) using water as solvent and converted in terms of polyethylene glycol.

Dimethyl formamide is used if measurement is impossible with water, tetrahydrofuran is used if it is still impossible, and hexafluoroisopropanol is used if measurement is still impossible.

Said organic solvent to be used for dissolving polymer A and polymer B is an organic solvent that can dissolve polymer A and polymer B to be used and may be selected according to the types of these polymers.

Specific examples include pentane, hexane, heptane, octane, nonane, n-decane, n-dodecane, n-tridecane, tetradecane, cyclohexane, cyclopentane,and other aliphatic hydrocarbon based solvents; benzene, toluene, xylene, 2-methyl naphthalene, and other aromatic hydrocarbon based solvents; ethyl acetate, methyl acetate, butyl acetate, butyl propionate, butyl butyrate, and other ester based solvents; chloroform, bromoform, methylene chloride, carbon tetrachloride, 1,2-dichloroethane, 1,1,1-trichloroethane, chlorobenzene, 2,6-dichlorotoluene, hexafluoroisopropanol, and other halogenated hydrocarbon based solvents; acetone, methylethylketone, methylisobutylketone, methylbutylketone, and other ketone based solvents; methanol, ethanol, 1-propanol, 2-propanol, and other alcohol based solvents; N-methyl-2-pyrolidone (NMP), dimethyl sulfoxide (DMSO), N,N-dimethyl formamide (DMF), N,N-dimethyl acetamide (DMA), propylene carbonate, trimethyl phosphate, 1,3-dimethyl-2-imidazolidinone, sulfolane, and other aprotic polar solvents; formic acid, acetic acid, propionic acid, butyric acid, lactic acid, and other carboxylic acid solvents; anisole, diethyl ether, tetrahydrofuran, diisopropyl ether, dioxane, diglyme, dimethoxy ethane, and other ether based solvents; 1-butyl-3-methyl imidazolium acetate, 1-butyl-3-methyl imidazolium hydrogen sulfate, 1-ethyl-3-imidazolium acetate, 1-ethyl-3-methyl imidazolium thiocyanate, and other ionic liquid; and mixtures thereof. Preferable are aromatic hydrocarbon based solvents, aliphatic hydrocarbon based solvents, halogenated hydrocarbon based solvents, alcohol based solvents, ether based solvents, aprotic polar solvents, and carboxylic acid solvents, more preferable being water-soluble alcohol based solvents, aprotic polar solvents, and carboxylic acid solvents, extremely preferable are aprotic polar solvents, and carboxylic acid solvents, and most preferable being N-methyl-2-pyrolidone, dimethyl sulfoxide, N,N-dimethyl formamide, N,N-dimethyl acetamide, propylene carbonate, formic acid, and acetic acid because they are so widely available and able dissolve a variety of polymers that they can suit various polymers used as polymer A and also because they can mix uniformly with water, alcohol based solvents, and other solvents listed later that can be used preferably as said poor solvent.

These organic solvents may be used as a mixture of two or more thereof or in combination with others, but from the viewpoint of producing particles with relatively small particle diameters and a narrow particle diameter distribution and avoiding troublesome work in a separation step for recycling of used solvents to reduce the process load for production, it is preferable to use a single organic solvent and more preferable to use a single organic solvent that can dissolve both polymer A and polymer B.

Said poor solvent for polymer A used for the present method, is defines as a solvent that does not dissolve polymer A. Here, for a solvent that does not dissolve polymer A, the polymer has a solubility of 1 mass% or less, preferably 0.5 mass% or less, and more preferably 0.1 mass% or less.

The present method uses a poor solvent for polymer A, but it is preferable that the poor solvent can dissolve polymer B in addition to being a poor solvent for polymer A. In such a case, polymer particles constituted of polymer A can be precipitated efficiently. It is also preferable that the solvent to dissolve polymer A and polymer B and the poor solvent for polymer A can mix homogeneously.

The type of poor solvent to be used effectively for the present method varies depending on the type of polymer A used or desirably depending on the type of both polymers A and B used, but specifically, it may be one solvent selected from the following: pentane, hexane, heptane, octane, nonane, n-decane, n-dodecane, n-tridecane, tetradecane, cyclohexane, cyclopentane,and other aliphatic hydrocarbon based solvents; benzene, toluene, xylene, 2-methyl naphthalene, and other aromatic hydrocarbon based solvents; ethyl acetate, methyl acetate, butyl acetate, butyl propionate, butyl butyrate, and other ester based solvents; chloroform, bromoform, methylene chloride, carbon tetrachloride, 1,2-dichloroethane, 1,1,1-trichloroethane, chlorobenzene, 2,6-dichlorotoluene, hexafluoroisopropanol, and other halogenated hydrocarbon based solvents; acetone, methyl ethyl ketone, methyl isobutyl ketone, methyl butyl ketone, and other ketone based solvents; methanol, ethanol, 1-propanol, 2-propanol, and other alcohol based solvents; dimethyl sulfoxide, N,N-dimethyl formamide, N,N-dimethyl acetamide, trimethyl phosphate, N-methyl-2-pyrolidone, 1,3-dimethyl-2-imidazolidinone, sulfolane, and other aprotic polar solvents; formic acid, acetic acid, propionic acid, butyric acid, lactic acid, and other carboxylic acid solvents; anisole, diethyl ether, tetrahydrofuran, diisopropyl ether, dioxane, diglyme, dimethoxy ethane, and other ether based solvents; and water.

From the viewpoint of efficient production of particles from polymer A, preferable ones include aromatic hydrocarbon based solvents, aliphatic hydrocarbon based solvents, alcohol based solvents, ether based solvents, and water, of which the most preferable are alcohol based solvent and water, and particularly preferable is water.

For the present method, metal-containing synthetic polymer particles can be produced when polymer A, polymer B, an organic solvent that dissolves them, and a poor solvent for polymer A are appropriately selected and used in combination to allow polymer A to be precipitated efficiently.

Here, it is necessary for the liquid prepared by mixing and dissolving polymers A and B, a metal component, and an organic solvent that dissolves them is phase-separated to form a solution phase composed mainly of polymer A and a solution phase composed mainly of polymer B. Here, the metal may form a solid phase instead of being dissolved, but it does not act as a phase as mentioned in the above description.

Note that the organic solvent used for the solution phase composed mainly of polymer A and organic solvent used for the solution phase composed mainly of polymer B may be either identical or different, but it is preferable that they are substantially identical.

The conditions to develop a state of two phase separation varies depending on types of polymers A and B, molecular weight of polymers A and B, type of the organic solvents, concentration of polymers A and B, and the temperature and pressure where the present method is to be carried out.

To produce conditions where phase separation easily takes place, it is preferable that the solubility parameter (which may be hereinafter referred to as SP value) of polymer A is largely different from that of polymer B.

Here, the difference in SP value is 1 $(J/cm^3)^{1/2}$ or more, more preferably 2 $(J/cm^3)^{1/2}$ or more, still more preferably 3 $(J/cm^3)^{1/2}$ or more, particularly preferably 5 $(J/cm^3)^{1/2}$ or more, and extremely preferably 8 $(J/cm^3)^{1/2}$ or more. Phase separation is caused easy if the SP value is in this range.

There are no specific limitations if both polymer A and polymer B can be dissolved in the organic solvent used, but the upper limit on the difference in SP value is preferably 20 $(J/cm^3)^{1/2}$ or less, more preferably 15 $(J/cm^3)^{1/2}$ or less, and still more preferably 10 $(J/cm^3)^{1/2}$ or less.

The SP value as referred to herein is calculated according to Fedor's estimation method and calculated based on cohesive energy density and molecular volume (hereinafter, sometimes referred simply to calculation method) ("SP-chi: Kiso • Oyo to Keisan Hoho (SP Value: Basics/Applications and Calculation Method), Hideki Yamamoto, Johokiko Co., Ltd, Mar. 31, 2005).

If the solubility parameter cannot be calculated by the estimation method, the SP value is calculated by the experiment method in which it is determined based on whether the polymer can dissolve in solvents with a known solubility parameter (hereinafter, sometimes referred to simply as experiment method) and used instead (Polymer Handbook Fourth Edition, J. Brand, Wiley, 1998).

Conditions to cause phase separation are set up according to a three-component phase diagram, which can be prepared based on a simple preliminary experiment to observe various states at different ratios of the three components of polymer A, polymer B and an organic solvent that dissolves them.

A phase diagram can be prepared by mixing and dissolving polymer A, polymer B and an organic solvent at least 3 or more, preferably 5 or more, and more preferably 10 or more, appropriately selected proportions, leaving them to stand, and determining whether an interface develops, and conditions for phase separation are identified based on differentiation of regions where two phases are formed and those where only one phase is formed.

To determine whether a liquid is in a phase separation state, polymers A and B, and a solvent are mixed at an appropriate proportion under the temperature and pressure conditions where the present invention is to be implemented, and subsequently, polymers A and B are dissolved completely, stirred adequately after the dissolution, left to stand for 3 days, and observed whether macroscopic phase separation has occurred.

In the case where a sufficiently stable emulsion is formed, however, macroscopic phase separation may not take place even when the liquid is left to stand for 3 days. In that case, occurrence of phase separation is judged by using an optical microscope or phase contrast microscope to observe whether microscopic phase separation takes place.

Phase separation occurs as a result of separation between the polymer A solution phase composed mainly of polymer A and the polymer B solution phase composed mainly of polymer B in the organic solvent. Here, the polymer

A solution phase is the phase to which mainly polymer A has been distributed while the polymer B solution phase is the phase to which mainly polymer B has been distributed. Here, the polymer A solution phase and the polymer B solution phase each appears to have a volume ratio depending on the type and content of polymers A and B, respectively.

The contents of polymer A and polymer B should, as a prerequisite, be such that a phase separation state can develop and also that they make the method industrially practicable and are as high as possible in the organic solvent, but they are preferably more than 1 mass% to 50 mass%, more preferably more than 1 mass% to 30 mass%, and still more preferably more than 2 mass% to 20 mass%.

With respect to the quantity of the metal component, an appropriate metal content is determined taking into consideration the ratio of distribution to the polymer A solution phase in the phase separation state and the metal content after calcination so that an intended quantity is introduced into the particles, but commonly, the content of elemental metal is in the range of 0.1 parts by mass to 100 parts by mass, preferably 0.1 parts by mass to 80 parts by mass, more preferably 0.1 parts by mass to 50 parts by mass, and particularly preferably 1 part by mass to 30 parts by mass, per 100 parts by mass of polymer A component.

With respect to the polymer A solution phase and the polymer B solution phase for the present method, the interfacial tension between the two phases is small since both phases are of an organic solvent, and this nature allows the resulting emulsion to be maintained stable, possibly acting to narrow the particle diameter distribution. In particular, this effect is noticeable when polymer A phase and polymer B phase are formed of the same organic solvent.

For the present method, the interfacial tension between the two phases are so small that the interfacial tension cannot be measured directly by the commonly used hanging-drop method in which measurements are made by adding a dissimilar solution to the solution, but the interfacial tension can be estimated from the surface tension of each phase in air. Denoting the surface tension of each phase in air by $r_1$ and $r_2$, the interfacial tension between them, $r_{12}$, is estimated as the absolute value of $r_{12} = r_1 - r_2$. Here, the value of $r_{12}$ is preferably in the range of more than 0 to 10 mN/m, more preferably more than 0 to 5 mN/m, still more preferably more than 0 to 3 mN/m, and particularly preferably more than 0 to 2 mN/m.

For the present method, the viscosity between the two phases has influence on the average particle diameter and particle diameter distribution, and the particle diameter distribution index tends to decrease with an decreasing viscosity ratio. If it is assumed that the viscosity ratio is defined as the ratio of the polymer A solution phase / polymer in the solution phase B under the temperature conditions where the present invention is to be implemented, it is preferably in the range of 0.1 or more and 10 or less, more preferably in the range of 0.2 or more and 5 or less, still more preferably in the range of 0.3 or more and 3 or less, particularly preferably in the range of 0.5 or more and 1.5 or less, and extremely preferably in the range of 0.8 or more and 1.2 or less.

A phase-separated system thus obtained is used to produce metal-containing synthetic polymer particles. A common reaction tank is used to produce particles. From the viewpoint of industrial practicability, the temperature range suitable for implementing the present invention is -50°C to 200°C, preferably -20°C to 150°C, more preferably 0°C to 120°C, still more preferably 10°C to 100°C, particularly preferably 20°C to 80°C, and most preferably 20°C to 50°C. From the viewpoint of industrial practicability, the pressure range suitable for implementing the present invention is from a reduced pressure to 100 atm, preferably 1 atm to 5 atm, more preferably 1 atm to 2 atm, and particularly preferably atmospheric pressure.

An emulsion is formed by mixing the components under such conditions to develop a phase-separated state.

Specifically, an emulsion is produced by applying a shear force to the resulting phase-separated solution.

In the emulsion formation step, an emulsion is produced in such a way that the polymer A solution phase form particle-like droplets. It is commonly seen that when a solution is phase-separated, such an emulsion tends to form easily when the volume of the polymer B solution phase is larger than that of the polymer A solution phase, and the ratio of the volume of the polymer A solution phase to that of the total volume of the two phases is preferably 0.4 or less, more preferably in the range of 0.4 to 0.1. When preparing said phase diagram, an appropriate range can be set up if the volume ratio of each component is measured at the same time.

The present production process aims to add a metal component into carbon particles. Various studies have been carried out and results show that in order to add a metal component efficiently and homogeneously to form metal-containing carbon particles, it is preferable that the metal component is added at a point before the emulsion formation step in the process to be carried out for producing metal-containing synthetic polymer particles.

The addition of a metal component may be performed during the emulsion formation step, before the emulsion formation step, or after the emulsion formation step, it is preferable that it is performed before the emulsion formation step. The addition of a metal component before the emulsion formation step is preferable because it serves to provide metal-containing carbon particles with a higher metal dispersibility.

Metal-containing synthetic polymer particles produced by the present production method are in the form of particles with a narrow particle diameter distribution, which is made possible because a highly uniform emulsion is formed in the emulsion formation step. This tendency is noticeable when a single solvent that can dissolve both polymers A and B is used. Thus, the use of a conventionally known stirring method is sufficiently effective to produce a shear force

required for the emulsion formation, and examples of said method to be used for mixing include liquid phase stirring by stirring blades, stirring by a continuous biaxial stirrer, mixing by a homogenizer, and ultrasonic irradiation.

When agitating blades are used for stirring, in particular, the stirring speed is preferably 50 rpm to 1200 rpm, more preferably 100 rpm to 1000 rpm, still more preferably 200 rpm to 800 rpm, and particularly preferably 300 rpm to 600 rpm, depending on the shape of the agitating blades used.

The agitating blade may specifically be in the shape of propeller, paddle, flat paddle, turbine, double cone, single cone, single ribbon, double ribbon, screw, or helical ribbon, but it is not limited thereto as long as a sufficient shear force can be applied to the system. In addition, baffle boards etc. may be provided in the tank to ensure efficient stirring.

For formation of an emulsion, not only a stirrer but also other generally known tools such as emulsifier and dispersing machine may be used. Specific examples include Homogenizer (supplied by IKA), Polytron (supplied by Kinematica, Inc.), T. K. Autohomemixer (supplied by Tokushu Kika Kogyo Co., Ltd.), other batch-wise emulsifiers, Ebara Milder (supplied by Ebara Corporation), T. K. Filmix, T. K. Pipeline Homomixer (supplied by Tokushu Kika Kogyo Co., Ltd.), Colloid Mill (supplied by Shinko-Pantec Co., Ltd.), Slusher, Trigonal Wet Grinder (supplied by Mitsui Miike Kakoki Co., Ltd.), ultrasonic homogenizers, and static mixers.

The resulting emulsion is then fed to a step for precipitating metal-containing synthetic polymer particles.

To produce metal-containing synthetic polymer particles, a poor solvent for polymer A is brought into contact with the emulsion produced in the above step to precipitate metal-containing synthetic polymer particles having a diameter corresponding to that of the emulsion droplets.

Bringing the poor solvent and the emulsion into contact with each other may be achieved by pouring the emulsion in the poor solvent, and pouring the poor solvent into the emulsion, of which pouring the poor solvent into the emulsion is referable.

Here, there are no specific limitations on the method to be used to pour the poor solvent as long as metal-containing synthetic polymer particles produced according to the present invention are obtained, and the useful methods include continuous dropping, divided addition, and on-step addition. In order to prevent widening in the particle diameter distribution and formation of bulky lumps exceeding 1,000 μm from being caused by coagulation, fusion, or coalescence of the emulsion at the time of adding the poor solvent, it is preferable to use continuous dropping and divided dropping, and it is most preferable to use continuous dropping to ensure industrially efficient implementation.

The duration of adding the poor solvent is 10 min or more and 50 hours or less, more preferably 30 min or more and 10 hours or less, and still more preferably 1 hour or more and 5 hours or less.

If the implementation duration is shorter than these ranges, widening in the particle diameter distribution and formation of bulky lumps may result from coagulation, fusion, or coalescence of the emulsion. An implementation duration longer than them is impractical from an industrial point of view.

Implementation in these ranges makes it possible to depress coagulation of particles during conversion from an emulsion into metal-containing synthetic polymer particles, thereby providing metal-containing synthetic polymer particles with a narrow particle diameter distribution.

Depending on the conditions of the emulsion, the quantity of the poor solvent to be added is preferably 0.1 part by mass to 10 parts by mass, more preferably 0.1 part by mass to 5 parts by mass, still more preferably 0.2 part by mass to 3 parts by mass, particularly preferably 0.2 part by mass to 1 part by mass, and most preferably 0.2 part by mass to 0.5 part by mass, per 1 part by mass of the total weight of the emulsion.

There are no specific limitations on the duration of contact between the poor solvent and the emulsion as long as it is sufficiently long for precipitation of metal-containing synthetic polymer particles, but in order to cause precipitation adequately and ensure high productivity, it is 5 min to 50 hours, preferably 5 min or more and 10 hours or less, more preferably 10 min or more and 5 hours or less, particularly preferably 20 min or more and 4 hours or less, and extremely preferably 30 min or more and 3 hours or less, after the end of addition of the poor solvent.

From a dispersion liquid of metal-containing synthetic polymer particles thus obtained, particulate powder can be recovered by solid-liquid separation using a generally known method such as filtration, decantation, vacuum filtration, compression filtration, centrifugal separation, centrifugal filtration, spray dry, acid precipitation, salt precipitation, and freeze coagulation.

The metal-containing synthetic polymer particles obtained by solid-liquid separation is cleaned with a solvent or the like, as required, to remove impurities and so on attached or contained, and then purified. Preferable solvents to be used here include the poor solvents mentioned above, and more preferable is a solvent selected from the group of water, methanol, and ethanol, or a mixture of two or more thereof.

The resulting metal-containing synthetic polymer particles may be dried to remove the residual solvent. The drying methods that can be used here include air drying, heat drying, vacuum drying, and freeze drying. The temperature used for heating is preferably lower than the glass transition temperature, and specifically, a temperature of 50 to 150°C is preferable.

The method according to the present invention is advantageous in that the organic solvent, polymer B, and/or the metal component that are separated in the solid-liquid separation step carried out to obtain metal-containing synthetic

polymer particles can be recycled to ensure their efficient use.

The solvent resulting from the solid-liquid separation step is actually a mixture of polymer B, organic solvent, poor solvent, and/or metal component. Removal of the poor solvent from the solvent component allows the remaining solvent mixture to be recycled for emulsion formation.

Some part of the metal component is not incorporated in the metal-containing synthetic polymer particles and left in this solvent mixture, and if it is recycled, there will be no loss of the metal component over the entire production process.

For removing the poor solvent, generally known methods may be used, including, specifically, simple distillation, reduced pressure distillation, precision distillation, thin film distillation, extraction, and membrane separation, of which simple distillation, reduced pressure distillation, and precision distillation ate preferable.

Heat is applied to the system when performing distillation operations such as simple distillation and reduced pressure distillation, possibly accelerating the thermal decomposition of polymer B and organic solvents, and therefore, they are performed in an atmosphere with as low an oxygen content as possible, preferably in an inert atmosphere. Specifically, they should be performed in an atmosphere of nitrogen, helium, argon, or carbon dioxide.

The metal-containing synthetic polymer particles thus produced have a number average particle diameter of 100 μm or less, preferably 50 μm or less, and more preferably 30 μm or less. As lower limit, it is 0.1 μm or more, preferably 0.5 μm or more, more preferably 0.7 μm or more, and particularly preferably 1 μm or more. The handleability deteriorates if the number average particle diameter is too small, whereas if it is too large, the resulting metal-containing carbon particles will have an increased diameter, which may be unpreferable from the viewpoint of dispersibility.

The number average particle diameter is determined by observing 100 particles randomly selected from scanning electronic microscope photographs, measuring their diameters, and making calculations by Equation (1) given below. Here, if a particle is not perfectly circular, its major axis is taken as its diameter.

The particle diameter distribution of the metal-containing carbon particles can be represented by their particle diameter distribution index, and it is in the range of 1.0 to 2.0, preferably 1.0 to 1.8, more preferably 1.0 to 1.5, and particularly preferably 1.0 to 1.3. A particle diameter distribution index out of these ranges is not preferable because in that case, it will be impossible to produce metal-containing carbon particles with a narrow particle diameter distribution. Here, the particle diameter distribution index is calculated by Equation (3) given below from the ratio of the volume average particle diameter to the number average particle diameter. The volume average particle diameter is determined by observing 100 particles randomly selected from scanning electronic microscope photographs, measuring their diameters, and making calculations by Equation (2) given below. Here, if a particle is not perfectly circular, its major axis is taken as its diameter.

[Mathematical formula 9]

$$Dn = \frac{\sum_{i=1}^{n} Ri}{n} \qquad (1)$$

$$Dv = \frac{\sum_{i=1}^{n} Ri^4}{\sum_{i=1}^{n} Ri^3} \qquad (2)$$

$$PDI = \frac{Dv}{Dn} \qquad (3)$$

Here, Ri, n, Dn, Dv, and PDI denote the particle diameter of a particular particle, number of measurements (100), number average particle diameter, volume average particle diameter, and particle diameter distribution index, respectively.

The metal-containing synthetic polymer particles used for the present invention have a sphericity of 80 or more, preferably 85 or more, more preferably 90 or more, still more preferably 92 or more, particularly preferably 95 or more,

and most preferably 99 or more.
As metal-containing synthetic polymer particles with a high sphericity tend to provide metal-containing carbon particles with a high sphericity, a sphericity in said range is preferable because in that case, perfectly spherical metal-containing carbon particles can be produced easily. Here, the sphericity is determined by observing particles by scanning electronic microscopy, measuring their major and minor axes, and making calculations from their average over randomly selected 30 particles by Equation (4).

[Mathematical formula 10]

$$\text{Sphericity} = \frac{\sum_{i=1}^{n}(\text{minor axes}/\text{major axes})}{n} \times 100 \qquad (4)$$

Here, n denotes the number of measurements, which is equal to 30.

The carbonization method for metal-containing synthetic polymer particles is described specifically below.

The carbonizing step is carried out by heating the metal-containing synthetic polymer particles in an inert gas atmosphere that does not react with the metal-containing synthetic polymer particles. It is preferable to use an inert gas such as helium, argon, and nitrogen.

The atmosphere used for heating may be either a flow type or a closed type, but a flow type atmosphere is preferable because the gas generated by heating can be removed. With respect to pressure, heating may be performed under either compressed pressure or reduced pressure, but commonly, it is performed under atmospheric pressure.

When carried out under atmospheric pressure, heating is performed at a temperature of 400°C or more, preferably 800°C or more. There are no specific limitations on the upper limit, but it is commonly 3,000°C and preferably 2,500°C.

The degree of graphitization of the metal-containing carbon particles tends to increase with an increasing heating temperature and an increasing duration during which the heating temperature is maintained. The heating temperature is preferably 400°C or more, more preferably 800°C or more, and most preferably 1,000°C or more.

The heating rate to reach the heating temperature is commonly 20°C/min or less, preferably 10°C/min or less, and more preferably 5°C/min or less. As the calcination temperature exceeds 20°C/min, the particles tend to undergo fusion.

Depending on the heating temperature, the heating-temperature holding duration is preferably 0.5 hour or more, more preferably 1 hour or more, still more preferably 5 hours or more, particularly preferably 10 hours or more, extremely considerably preferably 50 hours or more, as lower limit, and it is preferably 1,000 hours or less, more preferably 500 hours or less, and still more preferably 100 hours or less, as upper limit. With respect to the heating temperature, furthermore, the particles may be maintained at an appropriate temperature for an appropriate time, followed by further heating up to a desired heating temperature.

Said method may also be used in the case where the metal-containing synthetic polymer particles are carbonized, and it is preferable to perform pre-treatment as described below because it serves to allow the metal-containing synthetic polymer particles to form an oxidized structure and also serves to improve the carbonization yield and prevent fusion of particles.

An oxidized structure can be formed by heating the metal-containing synthetic polymer particles in an oxidizing atmosphere. Said oxidizing atmosphere may contain oxygen, sulfur, or the like, but it is preferably an air atmosphere. There are no specific limitations on the heating temperature as long as an oxidized structure is formed, but it is 100 to 300°C, preferably 150 to 250°C. A heating temperature of less than 100°C is not preferable because in that case, the formation of an oxidized structure does not progress, while the metal-containing synthetic polymer particles start to melt before an oxidized structure is formed if the temperature is above 300°C. The heating rate to reach the heating temperature is commonly 20°C/min or less, preferably 10°C/min or less, and more preferably 5°C/min or less. A calcination temperature of above 20°C/min is not preferable because it causes fusion of the particles. The heating temperature, furthermore, may be maintained for an appropriate time after heating up to a predetermined temperature. Depending on the heating temperature, the heating duration is 0.5 to 10 hours, preferably 0.5 to 5 hours, at the most. With respect to the heating temperature, furthermore, the particles may be maintained at an appropriate temperature for an appropriate time, followed by further heating up to a desired heating temperature.

Described below is the production process for porous carbon particles.

The carbon particles containing porous structures of specific size according to the present invention can be

produced by carbonizing synthetic polymer particles contain a daughter particle component (which may be referred to hereinafter as daughter-particle-containing polymer particles).

For the present method, polymer A refers to a polymeric material (synthetic resin) that constitutes said synthetic polymer particles, and there are no specific limitations on the synthetic resin that constitutes said daughter-particle-containing polymer particles as long as it is a carbonizable synthetic resin. Specifically, said particles can be produced by calcining daughter-particle-containing polymer particles constituted of at least one selected from the group of a polyolefin based copolymer, polyacrylonitrile based copolymer consisting of an acrylonitrile based monomer and a hydrophilic vinyl monomer, polyacrylamide based polymer, polyvinyl acetate based polymer, polyvinyl chloride based polymer, polyvinylidene chloride based polymer, polyamide, polyarylene ether, polyarylene sulfide, polysulfone, polyether ketone, polyether ether ketone, polyarylate, polyamide-imide, and polyimide.

Of these, daughter-particle-containing polymer particles produced by adding a daughter particle component to a polyacrylonitrile based copolymer consisting of an acrylonitrile based monomer and a hydrophilic vinyl monomer, polyamide, polyarylene ether, polyarylene sulfide, polysulfone, polyether ketone, polyether ether ketone, polyarylate, polyamide-imide, or polyimide are preferable because they serve to increase the degree of graphitization and produce higher-quality carbon particles, and daughter-particle-containing polymer particles produced by adding a daughter particle component to a polyacrylonitrile based copolymer consisting of an acrylonitrile based monomer and a hydrophilic vinyl monomer, polyamide-imide, polyetherimide, or polyimide are particularly preferable, of which a polyacrylonitrile based copolymer consisting of an acrylonitrile based monomer and a hydrophilic vinyl monomer, and polyamide-imide are the most preferable, because their particles will not suffer from significant fusion when carbonized.

In the case of polyamide-imide, although details have not yet been clarified sufficiently, it serves to produce porous metal-containing carbon particles probably because the cyclic imide structure and amide structure existing in their polymer chains can be easily carbonized and cyclized during the carbonizing step, and any appropriate particle production technique may be used without being limited to the above-mentioned one.

The daughter-particle-containing polymer particles used for the present invention have a number average particle diameter of 100 $\mu$m or less, preferably 50 $\mu$m or less, and more preferably 30 $\mu$m or less, as upper limit. As lower limit, it is 0.1 $\mu$m or more, preferably 0.5 $\mu$m or more, more preferably 0.7 $\mu$m or more, and particularly preferably 1 $\mu$m or more. The handleability deteriorates if the number average particle diameter is too small whereas if it is too large, the resulting carbon particles will increase in diameter, leading to a decrease in the dispersibility as filler in resin or the like. Accordingly, such particles may not be preferable in some cases.

The number average particle diameter is determined by observing 100 particles randomly selected from scanning electronic microscope photographs, measuring their diameters, and making calculations by Equation (1) given below. Here, if a particle is not perfectly circular, its major axis is taken as its diameter.

The particle diameter distribution of daughter-particle-containing polymer particles can be represented by their particle diameter distribution index. The particle diameter distribution index is in the range of 1.0 to 2.0, preferably 1.0 to 1.8, more preferably 1.0 to 1.5, and particularly preferably 1.0 to 1.3. A particle diameter distribution index out of these ranges is not preferable because in that case, it will be impossible to produce porous carbon particles with a narrow particle size distribution. Here, the particle diameter distribution index is calculated by Equation (3) given below from the ratio of the volume average particle diameter to the number average particle diameter. The volume average particle diameter is determined by observing 100 particles randomly selected from scanning electronic microscope photographs, measuring their diameters, and making calculations by Equation (2) given below. Here, if a particle is not perfectly circular, its major axis is taken as its diameter.

[Mathematical formula 11]

$$Dn = \frac{\sum_{i=1}^{n} Ri}{n} \quad (1)$$

$$Dv = \frac{\sum_{i=1}^{n} Ri^{4}}{\sum_{i=1}^{n} Ri^{3}} \quad (2)$$

$$PDI = \frac{Dv}{Dn} \quad (3)$$

Here, Ri, n, Dn, Dv, and PDI denote the particle diameter of a particular particle, number of measurements (100), number average particle diameter, volume average particle diameter, and particle diameter distribution index, respectively.

The daughter-particle-containing polymer particles used for the present invention have a sphericity of 80 or more, preferably 85 or more, more preferably 90 or more, still more preferably 92 or more, particularly preferably 95 or more, and most preferably 95 or more. As daughter-particle-containing polymer particles with a high sphericity tend to provide porous carbon particles with a high sphericity, a sphericity in said range is preferable because in that case, perfectly spherical porous carbon particles can be produced easily. Here, the sphericity is determined by observing particles by scanning electronic microscopy, measuring their major and minor axes, and making calculations from their average over randomly selected 30 particles by Equation (4).

[Mathematical formula 12]

$$\text{Sphericity} = \frac{\sum_{i=1}^{n} (\text{minor axes} / \text{major axes})}{n} \times 100 \quad (4)$$

Here, n denotes the number of measurements, which is equal to 30.

Daughter-particle-containing polymer particles to be used for the present invention contain a daughter particle component, and porous structures originating from the daughter particle component can be formed in carbon particles by calcining polymer particles that contain the daughter particle component.

The daughter particle component as referred to herein has shapes such as spherical, ellipsoidal, flattened, rock-like, konpeito-like (pointed sugar candy ball), and irregular, and the interior of the daughter particle component may be hollow or porous. The size of the pores in the carbon particles reflect the diameter and shape of the daughter particle component in the case where the daughter particle component disappears as a result of calcination, whereas the size of the pores reflect the interior structures of the daughter particles in the case where the daughter particle component remains after the calcination step. Whether it disappears or not can be controlled according to the material type of the daughter particle component, calcination conditions including temperature, atmosphere, and pressure, and the like.

Since the diameter, shape, and interior structure of the daughter particle component is reflected in the size of pores in the carbon particles, the daughter particle component commonly has a size in the range of 0.01 to 10 μm. It is preferably 0.01 to 1 μm, more preferably 0.01 to 0.5 μm, and most preferably 0.01 to 0.25 μm in order to making it possible to prevent a decrease in the density of the resulting carbon particles and improve the specific surface at the same time. Here, the diameter of the daughter-particle component is determined by fixing daughter-particle-containing polymer particles with epoxy resin or the like, preparing ultrathin sections or sectioned specimens for electronic microscope observation, and measuring the size of 20 pieces of the daughter-particle component in randomly selected daughter-particle-containing polymer particles by scanning electronic microscopy. If such observation is difficult, a transmission

electronic microscope is used for measurement. If such observation is difficult, a transmission microscope is used for measurement. If the daughter particle component is not perfectly spherical, its major axis is measured.

The daughter particle component may be of a material in the form of inorganic particles, organic particles, and the like.

Said inorganic particles may be of a material that disappears as a result of calcination or of a hollow material that does not disappear, but in many cases, inorganic particles will remain after the calcination step, indicating that hollow inorganic particles are preferable. Specifically, preferable hollow inorganic particles consist of a hollow core and a shell of a material as follows: carbon, silica, alumina, titanium oxide, barium titanate, magnesium titanate, calcium titanate, strontium titanate, zinc oxide, silica sand, clay, mica, wollastonite, diatomaceous earth, cerium chloride, colcothar, chrome oxide, cerium oxide, antimony trioxide, magnesium oxide, zirconium oxide, silicon carbide, silicon nitride, calcium carbonate, magnesium carbonate, and calcium phosphate. These may be used singly or as a mixture of two or more thereof. Being easily available, carbon nanotube, fullerene, ketjen black, hollow silica particles, and the like are preferable.

Said organic particles may be of a material that disappears as a result of calcination or of a hollow material that does not disappear, of which a material that disappears as a result of calcination is preferable. More preferable are organic materials that maintain the particle shape during the production process for daughter-particle-containing polymer particles, of which those insoluble in good solvents for the daughter-particle-containing polymer particles are particularly preferable.

Specifically, said organic particles are preferably of a material such as a polymer with a crosslinked structure produced by polymerizing monomers having two or more carbon-carbon double bonds in their molecules, a copolymer consisting of said monomers and others that are copolymerizable with them, and a core-shell rubber produced by graft polymerization of rubber particles with said monomers.

Said monomers having two or more carbon-carbon double bonds in their molecules include butadiene, isoprene, 2,3-dimethyl butadiene, 2-methyl-3-ethyl butadiene, 1,3-pentadiene, 3-methyl-1,3-pentadiene, 2-ethyl-1,3-pentadiene, 1,3-hexadiene, 2-methyl-1,3-hexadiene, 3,4-dimethyl-1,3-hexadiene, 1,3-heptadiene, 3-methyl-1,3-heptadiene, 1,3-octadiene, cyclopentadiene, chloroprene, myrcene, allyl (meth)acrylate, methallyl (meth)acrylate, allyl cinnamate, methallyl cinnamate, diallyl maleate, diallyl phthalate, diallyl terephthalate, diallyl isophthalate, divinylbenzene, ethylene glycol di (meth)acrylate, butanediol di(meth)acrylate, hexanediol di(meth)acrylate, trimethylolpropane di(meth)acrylate, trimethylolpropane tri(meth)acrylate, pentaerythritol tri(meth)acrylate, pentaerythritol tetra(meth)acrylate, polyethylene glycol di(meth)acrylate, and polypropylene glycol di(meth)acrylate.

Said other copolymerizable monomer include: olefin based monomers such as ethylene, propylene, 1-butene, 1-hexene, 1-octene, 4-methyl-1-pentene, 3-methyl-1-butene, vinyl chloride, vinylidene chloride, vinylidene fluoride, vinyl fluoride, trifluoroethylene, and tetrafluoroethylene; $\alpha$-olefin based monomers such as vinyl cycloalkane; cyclic olefin based monomers such as norbornene, and norbornadiene; aromatic vinyl based monomers such as styrene, $\alpha$-methyl styrene, 1-vinyl naphthalene, 3-methyl styrene, 4-propyl styrene, 4-cyclohexyl styrene, 4-dodecyl styrene, 2-ethyl-4-benzyl styrene, 4-(phenyl butyl) styrene, divinylbenzene and chlorostyrene; unsaturated carboxylate based monomers such as methyl (meth)acrylate, ethyl (meth)acrylate, n-propyl (meth)acrylate, n-butyl (meth)acrylate, t-butyl (meth)acrylate, n-hexyl (meth)acrylate, cyclohexyl (meth)acrylate, chloromethyl (meth)acrylate, 2-chloroethyl (meth)acrylate, 2-hydroxyethyl (meth)acrylate, 3-hydroxypropyl (meth)acrylate, 2,3,4,5,6-pentahydroxyhexyl (meth)acrylate, 2,3,4,5-tetrahydroxypentyl (meth)acrylate, phenyl (meth)acrylate, and benzyl (meth)acrylate; unsaturated carboxylic acid monomers such as (meth)acrylic acid, itaconic acid, crotonic acid, fumaric acid, maleic acid, citraconic acid, methaconic acid, glutaric acid, tetrahydrophthalic acid, isocrotonic acid, nadic acid, butene-tricarboxylic acid, monobutyl-fumaric acid, monoethyl-maleic acid, monomethyl-itaconic acid, p-vinyl-benzoic acid, and salts thereof; acrylonitrile based monomers such as acrylonitrile, methacrylonitrile, crotononitrile, and chloroacrylonitrile; unsaturated ester monomers such as vinyl acetate, vinyl propionate, vinyl butyrate, vinyl crotonate, vinyl hexanoate, vinyl pivalate, vinyl decanoate, vinyl myristate, vinyl palmitate, vinyl sorbate, vinyl lactate, and hydrolysates thereof; unsaturated monomers substituted with an amino group or a quaternary ammonium salt such as dimethylaminoethyl (meth)acrylate, diethylaminoethyl (meth)acrylate, vinyl pyrolidone, N-methylvinylpyridium chloride, (meth)allyl triethyl ammonium chloride, 2-hydroxy-3-(meth)acryloyloxy propyl trimethyl ammonium chloride, 2-vinyl pyridine, and 4-vinyl pyridine; unsaturated monomers substituted with an amide group such as (meth)acrylamide, N-hydroxyalkyl (meth)acrylamide, N-alkyl (meth)acrylamide, N,N-dialkyl (meth) acrylamide, and vinyl lactam; unsaturated monomers substituted with a sulfonic acid group such as vinyl sulfonic acid, methyl vinyl sulfonic acid, styrene sulfonic acid, (meth)acrylsulfonic acid, ethyl (meth)acrylate-2-sulfonate, and 2-acrylamide-2-hydroxypropane sulfonic acid; and and unsaturated monomers substituted with a phosphoric acid group such as propyl (meth)acrylate-3-chloro-2-phosphate, ethyl (meth)acrylate-2-phosphate, 3-allyloxy-2-hydroxypropane phosphoric acid.

For organic particles with a core-shell structure, preferable examples of the rubber component include polybutadiene, polyisoprene, poly(styrene-butadiene) copolymers, hydrogenated products of said block copolymerss, poly (acrylonitrile-butadiene) copolymers, poly(butadiene-isoprene) copolymers, poly(butadiene-butyl acrylate) copolymers,

poly(butyl (meth)acrylate-ethylene glycol di(meth)acrylate) copolymers, poly(butyl (meth)acrylate-propylenediol di(meth) acrylate) copolymers, poly(butyl (meth)acrylate-butanediol di(meth)acrylate) copolymers, poly(butyl (meth)acrylate-allyl (meth)acrylate) copolymers, and poly(butyl (meth)acrylate-divinylbenzene) copolymers.

Organic particles can be produced by generally known methods such as emulsion polymerization and suspension polymerization. They may be selected from commercially available products such as, specifically, Metabrane series supplied by Mitsubishi Rayon Co., Ltd., Nipol series supplied by Zeon Corporation, and Kane Ace series supplied by Kaneka Corporation.

Synthetic polymer particles containing a daughter particle component can be produced by a method as described below.

Thus, they can be produced by a procedure as follows: a polymer to be processed into particles (hereinafter, polymer A), polymer B which can dissolve in a poor solvent for polymer A, a daughter particle component, and an organic solvent are mixed to form an emulsion by allowing the daughter particle component which does not dissolve in the organic solvent to be dispersed in a two-phase separated system consisting of a solution phase composed mainly of polymer A (which may be hereinafter referred to as polymer A solution phase) and a solution phase composed mainly of polymer B (which may be hereinafter referred to as polymer B solution phase), followed by bringing it into contact with a poor solvent for polymer A to precipitate polymer A in the form of the polymer A solution phase containing the daughter particle component dispersed therein. To add metal into porous carbon particles, the metal component is mixed with polymer A and polymer B in carrying out the above process, to allow the metal component to be dispersed in the polymer A solution phase (it may be distributed in the polymer B solution phase as well), thereby providing polymer A in which the metal component and the daughter particle component are dispersed.

The above method is described in more detail below.

Said "two-phase separated system produced by mixing polymer A, polymer B, a daughter particle component, and an organic solvent and consisting of a solution phase composed mainly of polymer A and a solution phase composed mainly of polymer B", as mentioned above is a system that is separated into a solution phase mainly containing polymer A and a solution phase mainly containing polymer B when mixing polymer A, polymer B, the daughter particle component, and the organic solvent. Here, polymers A and B are dissolved in the organic solvent to form the polymer A solution phase and the polymer B solution phase, respectively, whereas the daughter particle component is not dissolved in the organic solvent.

The use of a system that undergoes such phase separation serves to form an emulsion when it is mixed under phase-separating conditions to cause emulsification.

Note that whether or not the polymer dissolves in the above step is judged according to whether it is dissolved up to more than 1 mass% in the organic solvent at the temperature where this method is carried out, that is, the temperature where polymer A and polymer B are dissolved and mixed to produce a two-phase separated solution.

It is necessary for this emulsion to consist of the polymer A solution phase acting as a dispersed phase and the polymer B solution phase acting as a continuous phase, with a daughter particle component existing in polymer A solution, although the daughter particle component may be distributed to both polymer A and polymer B solution phases. When a poor solvent for polymer A is brought into contact with this emulsion, polymer A is precipitated from the polymer A solution phase in the emulsion to form daughter-particle-containing polymer particles composed of polymer A and the daughter particle component.

For this method, there are no specific limitations on the combination of polymer A, polymer B, an organic solvent to dissolve them, a daughter particle component, and a poor solvent for polymer A, as long as polymer particles containing daughter particles as intended in this method are obtained.

For the present method, polymer A refers to the polymeric material that constitutes the daughter-particle-containing polymer particles.

Since the method is characterized by bringing polymer A into contact with a poor solvent to precipitate particles, it is preferable that the polymer is insoluble in poor solvents, more preferably insoluble in the poor solvents mentioned later, and particularly preferably insoluble in water.

Here, a water-insoluble polymer as referred to above has a water-solubility of 1 mass% or less, preferably 0.5 mass% or less, and more preferably 0.1 mass% or less.

Said polymer B may be a thermoplastic resin or a thermosetting resin, and it is preferably soluble in the organic solvent used for dissolving polymer A for the method and also in the poor solvent for polymer A, in particular, more preferably soluble in said organic solvent and also in alcohol based solvents or water from the viewpoint of high industrial handleability, and still more preferably soluble in said organic solvent and also in methanol, ethanol, or water.

Specific examples of polymer B include poly(vinyl alcohol) (which may be either completely saponified type or partially saponified type poly(vinyl alcohol)), poly(vinyl alcohol-ethylene) copolymer (which may be either a completely saponified type or a partially saponified type poly(vinyl alcohol-ethylene) copolymer), polyvinyl pyrolidone, poly(ethylene glycol), sucrose fatty acid ester, poly(oxy ethylene fatty acid ester), poly(oxy ethylene lauric fatty acid ester), poly(oxy ethylene glycol monofatty acid ester), poly(oxy ethylene alkyl phenyl ether), poly(oxy alkyl ether), polyacrylic acid, sodium

polyacrylate, polymethacrylic acid, sodium polymethacrylate, polystyrene sulfonic acid, sodium polystyrene sulfonate, polyvinyl pyrrolidinium chloride, poly(styrene-maleic acid) copolymer, aminopoly(acrylamide), poly(para-vinyl phenol), polyallyl amine, polyvinyl ether, polyvinyl formal, poly(acrylamide), poly(methacrylamide), poly(oxy ethylene amine), poly (vinyl pyrolidone), poly(vinyl pyridine), polyamino sulfone, polyethylene imine, and other synthetic resins; maltose, cellobiose , lactose, sucrose, and other disaccharides; cellulose, chitosan, hydroxyethyl cellulose, hydroxypropyl cellulose, methyl cellulose, ethyl cellulose, ethyl hydroxycellulose, carboxymethyl ethyl cellulose, carboxymethyl cellulose, sodium carboxymethyl cellulose, cellulose ester, and other cellulose derivatives; amylose and derivatives thereof, starch and derivatives thereof, dextrin, cyclodextrin, sodium alginate and derivatives thereof, and other polysaccharides and derivatives thereof; and gelatin , casein, collagen, albumin, fibroin, keratin, fibrin, carrageenan, chondroitin sulfate, gum arabic, agar, protein, and others; of which preferable are poly(vinyl alcohol) (which may be either completely saponified type or partially saponified type poly(vinyl alcohol)), poly(vinyl alcohol-ethylene) copolymer (which may be either a completely saponified type or a partially saponified type poly(vinyl alcohol-ethylene) copolymer), polyethylene glycol, sucrose fatty acid ester, poly(oxy ethylene alkyl phenyl ether), poly(oxy alkyl ether), poly(acrylic acid), poly(methacrylic acid), carboxymethyl cellulose, hydroxyethyl cellulose, hydroxypropyl cellulose, methyl cellulose, ethyl cellulose, ethyl hydroxycellulose, carboxymethyl ethyl cellulose, carboxymethyl cellulose, sodium carboxymethyl cellulose, cellulose ester, other cellulose derivatives, and polyvinyl pyrolidone, more preferable being poly(vinyl alcohol) (which may be either completely saponified type or partially saponified type poly(vinyl alcohol)), poly(vinyl alcohol-ethylene) copolymer (which may be either a completely saponified type or a partially saponified type poly(vinyl alcohol-ethylene) copolymer), polyethylene glycol, carboxymethyl cellulose, hydroxyethyl cellulose, hydroxypropyl cellulose, methyl cellulose, ethyl cellulose, ethyl hydroxycellulose, carboxymethyl ethyl cellulose, carboxymethyl cellulose, sodium carboxymethyl cellulose, cellulose ester, other cellulose derivatives, and polyvinyl pyrolidone, and particularly preferable are poly(vinyl alcohol) (which may be either completely saponified type or partially saponified type poly(vinyl alcohol)), poly(ethylene glycol), carboxymethyl cellulose, hydroxyethyl cellulose, hydroxypropyl cellulose, methyl cellulose, ethyl cellulose, ethyl hydroxycellulose, other cellulose derivatives, and polyvinyl pyrolidone.

With respect to the molecular weight of polymer B, the polymer preferably has a weight average molecular weight in the range of 1,000 to 100,000,000, more preferably 1,000 to 10,000,000, still more preferably 5,000 to 1,000,000, and particularly preferably 10,000 to 500,000, and most preferably 10,000 to 100,000.

The weight average molecular weight as referred to herein is defined as a weight average molecular weight measured by gel permeation chromatography (GPC) using water as solvent and converted in terms of polyethylene glycol.

Dimethyl formamide is used if measurement is impossible with water, tetrahydrofuran is used if it is still impossible, and hexafluoroisopropanol is used if measurement is still impossible.

The daughter particle component used for the present method is a material that is insoluble in polymer A, polymer B, the organic solvent that dissolves them, and the poor solvent for polymer A, and it is selected from among appropriate ones according to the type of the solvent used.

Said organic solvent to be used for dissolving polymer A and polymer B is an organic solvent that can dissolve polymer A and polymer B to be used and may be selected according to the types of these polymers.

Specific examples include pentane, hexane, heptane, octane, nonane, n-decane, n-dodecane, n-tridecane, tetradecane, cyclohexane, cyclopentane,and other aliphatic hydrocarbon based solvents; benzene, toluene, xylene, 2-methyl naphthalene, and other aromatic hydrocarbon based solvents; ethyl acetate, methyl acetate, butyl acetate, butyl propionate, butyl butyrate, and other ester based solvents; chloroform, bromoform, methylene chloride, carbon tetrachloride, 1,2-dichloroethane, 1,1,1-trichloroethane, chlorobenzene, 2,6-dichlorotoluene, hexafluoroisopropanol, and other halogenated hydrocarbon based solvents; acetone, methylethylketone, methylisobutylketone, methylbutylketone, and other ketone based solvents; methanol, ethanol, 1-propanol, 2-propanol, and other alcohol based solvents; N-methyl-2-pyrolidone (NMP), dimethyl sulfoxide (DMSO), N,N-dimethyl formamide (DMF), N,N-dimethyl acetamide (DMA), propylene carbonate, trimethyl phosphate, 1,3-dimethyl-2-imidazolidinone, sulfolane, and other aprotic polar solvents; formic acid, acetic acid, propionic acid, butyric acid, lactic acid, and other carboxylic acid solvents; anisole, diethyl ether, tetrahydrofuran, diisopropyl ether, dioxane, diglyme, dimethoxy ethane, and other ether based solvents; 1-butyl-3-methyl imidazolium acetate, 1-butyl-3-methyl imidazolium hydrogen sulfate, 1-ethyl-3-imidazolium acetate, 1-ethyl-3-methyl imidazolium thiocyanate, and other ionic liquid; and mixtures thereof. Preferable are aromatic hydrocarbon based solvents, aliphatic hydrocarbon based solvents, halogenated hydrocarbon based solvents, alcohol based solvents, ether based solvents, aprotic polar solvents, and carboxylic acid solvents, more preferable being water-soluble alcohol based solvents, aprotic polar solvents, and carboxylic acid solvents, extremely preferable are aprotic polar solvents, and carboxylic acid solvents, and most preferable being N-methyl-2-pyrolidone, dimethyl sulfoxide, N,N-dimethyl formamide, N,N-dimethyl acetamide, propylene carbonate, formic acid, and acetic acid because they are so widely available and able dissolve a variety of polymers that they can suit various polymers used as polymer A and also because they can mix uniformly with water, alcohol based solvents, and other solvents listed later that can be used preferably as said poor solvent.

These organic solvents may be used as a mixture of two or more thereof or in combination with others, but

from the viewpoint of producing particles with relatively small particle diameters and a narrow particle diameter distribution and avoiding troublesome work in a separation step for recycling of used solvents to reduce the process load for production, it is preferable to use a single organic solvent and more preferable to use a single organic solvent that can dissolve both polymer A and polymer B.

Said poor solvent for polymer A used for the present method, is defined as a solvent that does not dissolve polymer A. Here, for a solvent that does not dissolve polymer A, the polymer has a solubility of 1 mass% or less, preferably 0.5 mass% or less, and more preferably 0.1 mass% or less.

The present method uses a poor solvent for polymer A, but it is preferable that the poor solvent can dissolve polymer B in addition to being a poor solvent for polymer A. In such a case, polymer particles constituted of polymer A can be precipitated efficiently. It is also preferable that the solvent to dissolve polymer A and polymer B and the poor solvent for polymer A can mix homogenously.

The type of poor solvent to be used effectively for the present method varies depending on the type of polymer A used or desirably depending on the type of both polymers A and B used, but specifically, it may be one solvent selected from the following: pentane, hexane, heptane, octane, nonane, n-decane, n-dodecane, n-tridecane, tetradecane, cyclohexane, cyclopentane,and other aliphatic hydrocarbon based solvents; benzene, toluene, xylene, 2-methyl naphthalene, and other aromatic hydrocarbon based solvents; ethyl acetate, methyl acetate, butyl acetate, butyl propionate, butyl butyrate, and other ester based solvents; chloroform, bromoform, methylene chloride, carbon tetrachloride, 1,2-dichloroethane, 1,1,1-trichloroethane, chlorobenzene, 2,6-dichlorotoluene, hexafluoroisopropanol, and other halogenated hydrocarbon based solvents; acetone, methylethylketone, methylisobutylketone, methylbutylketone, and other ketone based solvents; methanol, ethanol, 1-propanol, 2-propanol, and other alcohol based solvents; dimethyl sulfoxide, N,N-dimethyl formamide, N,N-dimethyl acetamide, trimethyl phosphate, N-methyl-2-pyrolidone, 1,3-dimethyl-2-imidazolidinone, sulfolane, and other aprotic polar solvents; formic acid, acetic acid, propionic acid, butyric acid, lactic acid, and other carboxylic acid solvents; anisole, diethyl ether, tetrahydrofuran, diisopropyl ether, dioxane, diglyme, dimethoxy ethane, and other ether based solvents; and water.

From the viewpoint of efficient production of particles from polymer A, preferable ones include aromatic hydrocarbon based solvents, aliphatic hydrocarbon based solvents, alcohol based solvents, ether based solvents, and water, of which the most preferable are alcohol based solvent and water, and particularly preferable is water.

For the present method, synthetic polymer particles containing a daughter particle component can be produced when polymer A, polymer B, an organic solvent that dissolves them, and a poor solvent for polymer A are appropriately selected and used in combination to allow polymer A to be precipitated efficiently.

Here, it is necessary for the liquid prepared by mixing and dissolving polymers A and B, a daughter particle component, and an organic solvent that dissolves them is phase-separated to form a solution phase composed mainly of polymer A and a solution phase composed mainly of polymer B. Here, the daughter particle component commonly forms a solid phase instead of being dissolved, but it does not act as a phase as mentioned in the above description.

Note that the organic solvent used for the solution phase composed mainly of polymer A and organic solvent used for the solution phase composed mainly of polymer B may be either identical or different, but it is preferable that they are substantially identical.

The conditions to develop a state of two phase separation varies depending on types of polymers A and B, molecular weight of polymers A and B, type of the organic solvents, concentration of polymers A and B, and the temperature and pressure where the present method is to be carried out.

To produce conditions where phase separation easily takes place, it is preferable that the solubility parameter (which may be hereinafter referred to as SP value) of polymer A is largely different from that of polymer B.

Here, the difference in SP value is 1 $(J/cm^3)^{1/2}$ or more, more preferably 2 $(J/cm^3)^{1/2}$ or more, still more preferably 3 $(J/cm^3)^{1/2}$ or more, particularly preferably 5 $(J/cm^3)^{1/2}$ or more, and extremely preferably 8 $(J/cm^3)^{1/2}$ or more. Phase separation is caused easy if the SP value is in this range.

There are no specific limitations if both polymer A and polymer B can be dissolved in the organic solvent used, but the upper limit on the difference in SP value is preferably 20 $(J/cm^3)^{1/2}$ or less, more preferably 15 $(J/cm^3)^{1/2}$ or less, and still more preferably 10 $(J/cm^3)^{1/2}$ or less.

The SP value as referred to herein is calculated according to Fedor's estimation method and calculated based on cohesive energy density and molecular volume (hereinafter, sometimes referred simply to calculation method) ("SP-chi: Kiso • Oyo to Keisan Hoho (SP Value: Basics/Applications and Calculation Method), Hideki Yamamoto, Johokiko Co., Ltd, Mar. 31, 2005).

If the solubility parameter cannot be calculated by the estimation method, the SP value is calculated by the experiment method in which it is determined based on whether the polymer can dissolve in solvents with a known solubility parameter (hereinafter, sometimes referred to simply as experiment method) and used instead (Polymer Handbook Fourth Edition, J. Brand, Wiley, 1998).

Conditions to cause phase separation are set up according to a three-component phase diagram, which can be prepared based on a simple preliminary experiment to observe various states at different ratios of the three components

of polymer A, polymer B and an organic solvent that dissolves them.

A phase diagram can be prepared by mixing and dissolving polymer A, polymer B and an organic solvent at least 3 or more, preferably 5 or more, and more preferably 10 or more, appropriately selected proportions, leaving them to stand, and determining whether an interface develops, and conditions for phase separation are identified based on differentiation of regions where two phases are formed and those where only one phase is formed.

To determine whether a liquid is in a phase separation state, polymers A and B, and a solvent are mixed at an appropriate proportion under the temperature and pressure conditions where the present invention is to be implemented, and subsequently, polymers A and B are dissolved completely, stirred adequately after the dissolution, left to stand for 3 days, and observed whether macroscopic phase separation has occurred.

In the case where a sufficiently stable emulsion is formed, however, macroscopic phase separation may not take place even when the liquid is left to stand for 3 days. In that case, occurrence of phase separation is judged by using an optical microscope or phase contrast microscope to observe whether microscopic phase separation takes place.

Phase separation occurs as a result of separation between the polymer A solution phase composed mainly of polymer A and the polymer B solution phase composed mainly of polymer B in the organic solvent. Here, the polymer A solution phase is the phase to which mainly polymer A has been distributed while the polymer B solution phase is the phase to which mainly polymer B has been distributed. Here, the polymer A solution phase and the polymer B solution phase each appears to have a volume ratio depending on the type and content of polymer A and polymer B, respectively.

The contents of polymers A and B should, as a prerequisite, be such that a phase separation state can develop and also that they make the method industrially feasible and are as high as possible in the organic solvent, but they are preferably more than 1 mass% to 50 mass%, more preferably more than 1 mass% to 30 mass%, and still more preferably more than 2 mass% to 20 mass%.

With respect to the quantity of the daughter particle component, an appropriate daughter particle content is determined taking into consideration the ratio of distribution to the polymer A solution phase in the phase separation state and the quantity of porous state after calcination so that an intended porosity is achieved, but commonly, the content of the daughter particles is in the range of 0.1 parts by mass to 100 parts by mass, preferably 0.1 parts by mass to 80 parts by mass, more preferably 0.1 parts by mass to 50 parts by mass, and particularly preferably 1 part by mass to 30 parts by mass, per 100 parts by mass of polymer A component.

With respect to the polymer A solution phase and the polymer B solution phase for the present method, the interfacial tension between the two phases is small since both phases are of an organic solvent, and this nature allows the resulting emulsion to be maintained stable, possibly serving to decrease the particle diameter distribution index. In particular, this effect is noticeable when polymer A phase and polymer B phase are formed of the same organic solvent.

For the present method, the interfacial tension between the two phases are so small that the interfacial tension cannot be measured directly by the commonly used hanging-drop method in which measurements are made by adding a dissimilar solution to the solution, but the interfacial tension can be estimated from the surface tension of each phase in air. Denoting the surface tension of each phase in air by $r_1$ and $r_2$, the interfacial tension between them, $r_{12}$, is estimated as the absolute value of $r_{12} = r_1 - r_2$. Here, the value of $r_{12}$ is preferably in the range of more than 0 to 10 mN/m, more preferably more than 0 to 5 mN/m, still more preferably more than 0 to 3 mN/m, and particularly preferably more than 0 to 2 mN/m.

For the present method, the viscosity between the two phases has influence on the average particle diameter and particle diameter distribution, and the particle diameter distribution index tends to decrease with an decreasing viscosity ratio. If it is assumed that the viscosity ratio is defined as the ratio of the polymer A solution phase / polymer in the solution phase B under the temperature conditions where the present invention is to be implemented, it is preferably in the range of 0.1 or more and 10 or less, more preferably in the range of 0.2 or more and 5 or less, still more preferably in the range of 0.3 or more and 3 or less, particularly preferably in the range of 0.5 or more and 1.5 or less, and extremely preferably in the range of 0.8 or more and 1.2 or less.

A phase-separated system thus obtained is used to produce daughter-particle-containing polymer particles. A common reaction tank is used to produce particles. From the viewpoint of industrial practicability, the temperature range suitable for implementing the present invention is -50°C to 200°C, preferably -20°C to 150°C, more preferably 0°C to 120°C, still more preferably 10°C to 100°C, particularly preferably 20°C to 80°C, and most preferably 20°C to 50°C. From the viewpoint of industrial practicability, the pressure range suitable for implementing the present invention is from a reduced pressure to 100 atm, preferably 1 atm to 5 atm, more preferably 1 atm to 2 atm, and particularly preferably atmospheric pressure.

An emulsion is formed by mixing the components under such conditions to develop a phase-separated state.

Specifically, an emulsion is produced by applying a shear force to the resulting phase-separated solution.

In the emulsion formation step, an emulsion is produced in such a way that the polymer A solution phase forms particle-like droplets. It is commonly seen that when a solution is phase-separated, such an emulsion tends to form easily when the volume of the polymer B solution phase is larger than that of the polymer A solution phase, and the ratio of the volume of the polymer A solution phase to that of the total volume of the two phases is preferably 0.4 or less,

more preferably in the range of 0.4 to 0.1. When preparing said phase diagram, an appropriate range can be set up if the volume ratio of each component is measured at the same time.

The present production process aims to add a daughter particle component into daughter-particle-containing polymer particles. Various studies have been carried out and results show that in order to add a daughter particle component efficiently and homogenously to form daughter-particle-containing polymer particles, it is preferable that the daughter particle component is added at a point before the emulsion formation step in the process to be carried out for producing daughter-particle-containing polymer particles.

The addition of a daughter particle component may be performed during the emulsion formation step, before the emulsion formation step, or after the emulsion formation step, it is preferable that it is performed before the emulsion formation step. The addition of a daughter particle component before the emulsion formation step is preferable because it serves to increase the number of hollow structures in the resulting porous carbon particles.

Daughter-particle-containing polymer particles produced by the present production method are in the form of particles with a narrow particle diameter distribution, which is made possible because a highly uniform emulsion is formed in the emulsion formation step. This tendency is noticeable when a single solvent that can dissolve both polymers A and B is used. Thus, the use of a conventionally known stirring method is sufficiently effective to produce a shear force required for the emulsion formation, and examples of said method to be used for mixing include liquid phase stirring by stirring blades, stirring by a continuous biaxial stirrer, mixing by a homogenizer, and ultrasonic irradiation.

When agitating blades are used for stirring, in particular, the stirring speed is preferably 50 rpm to 1200 rpm, more preferably 100 rpm to 1000 rpm, still more preferably 200 rpm to 800 rpm, and particularly preferably 300 rpm to 600 rpm, depending on the shape of the agitating blades used.

The agitating blade may specifically be in the shape of propeller, paddle, flat paddle, turbine, double cone, single cone, single ribbon, double ribbon, screw, or helical ribbon, but it is not limited thereto as long as a sufficient shear force can be applied to the system. In addition, baffle boards etc. may be provided in the tank to ensure efficient stirring.

For formation of an emulsion, not only a stirrer but also other generally known tools such as emulsifier and dispersing machine may be used. Specific examples include Homogenizer (supplied by IKA), Polytron (supplied by Kinematica, Inc.), T. K. Autohomemixer (supplied by Tokushu Kika Kogyo Co., Ltd.), other batch-wise emulsifiers, Ebara Milder (supplied by Ebara Corporation), T. K. Filmix, T. K. Pipeline Homomixer (supplied by Tokushu Kika Kogyo Co., Ltd.), Colloid Mill (supplied by Shinko-Pantec Co., Ltd.), Slusher, Trigonal Wet Grinder (supplied by Mitsui Miike Kakoki Co., Ltd.), ultrasonic homogenizers, and static mixers.

The resulting emulsion is then fed to a step for precipitating daughter-particle-containing polymer particles.

To produce daughter-particle-containing polymer particles, a poor solvent for polymer A is brought into contact with the emulsion produced in the above step to precipitate daughter-particle-containing polymer particles having a diameter corresponding to that of the emulsion droplets.

Bringing the poor solvent and the emulsion into contact with each other may be achieved by pouring the emulsion in the poor solvent, and pouring the poor solvent into the emulsion, of which pouring the poor solvent into the emulsion is referable.

Here, there are no specific limitations on the method to be used to pour the poor solvent as long as daughter-particle-containing polymer particles produced according to the present invention are obtained, and the useful methods include continuous dropping, divided addition, and on-step addition. In order to prevent widening in the particle diameter distribution and formation of bulky lumps exceeding 1,000 μm from being caused by coagulation, fusion, or coalescence of the emulsion at the time of adding the poor solvent, it is preferable to use continuous dropping and divided dropping, and it is most preferable to use continuous dropping to ensure industrially efficient implementation.

The duration of adding the poor solvent is 10 min or more and 50 hours or less, more preferably 30 min or more and 10 hours or less, and still more preferably 1 hour or more and 5 hours or less.

If the implementation duration is shorter than these ranges, widening in the particle diameter distribution and formation of bulky lumps may result from coagulation, fusion, or coalescence of the emulsion. An implementation duration longer than them is impractical from an industrial point of view.

Implementation in these ranges makes it possible to depress coagulation of particles during conversion from an emulsion into daughter-particle-containing polymer particles, thereby providing precursor particles that contain daughter particles with a narrow particle diameter distribution.

Depending on the conditions of the emulsion, the quantity of the poor solvent to be added is preferably 0.1 part by mass to 10 parts by mass, more preferably 0.1 part by mass to 5 parts by mass, still more preferably 0.2 part by mass to 3 parts by mass, particularly preferably 0.2 part by mass to 1 part by mass, and most preferably 0.2 part by mass to 0.5 part by mass, per 1 part by mass of the total weight of the emulsion.

There are no specific limitations on the duration of contact between the poor solvent and the emulsion as long as it is sufficiently long for precipitation of daughter-particle-containing polymer particles, but in order to cause precipitation adequately and ensure high productivity, it is 5 min to 50 hours, preferably 5 min or more and 10 hours or less, more preferably 10 min or more and 5 hours or less, particularly preferably 20 min or more and 4 hours or less, and extremely

preferably 30 min or more and 3 hours or less, after the end of addition of the poor solvent.

From a dispersion liquid of daughter-particle-containing polymer particles thus obtained, particulate powder can be recovered by solid-liquid separation using a generally known method such as filtration, decantation, vacuum filtration, compression filtration, centrifugal separation, centrifugal filtration, spray dry, acid precipitation, salt precipitation, and freeze coagulation.

The daughter-particle-containing polymer particles obtained by solid-liquid separation is cleaned with a solvent etc., as required, to remove impurities etc. attached or contained, and then purified. Preferable solvents to be used here include the poor solvents mentioned above, and more preferable is a solvent selected from the group of water, methanol, and ethanol, or a mixture of two or more thereof.

The resulting daughter-particle-containing polymer particles may be dried to remove the residual solvent. The drying methods that can be used here include air drying, heat drying, vacuum drying, and freeze drying. The temperature used for heating is preferably lower than the glass transition temperature, and specifically, a temperature of 50 to 150°C is preferable.

The method according to the present invention is advantageous in that the organic solvent, polymer B, and/or the daughter particle component that are separated in the solid-liquid separation step carried out to obtain daughter-particle-containing polymer particles can be recycled to ensure their efficient use.

The solvent resulting from the solid-liquid separation step is actually a mixture of polymer B, organic solvent, poor solvent, and/or daughter particle component. Removal of the poor solvent from the solvent component allows the remaining solvent mixture to be recycled for emulsion formation.

Some part of the daughter particle component is not incorporated in the daughter-particle-containing polymer particles and left in this solvent mixture, and if it is recycled, there will be no loss of the daughter particle component over the entire production process.

For removing the poor solvent, generally known methods may be used, including, specifically, simple distillation, reduced pressure distillation, precision distillation, thin film distillation, extraction, and membrane separation, of which simple distillation, reduced pressure distillation, and precision distillation ate preferable.

Heat is applied to the system when performing distillation operations such as simple distillation and reduced pressure distillation, possibly accelerating the thermal decomposition of polymer B and organic solvents, and therefore, they are performed in an atmosphere with as low an oxygen content as possible, preferably in an inactive atmosphere. Specifically, they should be performed in an atmosphere of nitrogen, helium, argon, or carbon dioxide.

The carbonization method for daughter-particle-containing polymer particles is described specifically below.

The carbonizing step is carried out by heating the daughter-particle-containing polymer particles in an inert gas atmosphere that does not react with the daughter-particle-containing polymer particles. It is preferable to use an inert gas such as helium, argon, and nitrogen.

The atmosphere used for heating may be either a flow type or a closed type, but a flow type atmosphere is preferable because the gas generated by heating can be removed. With respect to pressure, heating may be performed under either compressed pressure or reduced pressure, but commonly, it is performed under atmospheric pressure. When carried out under atmospheric pressure, heating is performed at a temperature of 400°C or more, preferably 800°C or more. There are no specific limitations on the upper limit, but it is commonly 3,000°C and preferably 2,500°C.

The degree of graphitization of the porous carbon particles tends to increase with an increasing heating temperature and an increasing duration during which the heating temperature is maintained. The heating temperature is preferably 400°C or more, more preferably 800°C or more, and most preferably 1000°C or more.

The heating rate to reach the heating temperature is commonly 20°C/min or less, preferably 10°C/min or less, and more preferably 5°C/min or less. As the calcination temperature exceeds 20°C/min, the particles tend to undergo fusion.

Depending on the heating temperature, the heating-temperature holding duration is preferably 0.5 hour or more, more preferably 1 hour or more, still more preferably 5 hours or more, particularly preferably 10 hours or more, extremely considerably preferably 50 hours or more, as lower limit, and it is preferably 1,000 hours or less, more preferably 500 hours or less, and still more preferably 100 hours or less, as upper limit. With respect to the heating temperature, furthermore, the particles may be maintained at an appropriate temperature for an appropriate time, followed by further heating up to a desired heating temperature.

Said method may also be used in the case where the daughter-particle-containing polymer particles are carbonized, and it is preferable to perform pre-treatment as described below because it serves to allow the daughter-particle-containing polymer particles to form an oxidized structure and also serves to improve the carbonization yield and prevent fusion of particles.

An oxidized structure can be formed by heating the daughter-particle-containing polymer particles in an oxidizing atmosphere. Said oxidizing atmosphere may contain oxygen, sulfur, or the like, but it is preferably an air atmosphere. There are no specific limitations on the heating temperature as long as an oxidized structure is formed, but it is 100 to 300°C, preferably 150 to 250°C. A heating temperature of less than 100°C is not preferable because in that case, the

formation of an oxidized structure does not progress, while the daughter-particle-containing polymer particles start to melt before an oxidized structure is formed if the temperature is above 300°C. The heating rate to reach the heating temperature is commonly 20°C/min or less, preferably 10°C/min or less, and more preferably 5°C/min or less. A calcination temperature of above 20°C/min is not preferable because it causes fusion of the particles. The heating temperature, furthermore, may be maintained for an appropriate time after heating up to a predetermined temperature. Depending on the heating temperature, the heating duration is 0.5 to 10 hours, preferably 0.5 to 5 hours, at the most. With respect to the heating temperature, furthermore, the particles may be maintained at an appropriate temperature for an appropriate time, followed by further heating up to a desired heating temperature.

The carbon particles, metal-containing carbon particles and porous carbon particles produced according to the present invention have a narrow particle diameter distribution to show high dispersibility and gap-filling capability when used with resin or rubber, and has a high degree of graphitization to serve effectively in producing a resin modifying agent, electric conductive rubber, anisotropically conductive particles, chromatographic carrier, molecule adsorption agent, toner, negative electrode material for lithium ion batteries, catalyst for fuel cells, catalyst carrier, activated carbon, electrode for capacitor, carrier for diagnosis, and contrast dye for MRI.

## Examples

A more detailed description is given below with reference to Examples. Note that the present invention is not limited to these examples. The measurements used in Examples were made as described below.

### (1) Measurement of weight average molecular weight

The weight average molecular weight was determined by gel permeation chromatography, and molecular weight calculation was carried out based on comparison with a calibration curve prepared with polystyrene.

Equipment: LC-10Aseries, supplied by Shimadzu Corporation
Column: KD-806M, supplied by Showa Denko K.K.
Mobile phase: 10 mmol/L lithium bromide / dimethyl formamide solution
Flow rate: 1.0 ml/min
Detector: differential refractometer
Column temperature: 40°C

### (2) Calculation methods for number average particle diameter, volume average particle diameter, and particle diameter distribution index

For measuring the number average particle diameter, a scanning electronic microscope (model JSM-6301NF scanning electronic microscope, supplied by JEOL Ltd.) was used to observe particles. Here, if a particle is not perfectly circular, its major axis was measured and used as its particle diameter.
The number average particle diameter (Dn) and the volume average particle diameter (Dv) were calculated by Equations (1) and (2), respectively, based on the average of measurements over 100 randomly selected particles.
The particle diameter distribution index (PDI) was calculated by Equation (3).
[Mathematical formula 13]

$$Dn = \frac{\sum_{i=1}^{n} Ri}{n} \quad (1)$$

$$Dv = \frac{\sum_{i=1}^{n} Ri^{4}}{\sum_{i=1}^{n} Ri^{3}} \quad (2)$$

$$PDI = \frac{Dv}{Dn} \quad (3)$$

Here, Ri, n, Dn, Dv, and PDI denote the particle diameter of a particular particle, number of measurements (100), number average particle diameter, volume average particle diameter, and particle diameter distribution index, respectively.

(3) Measurement of sphericity

The sphericity was determined by observing particles by scanning electronic microscopy (model JSM-6301NF scanning electronic microscope, supplied by JEOL Ltd.), measuring their major and minor axes, and making calculations from the average over randomly selected 30 particles by Equation (4).

[Mathematical formula 14]

$$\text{Sphericity} = \frac{\sum_{i=1}^{n}(\text{minor axes} / \text{major axes})}{n} \times 100 \quad (4)$$

Here, n denotes the number of measurements, which is equal to 30.

(4) Degree of graphitization

Carbon particles were loaded in a resonance Raman spectrometer (INF-300, supplied by HORIBA Jobin Yvon S.A.S.), and measurements were made using laser with a wavelength of 532 nm, followed by calculating the ratio (B)/(A) where (A) and (B) represent the height at 1360±100 $cm^{-1}$ and that at 1580±100 $cm^{-1}$, respectively.

(5) Quantitative analysis of metal content

Measurements were made by ICP mass analysis (model 4500, supplied by Agilent) and ICP luminescence spectroscopy analysis (Optima 4300DV, supplied by Perkin Elmer). Specimens were prepared as follows.

A 0.1 g amount of metal-containing carbon particles were heated and decomposed in sulfuric acid and nitric acid. This specimen solution was concentrated by heating until sulfuric acid started to release fumes, and diluted nitric acid was added to an appropriate volume, followed by quantitative analysis using the above devices.

(6) Measurement of diameter of pore structures and daughter particles Particles were fixed with epoxy resin for electronic microscopy, and a sectioned specimen was cut out and observed by scanning electronic microscopy at a magnification of 5,000 for analysis of the pore structures and porous state in porous carbon particles or carbon particles.

[Production example 1]

First, 99 parts by mass of acrylonitrile and 1 part by mass of itaconic acid were dissolved in 400 parts by mass of dimethyl sulfoxide in a reaction vessel equipped with a condenser and blade stirrer, and then 0.5 parts by mass of benzoyl peroxide was added, followed by heating the mixture up to 70°C and maintaining it there for 6 hours to achieve polymerization, thereby preparing a polyacrylonitrile solution. The solution had a weight average molecular weight of 530,000. To the solution, 172 parts by mass of polyvinyl alcohol (Gohsenol (registered trademark) GL-05, weight average molecular weight 10,600, SP value 32.8 $(J/cm^3)^{1/2}$, supplied by Nippon Synthetic Chemical Industry Co., Ltd. industry) and 2,776 parts by mass of dimethyl sulfoxide were added, followed by heating and stirring the mixture at 80°C until the polymer was dissolved completely. After lowering the temperature of the system back to room temperature, 1,500 parts by mass of ion-exchanged water, which was used as poor solvent, was added through a water supply pump at a rate of 25 parts by mass/min while stirring the solution at 450 rpm, thereby precipitating particles. The resulting particles were isolated, rinsed, and dried. The resulting polyacrylonitrile copolymer particles had a number average particle diameter of 8.0 μm, particle diameter distribution index of 1.30, and sphericity of 91. A scanning electronic microscope photograph of the resulting particles is given in Fig. 1. Note that the SP value of this polymer was calculated at 29.5 $(J/cm^3)^{1/2}$.

[Production example 2]

First, 100 parts by mass of acrylonitrile was dissolved in 400 parts by mass of dimethyl sulfoxide in a reaction vessel equipped with a condenser and blade stirrer, and then 0.5 parts by mass of benzoyl peroxide was added, followed by heating the mixture up to 70°C and maintaining it there for 6 hours to achieve polymerization, thereby preparing a polyacrylonitrile solution. The solution had a weight average molecular weight of 470,000. To the solution, 100 parts by mass of polyvinyl alcohol (Gohsenol (registered trademark) GL-05, weight average molecular weight 10,600, SP value 32.8 $(J/cm^3)^{1/2}$, supplied by Nippon Synthetic Chemical Industry Co., Ltd. industry) and 1400 parts by mass of dimethyl sulfoxide were added, followed by heating and stirring the mixture at 80°C until the polymer was dissolved completely. After lowering the temperature of the system back to room temperature, 1,500 parts by mass of ion-exchanged water, which was used as poor solvent, was added through a water supply pump at a rate of 25 parts by mass/min while stirring the solution at 450 rpm, thereby precipitating particles. The resulting particles were isolated, rinsed, and dried. The resulting polyacrylonitrile particles had a number average particle diameter of 4.6 μm, particle diameter distribution index of 1.16, and sphericity of 96. A scanning electronic microscope photograph of the resulting particles is given in Fig. 2. Note that the SP value of this polymer was calculated at 29.5 $(J/cm^3)^{1/2}$.

[Production example 3]

First, 99 parts by mass of acrylonitrile and 1 part by mass of itaconic acid were dissolved in 400 parts by mass of dimethyl sulfoxide in a reaction vessel equipped with a condenser and blade stirrer, and then 0.5 parts by mass of benzoyl peroxide was added, followed by heating the mixture up to 70°C and maintaining it there for 6 hours to achieve polymerization, thereby preparing a polyacrylonitrile solution. The solution had a weight average molecular weight of 530,000. To the solution, 119 parts by mass of polyvinyl alcohol (Gohsenol (registered trademark) GL-05, weight average molecular weight 10,600, SP value 32.8 $(J/cm^3)^{1/2}$, supplied by Nippon Synthetic Chemical Industry Co., Ltd. industry) and 1761 parts by mass of dimethyl sulfoxide were added, followed by heating and stirring the mixture at 80°C until the polymer was dissolved completely. After lowering the temperature of the system down to 60°C, 2,380 parts by mass of ion-exchanged water, which was used as poor solvent, was added through a water supply pump at a rate of 20 parts by mass/min while stirring the solution at 450 rpm, thereby precipitating particles. The resulting particles were isolated, rinsed, and dried. The resulting polyacrylonitrile copolymer particles had a number average particle diameter of 19.1 μm, particle diameter distribution index of 1.38, and sphericity of 91. Note that the SP value of this polymer was calculated at 29.5 $(J/cm^3)^{1/2}$.

[Production example 4]

First, 99 parts by mass of acrylonitrile and 1 part by mass of itaconic acid were dissolved in 400 parts by mass of dimethyl sulfoxide in a reaction vessel equipped with a condenser and blade stirrer, and then 0.5 parts by mass of benzoyl peroxide was added, followed by heating the mixture up to 70°C and maintaining it there for 6 hours to achieve polymerization, thereby preparing a polyacrylonitrile solution. The solution had a weight average molecular weight of 530,000. To the solution, 172 parts by mass of polyvinyl alcohol (Gohsenol (registered trademark) GL-05, weight average molecular weight 10,600, SP value 32.8 $(J/cm^3)^{1/2}$, supplied by Nippon Synthetic Chemical Industry Co., Ltd. industry), 2,776 parts by mass of dimethyl sulfoxide, and 10 parts by mass of phthalocyanine iron (supplied by Wako Pure Chemical Industries, Ltd.) were added, followed by heating and stirring the mixture at 80°C until the polymer was dissolved

completely. After lowering the temperature of the system back to room temperature, 1,500 parts by mass of ion-exchanged water, which was used as poor solvent, was added through a water supply pump at a rate of 25 parts by mass/min while stirring the solution at 450 rpm, thereby precipitating particles. The resulting particles were isolated, rinsed, and dried to provide polyacrylonitrile copolymer particles containing phthalocyanine iron. The resulting polyacrylonitrile copolymer particles had a number average particle diameter of 7.5 μm, particle diameter distribution index of 1.40, and sphericity of 91, and quantitative analysis for the metal content in the metal-containing synthetic polymer particles showed that 4.8 mg of iron was contained per gram of the metal-containing synthetic polymer particles. Note that the SP value of the polyacrylonitrile copolymer was calculated at 29.5 $(J/cm^3)^{1/2}$.

[Production example 5]

First, 99 parts by mass of acrylonitrile and 1 part by mass of itaconic acid were dissolved in 400 parts by mass of dimethyl sulfoxide in a reaction vessel equipped with a condenser and blade stirrer, and then 0.5 parts by mass of benzoyl peroxide was added, followed by heating the mixture up to 70°C and maintaining it there for 6 hours to achieve polymerization, thereby preparing a polyacrylonitrile solution. The solution had a weight average molecular weight of 530,000. To the solution, 172 parts by mass of polyvinyl alcohol (Gohsenol (registered trademark) GL-05, weight average molecular weight 10,600, SP value 32.8 $(J/cm^3)^{1/2}$, supplied by Nippon Synthetic Chemical Industry Co., Ltd. industry), 10 parts by mass of organic particles (Metabrane (registered trademark) C223A, particle diameter 0.2 μm, supplied by Mitsubishi Rayon Co., Ltd.), and 2,776 parts by mass of dimethyl sulfoxide were added, followed by heating and stirring the mixture at 80°C until the polymer was dissolved completely. After lowering the temperature of the system back to room temperature, 1,500 parts by mass of ion-exchanged water, which was used as poor solvent, was added through a water supply pump at a rate of 25 parts by mass/min while stirring the solution at 450 rpm, thereby precipitating particles. The resulting particles were isolated, rinsed, and dried. The resulting polyacrylonitrile copolymer particles had a number average particle diameter of 11.6 μm, particle diameter distribution index of 1.35, and sphericity of 93. Note that the SP value of this polymer was calculated at 29.5 $(J/cm^3)^{1/2}$. Observation of the cross sections of the daughter-particle-containing polymer particles showed that daughter particles of 0.2 μm were contained.

[Production example 6]

First, 99 parts by mass of acrylonitrile and 1 part by mass of itaconic acid were dissolved in 400 parts by mass of dimethyl sulfoxide in a reaction vessel equipped with a condenser and blade stirrer, and then 0.5 parts by mass of benzoyl peroxide was added, followed by heating the mixture up to 70°C and maintaining it there for 6 hours to achieve polymerization, thereby preparing a polyacrylonitrile solution. The solution had a weight average molecular weight of 530,000. To the solution, 172 parts by mass of polyvinyl alcohol (Gohsenol (registered trademark) GL-05, weight average molecular weight 10,600, SP value 32.8 $(J/cm^3)^{1/2}$, supplied by Nippon Synthetic Chemical Industry Co., Ltd. industry), 10 parts by mass of organic particles (Kane Ace (registered trademark) FM50, particle diameter 0.2 μm, supplied by Kaneka Corporation), and 2,776 parts by mass of dimethyl sulfoxide were added, followed by heating and stirring the mixture at 80°C until the polymer was dissolved completely. After lowering the temperature of the system back to room temperature, 1,500 parts by mass of ion-exchanged water, which was used as poor solvent, was added through a water supply pump at a rate of 25 parts by mass/min while stirring the solution at 450 rpm, thereby precipitating particles. The resulting particles were isolated, rinsed, and dried. The resulting polyacrylonitrile copolymer particles had a number average particle diameter of 8.6 μm, particle diameter distribution index of 1.32, and sphericity of 94. Note that the SP value of this polymer was calculated at 29.5 $(J/cm^3)^{1/2}$. Observation of the cross sections of the daughter-particle-containing polymer particles showed that daughter particles of 0.2 μm were contained.

[Production example 7]

First, 15 parts by mass of polyamide-imide (TI 5013E-P, weight average molecular weight 66,000, supplied by Toray Industries, Inc.), 30 parts by mass of N-methyl-2-pyrrolidinone, 5 parts by mass of polyvinyl alcohol (Gohsenol (registered trademark) GL-05, weight average molecular weight 10,600, SP value 32.8 $(J/cm^3)^{1/2}$, supplied by Nippon Synthetic Chemical Industry Co., Ltd. industry) were put in a reaction vessel equipped with a condenser and blade stirrer, followed by heating and stirring the mixture at 80°C until the polymer was dissolved completely. After lowering the temperature of the system back to room temperature, 50 parts by mass of ion-exchanged water, which was used as poor solvent, was added through a water supply pump at a rate of 3.2 parts by mass/min while stirring the solution at 450 rpm, thereby precipitating particles. The resulting particles were isolated, rinsed, and dried. The resulting polyacrylonitrile particles had a number average particle diameter of 21.4 μm, particle diameter distribution index of 1.22, and sphericity of 97. Note that the SP value of this polymer was calculated at 31.0 $(J/cm^3)^{1/2}$. A scanning electronic microscope photograph of the resulting particles is given in Fig. 5.

[Production example 8]

First, 5 parts by mass of polyamide-imide (TI 5013E-P, weight average molecular weight 66,000, supplied by Toray Industries, Inc.), 40 parts by mass of dimethyl sulfoxide, 5 parts by mass of polyvinyl alcohol (Gohsenol (registered trademark) GL-05, weight average molecular weight 10,600, SP value 32.8 $(J/cm^3)^{1/2}$, supplied by Nippon Synthetic Chemical Industry Co., Ltd. industry) were put in a reaction vessel equipped with a condenser and blade stirrer, followed by heating and stirring the mixture at 80°C until the polymer was dissolved completely. After lowering the temperature of the system back to room temperature, 50 parts by mass of ion-exchanged water, which was used as poor solvent, was added through a water supply pump at a rate of 0.4 parts by mass/min while stirring the solution at 450 rpm, thereby precipitating particles. The resulting particles were isolated, rinsed, and dried. The resulting polyacrylonitrile particles had a number average particle diameter of 4.9 μm, particle diameter distribution index of 1.41, and sphericity of 97. Note that the SP value of this polymer was calculated at 31.0 $(J/cm^3)^{1/2}$.

[Production example 9]

First, 96 parts by mass of acrylonitrile, 3 parts by mass of methacrylic acid, and 1 part by mass of itaconic acid were dissolved in 400 parts by mass of dimethyl sulfoxide in a reaction vessel equipped with a condenser and blade stirrer, and then 0.5 parts by mass of benzoyl peroxide was added, followed by heating the mixture up to 70°C and maintaining it there for 6 hours to achieve polymerization, thereby preparing a polyacrylonitrile based copolymer solution. The solution had a weight average molecular weight of 560,000. To the solution, 172 parts by mass of polyvinyl alcohol (Gohsenol (registered trademark) GL-05, weight average molecular weight 10,600, SP value 32.8 $(J/cm^3)^{1/2}$, supplied by Nippon Synthetic Chemical Industry Co., Ltd. industry) and 2,776 parts by mass of dimethyl sulfoxide were added, followed by heating and stirring the mixture at 80°C until the polymer was dissolved completely. After lowering the temperature of the system back to room temperature, 1,500 parts by mass of ion-exchanged water, which was used as poor solvent, was added through a water supply pump at a rate of 25 parts by mass/min while stirring the solution at 450 rpm, thereby precipitating particles. The resulting particles were isolated, rinsed, and dried. The resulting polyacrylonitrile copolymer particles had a number average particle diameter of 6.7 μm, particle diameter distribution index of 1.36, and sphericity of 88. Note that the SP value of this polymer was calculated at 29.5 $(J/cm^3)^{1/2}$.

[Production example 10]

First, 95 parts by mass of acrylonitrile, 4 parts by mass of methyl acrylate, and 1 part by mass of itaconic acid were dissolved in 400 parts by mass of dimethyl sulfoxide in a reaction vessel equipped with a condenser and blade stirrer, and then 0.5 parts by mass of benzoyl peroxide was added, followed by heating the mixture up to 70°C and maintaining it there for 6 hours to achieve polymerization, thereby preparing a polyacrylonitrile based copolymer solution. The solution had a weight average molecular weight of 490,000. To the solution, 172 parts by mass of polyvinyl alcohol (Gohsenol (registered trademark) GL-05, weight average molecular weight 10,600, SP value 32.8 $(J/cm^3)^{1/2}$, supplied by Nippon Synthetic Chemical Industry Co., Ltd. industry) and 2,776 parts by mass of dimethyl sulfoxide were added, followed by heating and stirring the mixture at 80°C until the polymer was dissolved completely. After lowering the temperature of the system back to room temperature, 1,500 parts by mass of ion-exchanged water, which was used as poor solvent, was added through a water supply pump at a rate of 25 parts by mass/min while stirring the solution at 450 rpm, thereby precipitating particles. The resulting particles were isolated, rinsed, and dried. The resulting polyacrylonitrile copolymer particles had a number average particle diameter of 8.2 μm, particle diameter distribution index of 1.40, and sphericity of 90. Note that the SP value of this polymer was calculated at 29.5 $(J/cm^3)^{1/2}$.

[Example 1]

A 10.0 g portion of the polyacrylonitrile copolymer particles prepared in Production example 1 was put on a porcelain dish, heated for 50 min to reach 250°C in air in a muffle furnace (FP41, supplied by Yamato Co., Ltd.), and maintained there for 1 hour. Subsequently, it was heated for 2 hours and 30 min to reach 1,000°C in a nitrogen flow to provide 3.3 g of carbon particles. The resulting carbon particles had a number average particle diameter of 5.7 μm, particle diameter distribution index of 1.35, and sphericity of 90, and the height ratio (B)/(A), which represents the degree of graphitization, was 1.03. A scanning electronic microscope photograph of the resulting particles is given in Fig. 3.

[Example 2]

A 10.0 g portion of the polyacrylonitrile copolymer particles prepared in Production example 1 was put on a porcelain dish, heated for 50 min to reach 250°C in air in a muffle furnace (FP41, supplied by Yamato Co., Ltd.), and

maintained there for 1 hour. Subsequently, it was heated for 3 hours and 30 min to reach 1,300°C in a nitrogen flow to provide 2.2 g of carbon particles. The resulting carbon particles had a number average particle diameter of 7.1 μm, particle diameter distribution index of 1.32, and sphericity of 81, and the height ratio (B)/(A), which represents the degree of graphitization, was 1.07.

[Example 3]

A 10.0 g portion of the polyacrylonitrile copolymer particles prepared in Production example 3 was put on a porcelain dish, heated for 50 min to reach 250°C in air in an atmosphere furnace (FP41, supplied by Yamato Co., Ltd.), and maintained there for 1 hour. Subsequently, it was heated for 2 hours and 30 min to reach 1,000°C in a nitrogen flow to provide 5.3 g of carbon particles. The resulting carbon particles had a number average particle diameter of 17.7 μm, particle diameter distribution index of 1.26, and sphericity of 88, and the height ratio (B)/(A), which represents the degree of graphitization, was 1.00.

[Example 4]

A 10.0 g portion of the polyamide-imide particles prepared in Production example 7 was put on a porcelain dish, heated for 50 min to reach 250°C in air in a muffle furnace (FP41, supplied by Yamato Co., Ltd.), and maintained there for 1 hour. Subsequently, it was heated for 3 hours and 30 min to reach 1,000°C in a nitrogen flow to provide 5.5 g of carbon particles. The resulting carbon particles had a number average particle diameter of 18.6 μm, particle diameter distribution index of 1.30, and sphericity of 94, and the height ratio (B)/(A), which represents the degree of graphitization, was 1.00. A scanning electronic microscope photograph of the resulting carbon particles is given in Fig. 6.

[Example 5]

A 10.0 g portion of the polyamide-imide particles prepared in Production example 8 was put on a porcelain dish, heated for 50 min to reach 250°C in air in a muffle furnace (FP41, supplied by Yamato Co., Ltd.), and maintained there for 1 hour. Subsequently, it was heated for 6 hours and 40 min to reach 1,000°C in a nitrogen flow to provide 2.7 g of carbon particles. The resulting carbon particles had a number average particle diameter of 4.1 μm, particle diameter distribution index of 1.29, and sphericity of 96, and the height ratio (B)/(A), which represents the degree of graphitization, was 1.02.

[Example 6]

A 10.0 g portion of the metal-containing polyacrylonitrile copolymer particles prepared in Production example 4 was put on a porcelain dish, heated for 50 min to raise the temperature from room temperature to 250°C in air in a muffle furnace (FP41, supplied by Yamato Co., Ltd.), and maintained there for 1 hour. Subsequently, it was heated for 2 hours and 30 min to reach 1,000°C in a nitrogen flow to provide 3.3 g of metal-containing carbon particles. The resulting carbon particles had a number average particle diameter of 5.3 μm, particle diameter distribution index of 1.46, and sphericity of 90, and the height ratio (B)/(A), which represents the degree of graphitization, was 1.13. Quantitative analysis for the metal content in the metal-containing carbon particles was carried out, and results showed that 17 mg of iron was contained per gram of the metal-containing carbon particles.

The electrode activity for oxygen reduction of the resulting metal-containing carbon particles was measure using a tripole rotary electrode cell. Results showed that the electrode activity for oxygen reduction of the carbon particles prepared in Example 6 was -0.23 ($mA/cm^2$), indicating that they were able to serve as catalyst for oxygen reduction.

Measurements were also made to determine the magnetization characteristics of the resulting metal-containing carbon particles using a vibrating sample magnetometer (BHV-50HM, supplied by Riken Denshi). Results showed that the magnetic susceptibility was 1.0 emu/g at 10 kOe, indicating that they had magnetic characteristics.

[Example 7]

A 10.0 g portion of the polyacrylonitrile copolymer particles prepared in Production example 5 was put on a porcelain dish, heated for 50 min to reach 250°C in air in a muffle furnace (FP41, supplied by Yamato Co., Ltd.), and maintained there for 1 hour. Subsequently, it was heated for 2 hours and 30 min to reach 1,000°C in a nitrogen flow to provide 1.7 g of carbon particles. The resulting carbon particles had a number average particle diameter of 7.7 μm, particle diameter distribution index of 1.71, and sphericity of 85, and the height ratio (B)/(A), which represents the degree of graphitization, was 1.00. A scanning electronic microscope observation of a cross section of a resulting porous carbon particle is given in Fig. 7, and a scanning electronic microscope of the surfaces of particles is given in Fig. 8. Fig. 7 and

Fig. 8 show that the particles have a smooth surface and contain a porous structure in their interior. Observation of the cross sections of the porous carbon particles showed that porous structures of 0.5 μm were contained. For comparison, the cross sections of the carbon particles produced in Example 1 were observed (Fig. 9), but no porous structures were found in their interior.

[Example 8]

A 10.0 g portion of the polyacrylonitrile copolymer particles prepared in Production example 6 was put on a porcelain dish, heated for 50 min to reach 250°C in air in a muffle furnace (FP41, supplied by Yamato Co., Ltd.), and maintained there for 1 hour. Subsequently, it was heated for 2 hours and 30 min to reach 1,000°C in a nitrogen flow to provide 1.7 g of carbon particles. The resulting porous carbon particles had a number average particle diameter of 7.0 μm, particle diameter distribution index of 1.31, and sphericity of 87, and the height ratio (B)/(A), which represents the degree of graphitization, was 1.00. The cross sections and surfaces of the porous carbon particles were observed as in Example 7, showing that porous structures of 1.0 μm were contained. It was also seen that their surface was smooth and their interior was porous.

[Example 9]

A 10.0 g portion of the polyacrylonitrile copolymer particles prepared in Production example 9 was put on a porcelain dish, heated for 50 min to reach 250°C in air in an atmosphere furnace (FP41, supplied by Yamato Co., Ltd.), and maintained there for 1 hour. Subsequently, it was heated for 2 hours and 30 min to reach 1,000°C in a nitrogen flow to provide 6.1 g of carbon particles. The resulting carbon particles had a number average particle diameter of 4.4 μm, particle diameter distribution index of 1.41, and sphericity of 86, and the height ratio (B)/(A), which represents the degree of graphitization, was 1.01.

[Example 10]

A 10.0 g portion of the polyacrylonitrile copolymer particles prepared in Production example 10 was put on a porcelain dish, heated for 50 min to reach 250°C in air in an atmosphere furnace (FP41, supplied by Yamato Co., Ltd.), and maintained there for 1 hour. Subsequently, it was heated for 2 hours and 30 min to reach 1,000°C in a nitrogen flow to provide 5.2 g of carbon particles. The resulting carbon particles had a number average particle diameter of 6.8 μm, particle diameter distribution index of 1.49, and sphericity of 84, and the height ratio (B)/(A), which represents the degree of graphitization, was 1.02.

[Comparative example 1]

A 10.0 g portion of the polyacrylonitrile particles prepared in Production example 2 was put on a porcelain dish, heated at a rate of 5°C/min to reach 250°C in air in a muffle furnace (FP41, supplied by Yamato Co., Ltd.), and maintained there for 1 hour. As a result, particles were fused together to form aggregates, and it was impossible to determine their particle diameters and sphericity.

[Comparative example 2]

Carbon particles were prepared according to the method for producing carbon particles from phenol particles proposed in Patent document 1 (Japanese Unexamined Patent Publication (Kokai) No. SHO 63-129006), which is known as a method for calcining a carbon particle precursor formed by polymerization. Specifically, 100 parts by mass of a mixed aqueous solution consisting of 18 parts by mass of hydrochloric acid and 9 parts by mass of formaldehyde was put in a reaction vessel, and 3.3 parts by mass of an aqueous solution with a ratio of phenol/water = 90/10 parts by mass was added, followed by stirring for 60 seconds. While the solution was left to stand after the stirring, the interior of the system was gradually clouded, resulting in the formation of pink resinous material in 30 min. Subsequently, it was heated for 60 minutes while stirring to reach 80°C, and maintained there for 20 minutes. Then, it was separated, rinsed, treated in an aqueous ammonia solution of 0.2 parts by mass at a temperature of 60°C for 60 minutes, rinsed again, and dried at a temperature of 100°C to provide phenol formaldehyde resin particles. The phenol formaldehyde resin particles had a number average particle diameter of 19.2 μm, particle diameter distribution index of 2.79, and sphericity of 57. A 9.2 g portion of the resulting particles was put on a porcelain dish, heated for 2 hours to reach 800°C in a muffle furnace (FP41, supplied by Yamato Co., Ltd.) in a nitrogen flow, and maintained there for 80 minutes to provide 2.7 g of carbon particles. The resulting carbon particles had a number average particle diameter of 10.1 μm, particle diameter distribution index of 2.84, and sphericity of 86, and the height ratio (B)/(A), which represents the degree of graphitization, was 0.85.

A scanning microscope photograph of the resulting particles is given in Fig. 4.

[Comparative example 3]

Carbon particles were prepared with reference to Patent document 4 (Japanese Unexamined Patent Publication (Kokai) No. 2009-295441). Then, 10 parts by mass of phthalocyanine iron was added to the polyacrylonitrile solution prepared in Production example 1, and the resulting solution was subjected to electrospinning under the conditions of an applied voltage of 25 kV, discharge pressure of 5 kPa, discharge nozzle end diameter of 0.31 mm, nozzle-to-collector distance of 0.20 m to provide nanofiber nonwoven fabric. The resulting nonwoven fabric was put in an oven, heated for 50 min to raise the temperature from room temperature to 250°C in an air atmosphere, and maintained there for 1 hour. Subsequently, it was heated for 2 hours and 30 min to reach 1,000°C in a nitrogen flow to perform carbonization. The resulting nonwoven fabric, along with a zirconia ball with a diameter of 1.5 mm, was set up in a ball mill and crushed for 10 minutes at a rotating speed of 800 rpm. The resulting crushed material was passed through a sieve of 105 μm to provide carbon particles. The resulting carbon particles had a number average particle diameter of 43.2 μm, particle diameter distribution index of 5.8, showing a very wide distribution. The sphericity was 20, and the height ratio (B)/(A), which represents the degree of graphitization, was 0.94.

Quantitative analysis for metal content was carried out, and results showed that 20 mg of iron was contained per gram of the metal-containing carbon particles.

## Claims

1. A carbon particle production process comprising mixing polymer A which is at least one or more selected from the group of a polyolefin based copolymer, polyacrylonitrile based copolymer produced by copolymerization of an acrylonitrile based monomer and a hydrophilic vinyl monomer, polyacrylamide based polymer, polyvinyl acetate based polymer, polyvinyl chloride based polymer, polyvinylidene chloride based polymer, polyamide, polyarylene ether, polyarylene sulfide, polysulfone, polyether ketone, polyether ether ketone, polyarylate, polyamide-imide, and polyimide, and polymer B which is dissimilar thereto in an organic solvent, to form an emulsion in a phase-separated system consisting of a solution phase containing polymer A as primary constituent and another solution phase containing polymer B as primary constituent, which is then brought into contact with a poor solvent for polymer A to precipitate polymer A, followed by producing particles of polymer A and carbonizing the resulting polymer particles.

2. A carbon particle production process as claimed in claim 1 wherein said polymer A is either a polyacrylonitrile based copolymer produced by copolymerization of an acrylonitrile based monomer and a hydrophilic vinyl monomer, or a polyamide-imide.

3. A carbon particle production process as claimed in either claim 1 or 2 wherein said polyacrylonitrile based copolymer produced by copolymerization of an acrylonitrile based monomer and a hydrophilic vinyl monomer is an acrylonitrile copolymer produced by copolymerizing 100 parts by mass of an acrylonitrile based monomer with more than 0 to 25 parts by mass of a hydrophilic vinyl monomer.

4. A carbon particle production process as claimed in any of claims 1 to 3 wherein said hydrophilic vinyl monomer contains one or more of the following: hydroxyl group, carboxyl group, amino group, amide group, sulfonic acid group and phosphoric acid group.

5. A carbon particle production process as claimed in any of claims 1 to 4 wherein said hydrophilic vinyl monomer contains one or more of the following: amide group and carboxyl group.

6. A carbon particle production process as claimed in any of claims 1 to 5 wherein said hydrophilic vinyl monomer is one or more selected from the following: acrylic acid, methacrylic acid, itaconic acid, and acrylamide.

7. A carbon particle production process wherein polyamide-imide particles are carbonized.

8. A carbon particle production process as claimed in any of claims 1 to 7 wherein the degree of graphitization is 1.0 or more.

9. A carbon particle production process as claimed in any of claims 1 to 8 wherein the number-average particle diameter is 0.1 to 100 μm and the particle diameter distribution index is 1.0 to 2.0.

**10.** A carbon particle production process as claimed in any of claims 1 to 9 wherein said synthetic polymer particles contains metal.

**11.** A carbon particle production process as claimed in any of claims 1 to 10 wherein said synthetic polymer particles contain a daughter particle component and can be carbonized into porous carbon particles.

**12.** A carbon particle production process as claimed in any of claims 1 to 9 and 11 wherein said daughter particles are organic particles.

**13.** Carbon particles having a number average particle diameter of 0.1 to 100 μm, a particle diameter distribution index of 1.0 to 2.0, a degree of graphitization of 1.0 or more, and a sphericity of 80 or more.

**14.** Carbon particles as claimed in claim 13 that contain metal.

**15.** Carbon particles as claimed in claim 14 wherein said metal is a transition metal.

**16.** Metal-containing carbon particles as claimed in either claim 14 or 15 wherein said metal is at least one selected from the group of cobalt, iron, manganese, nickel, copper, titanium, chrome, and zinc.

**17.** Carbon particles as claimed in any of claims 14 to 16 wherein said metal is a magnetic metal.

**18.** A catalyst comprising carbon particles as claimed in any of claims 14 to 17.

[Fig. 1]

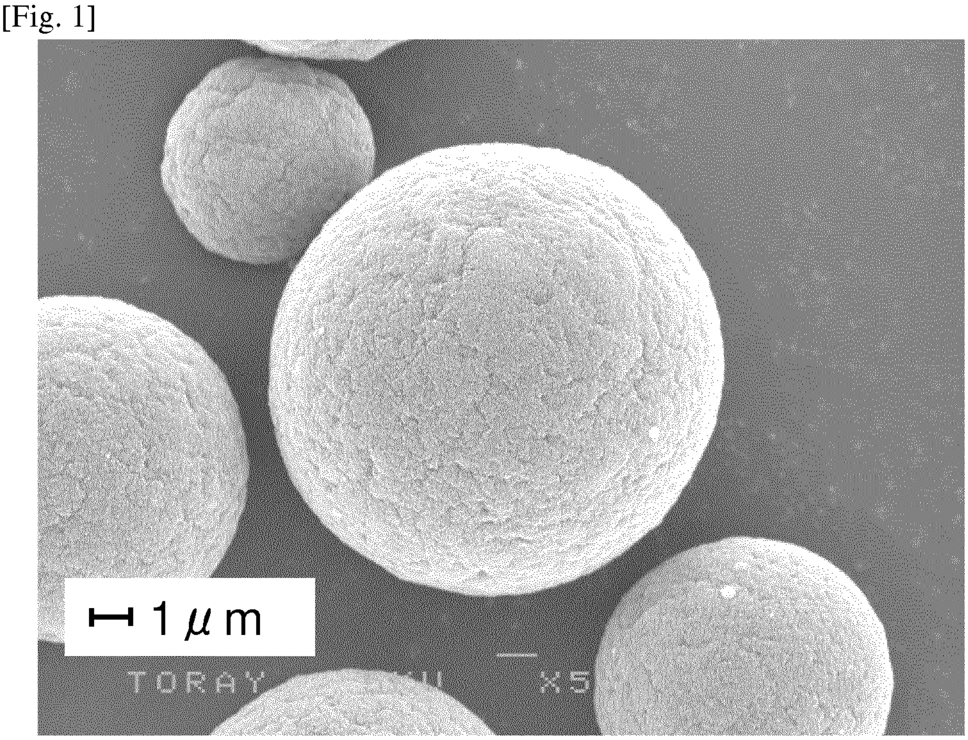


[Fig. 2]

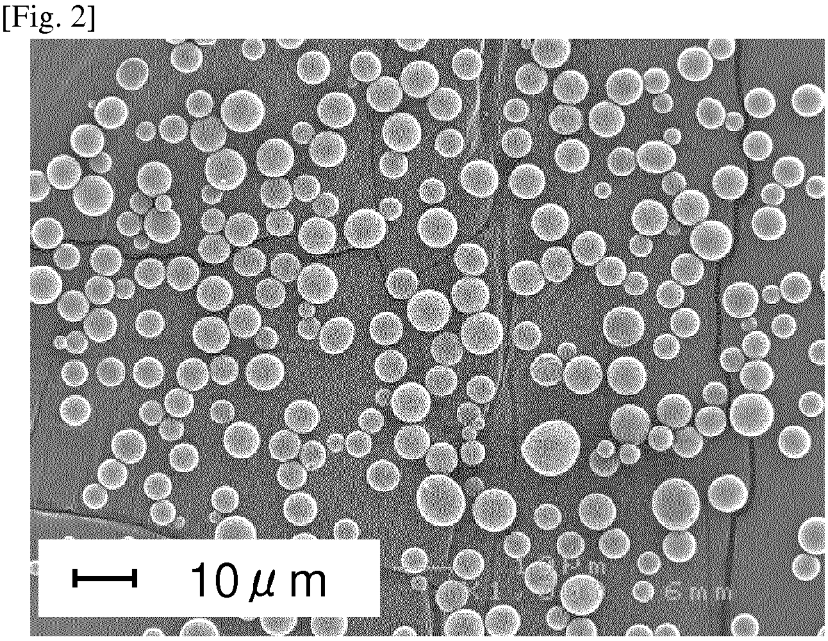

[Fig. 3]

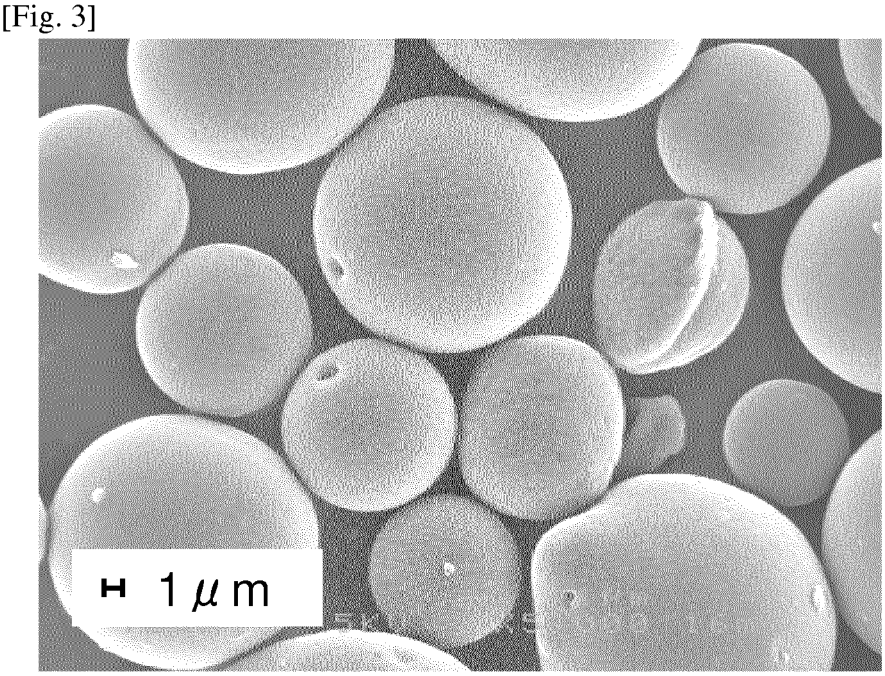


[Fig. 4]

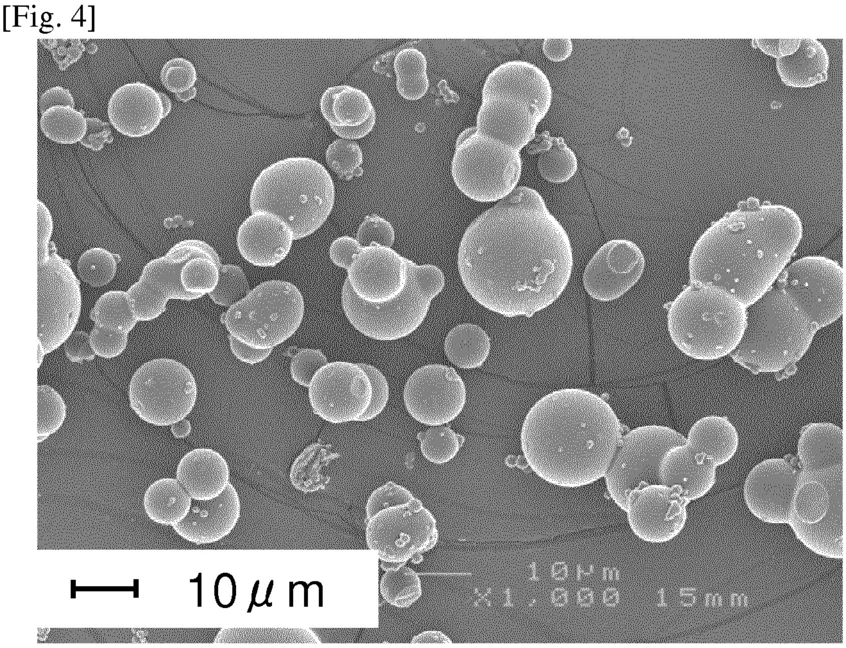

[Fig. 5]

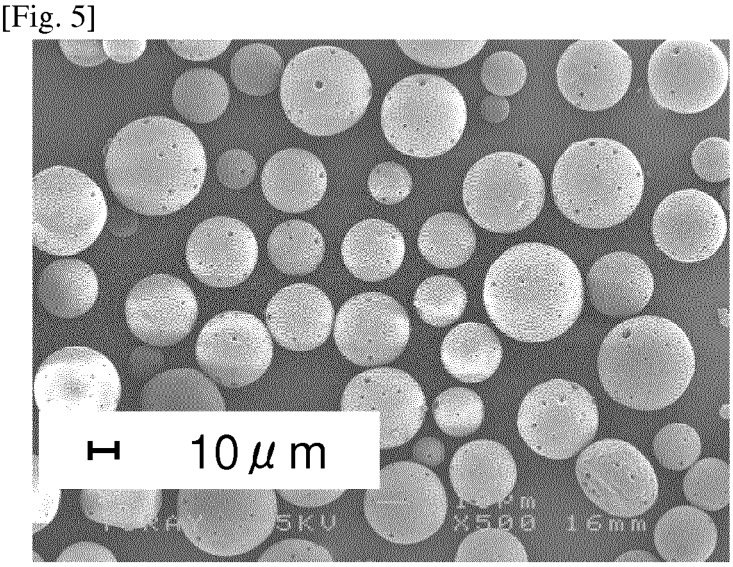


[Fig. 6]

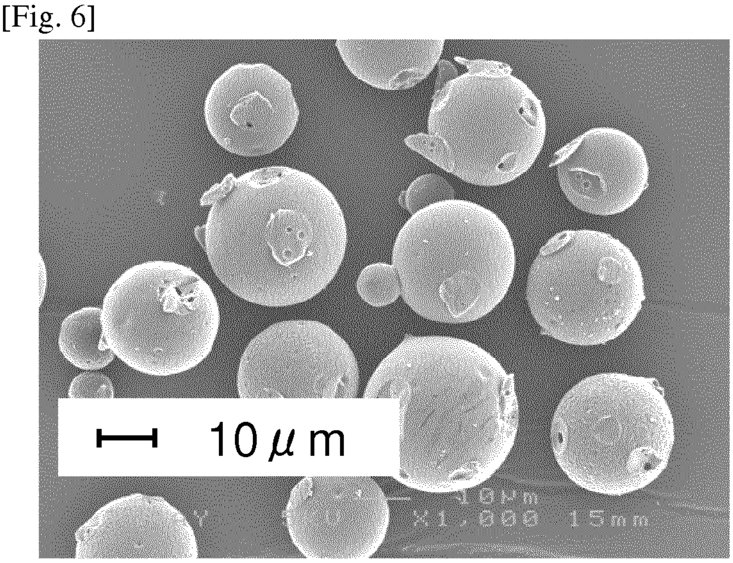

[Fig. 7]

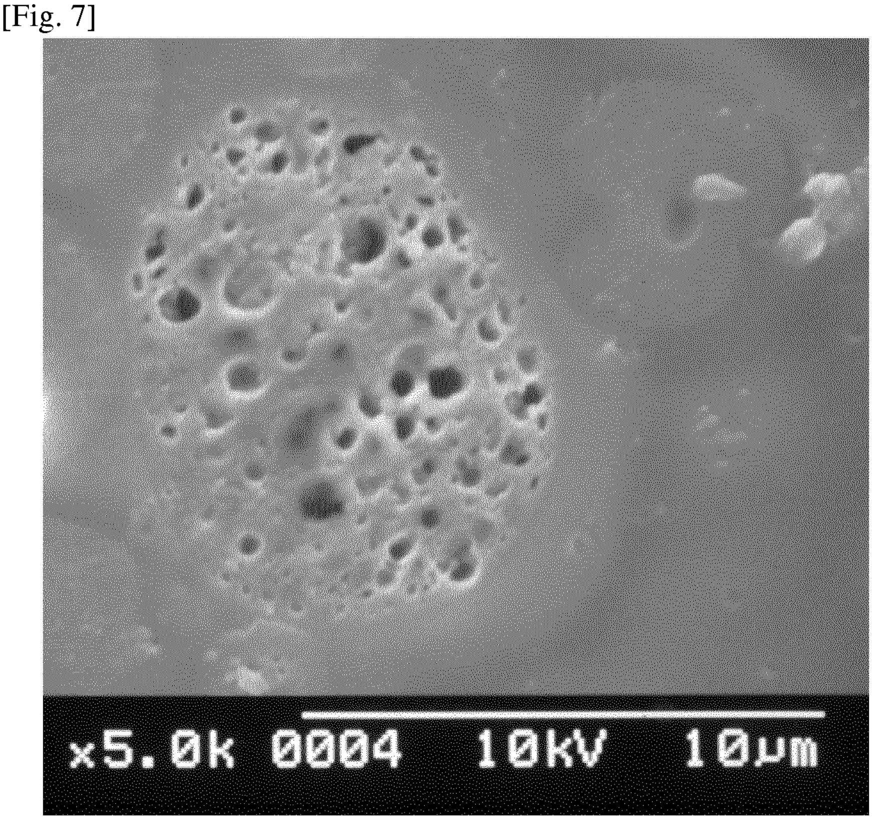

[Fig. 8]

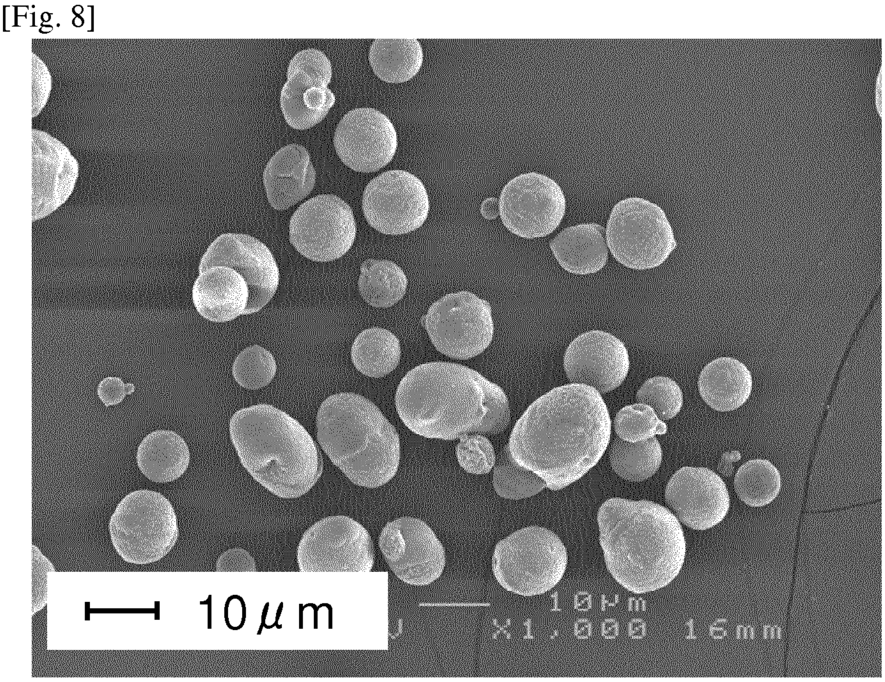


[Fig. 9]

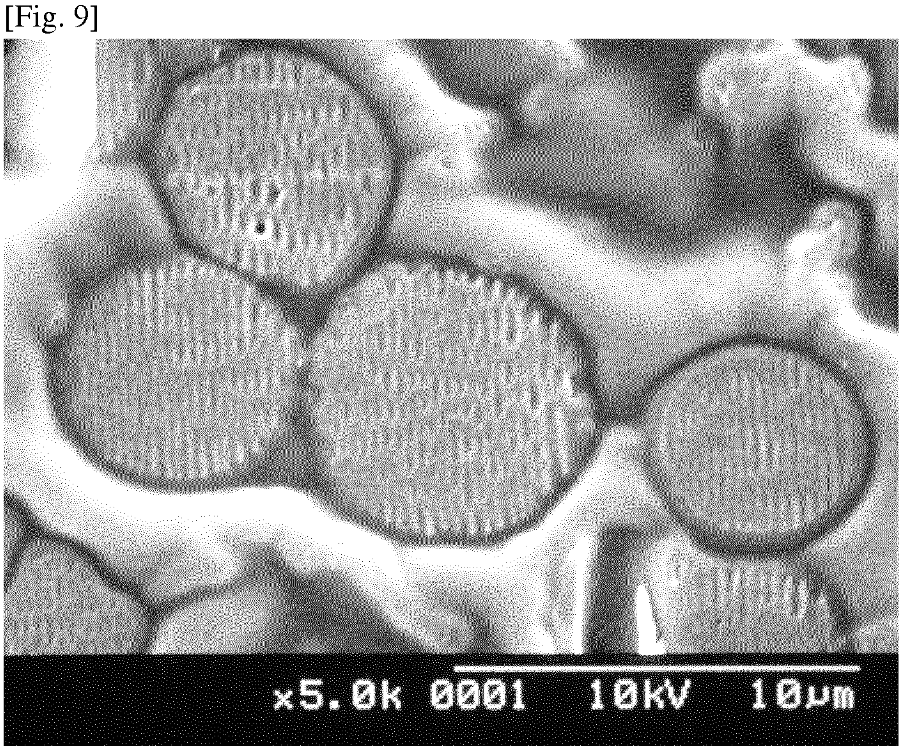

## INTERNATIONAL SEARCH REPORT

International application No.
PCT/JP2010/072957

A. CLASSIFICATION OF SUBJECT MATTER
*C01B31/02*(2006.01)i, *B01J23/745*(2006.01)i

According to International Patent Classification (IPC) or to both national classification and IPC

B. FIELDS SEARCHED

Minimum documentation searched (classification system followed by classification symbols)
C01B31/02, B01J23/745

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>A | JP 7-33420 A (Toho Rayon Co., Ltd.),<br>03 February 1995 (03.02.1995),<br>claim 1; paragraphs [0018] to [0021], [0036]<br>(Family: none) | 7-18<br>1-6 |
| X<br>A | JP 64-29493 A (Nippon Carbon Co., Ltd.),<br>31 January 1989 (31.01.1989),<br>claim 18; page 5, upper right column, lines 1 to 8<br>(Family: none) | 13-17<br>1-12,18 |
| X<br>A | JP 64-81890 A (Nippon Carbon Co., Ltd.),<br>28 March 1989 (28.03.1989),<br>claim 20; page 6, upper right column, line 3 to lower left column, line 1<br>(Family: none) | 13-18<br>1-12 |

☒ Further documents are listed in the continuation of Box C.    ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>28 March, 2011 (28.03.11) | Date of mailing of the international search report<br>05 April, 2011 (05.04.11) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No.
PCT/JP2010/072957

C (Continuation). DOCUMENTS CONSIDERED TO BE RELEVANT

| Category* | Citation of document, with indication, where appropriate, of the relevant passages | Relevant to claim No. |
|---|---|---|
| X<br>A | JP 3-187908 A (Nippon Carbon Co., Ltd.), 15 August 1991 (15.08.1991), examples 2 to 4; page 6, upper right column, line 17 to lower left column, line 1 (Family: none) | 13-17<br>1-12,18 |
| X<br>A | JP 2-26821 A (Showa Denko Kabushiki Kaisha), 29 January 1990 (29.01.1990), page 2, upper left column, lines 1 to 12; example 3 (Family: none) | 13-17<br>1-12,18 |
| X<br>A | JP 2000-223121 A (TDK Corp. et al.), 11 August 2000 (11.08.2000), paragraphs [0018], [0019], [0027], [0044] (Family: none) | 13-17<br>1-12,18 |
| X<br>A | JP 2003-238135 A (Mitsui Mining Co., Ltd.), 27 August 2003 (27.08.2003), paragraphs [0108], [0111], [0114] (Family: none) | 13-17<br>1-12,18 |
| A | JP 8-321300 A (Canon Inc.), 03 December 1996 (03.12.1996), paragraphs [0050] to [0054] & US 5702845 A | 1-18 |

Form PCT/ISA/210 (continuation of second sheet) (July 2009)

**INTERNATIONAL SEARCH REPORT**

International application No. PCT/JP2010/072957

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The invention described in claim 1 and the invention described in claim 7 share such a common technical feature that polymer microparticles are carbonized/burned to produce carbon microparticles. However, the technical feature is known (if necessary, see, for example, [0010] and [0011] in the description of the present application), and therefore it cannot be considered that the invention described in claim 1 and the invention described in claim 7 have the same or corresponding special technical feature in the meaning within PCT Rule 13.2, second sentence.

Consequently, the groups of inventions stated below cannot be regarded as

(continued to extra sheet)

1. ☒ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:
4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☒ No protest accompanied the payment of additional search fees.

Form PCT/ISA/210 (continuation of first sheet (2)) (July 2009)

INTERNATIONAL SEARCH REPORT

International application No.
PCT/JP2010/072957

Continuation of Box No.III of continuation of first sheet(2)

a single invention or a group of inventions so linked as to form a single general inventive concept.

(Invention 1) The inventions described in claims 1-6 and 8-18.
Inventions relating to: a process for producing carbon microparticles, characterized by comprising forming an emulsion in a system prepared by mixing at least one polymer A selected from the group consisting of a polyolefin copolymer, a polyacrylonitrile copolymer comprising a copolymer of an acrylonitrile monomer and a hydrophilic vinyl monomer, a polyacrylamide polymer, a poly(vinyl acetate) polymer, a poly(vinyl chloride) polymer, a poly(vinylidene chloride) polymer, a polyamide, a poly(arylene ether), a poly(arylene sulfide), a polysulfone, a poly(ether ketone), a poly(ether ether ketone), a polyarylate, a polyamideimide and a polyimide with a polymer B that is different from the polymer A in an organic solvent to cause the phase separation between a liquid phase containing the polymer A as the main component and a liquid phase containing the polymer B as the main component, bringing the emulsion into contact with a poor solvent for the polymer A to cause the precipitation of the polymer A, thereby producing microparticles of the polymer A, and carbonizing/burning the polymer microparticles; and the carbon microparticles.

(Invention 2) The invention described in claim 7.
An invention relating to a process for producing carbon microparticles, characterized by comprising carbonizing/burning polyamideimide microparticles.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2009293052 A
- JP 2009292651 A
- JP 2009291707 A
- JP 2001122607 A
- JP HEI5254814 B
- JP 2007269551 A
- JP SHO63129006 B
- JP 2003226720 A
- JP SHO6126505 B
- JP HEI05139711 B
- WO 2005098998 A
- JP 2009295441 A

### Non-patent literature cited in the description

- SP-chi: Kiso • Oyo to Keisan Hoho. Johokiko Co., Ltd, 31 March 2005
- Polymer Handbook. Wiley, 1998